# EUROPEAN PATENT APPLICATION

(11) **EP 1 867 640 A1**
(43) Date of publication of application: **19.12.2007**
(21) Application number: 06729612.9
(22) Date of filing: 22.03.2006
(51) Int. Cl.: C07D 285/135, A61K 31/433, A61K 31/454, A61P 35/00, A61P 43/00, C07D 417/04

(54) **AGENT FOR TREATMENT OF SOLID TUMOR**

(30) Priority: 22.03.2005 JP 2005081147
(71) Applicant: KYOWA HAKKO KOGYO CO., LTD., Chiyoda-ku, Tokyo 100-8185 (JP); Fujifilm Corporation, Minato-ku Tokyo 106-0031 (JP)
(72) Inventor: MURAKATA, Chikara Pharm. Res. Center, Kyowa Hakko, Sunto--gun, Shizuoka 4118731 (JP); KATO, Kazuhiko Pharm. Res. Center, Kyowa Hakko, Sunto-gun, Shizuoka, 4118731 (JP); YAMAMOTO, Junichiro Pharm. Res. Center, Kyowa Hakko, Sunto-gun, Shizuoka 4118731 (JP); NAKAI, Ryuichiro Pharm. Res. Center, Kyowa Hakko, Sunto-gun, Shizuoka, 4118731 (JP); OKAMOTO, Seiho Pharm. Res. Center, Kyowa Hakko, Sunto-gun, Shizuoka, 4118731 (JP); INO, Yoji, Ohtemachi 1-chome, Chiyoda-ku, Tokyo, 1008185 (JP); KITAMURA, Yushi Pharm. Res. Center, Kyowa Hakko, Sunto-gun, Shizuoka, 4118731 (JP); SAITOH, Toshikazu Sakai Research Laboratories, sakai-shi, Osaka 5908554 (JP); KATSUHIRA, Takeshi Sakai Research Laboratories, Sakai-shi, Osaka 5908554 (JP)
(74) Representative: Polypatent
(86) International application number: PCT/JP2006/305645
(87) International publication number: WO 2006/101102

(57) **Abstract**

A therapeutic and/or prophylactic agent for a solid tumor, which comprises a thiadiazoline derivative represented by the general formula (I), or a pharmaceutically acceptable salt thereof: 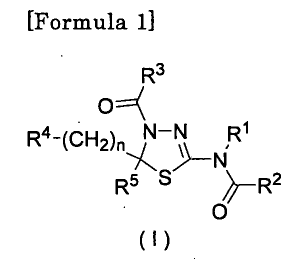 [wherein, n represents an integer of 1 to 3, R¹ represents a hydrogen atom, R² represents lower alkyl, or R¹ and R² are combined together to represent alkylene, R³ represents lower alkyl, R⁴ represents NHSO₂R⁶ (wherein R⁶ represents hydroxy or the like) or the like, and R⁵ represents aryl or the like] and the like are provided.

## Description

### Technical Field

The present invention relates to a therapeutic and/or prophylactic agent for a solid tumor, and an optically active thiadiazoline derivative useful as a therapeutic and/or prophylactic agent for a solid tumor.

### Background Art

In chemotherapies of cancers, a variety of antitumor agents including microtubule acting agents such as taxanes and vinca alkaloids, topoisomerase inhibitors, alkylating agents, and the like are used. These antitumor agents have various problems, for example, applicable cancers are limited, they cause side effects such as bone marrow toxicity and neuropathy, and they may encounter appearance of resistant tumors [Nature Reviews Cancer, Vol. 3, p.502 (2003)].

In recent years, molecule targeting type antitumor agents have been reported, which exhibit effectiveness against a specific cancer. Imatinib and gefitinib, which are tyrosine kinase inhibitors, exhibit effectiveness against chronic myeloid leukemia and non-small cell lung cancer, respectively, for which antitumor agents available are ineffective. However, the cancers against which they exhibit effectiveness are limited. Clinical cases are also reported in which acquisition of resistance is observed [Nature Reviews Drug Discovery, Vol. 3, p.1001 (2004)]. Therefore, novel antitumor agents that are improved to solve these problems have been desired.

The mitotic kinesins are proteins that are involved in the mitotic spindle regulation, and play an essential role for progression of the mitotic phase in cell cycle. The mitotic kinesin Eg5, one of the mitotic kinesins, is a bipolar homotetramer molecule, and is known to be involved in the formation of the bipolar spindle structure by crosslinking two of microtubules of the same direction and moving them in the direction toward the + (plus) end to cause sliding of two of the antiparallel microtubules, thereby keep - (minus) ends of microtubules at a distance and separate spindle pole bodies [Cell, Vol. 83, p.1159 (1995); J. Cell Biol., Vol. 150, p.975 (2000); Jikken Igaku (Experimental Medicine), Vol. 17, p.439 (1999)]. Therefore, Eg5 inhibitors are considered promising as therapeutic agents of diseases relating to cell proliferation [WO2001/98278; WO2002/56880; WO2002/57244; Trends in Cell Biology, Vol. 12, p.585 (2002)]. As Eg5 inhibitors, there are known, for example, quinazolin-4-one derivatives (WO2001/30768, WO2003/039460, and the like), triphenylmethane derivatives (WO2002/56880), thiadiazoline derivatives (refer to Patent documents 1 to 3), and the like.

There are further known thiadiazoline derivatives having a lower alkanoylamino group at the 2-position, a lower alkanoyl group at the 4-position, and a substituted or unsubstituted aryl group and a lower alkyl group at the 5-position (see, Non-patent documents 1 to 3). Moreover, thiadiazoline derivatives useful as antitumor agents are known (see, Patent documents 2 to 4). For example, the compounds represented by the following formulas (P) to (U) and the like are known to suppress proliferation of colon cancer cells (see, Patent document 4). 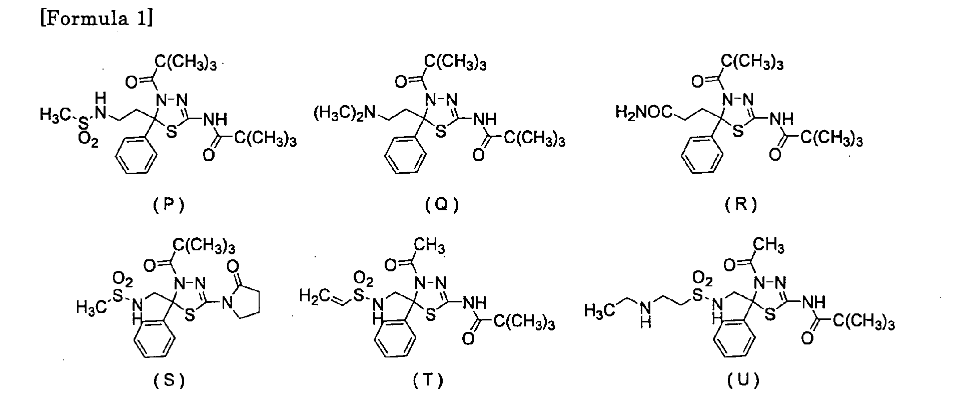 [Patent document 1] International Patent Publication WO2004/092147
[Patent document 2] International Patent Publication WO2004/111023
[Patent document 3] International Patent Publication WO2004/111024
[Patent document 4] International Patent Publication WO2003/051854
[Non-patent document 1] J. Chem. Soc. Chem. Comm., 1982, p.901
[Non-patent document 2] Arch. Pharm. Res., 2002, Vol. 25, p.250
[Non-patent document 3] CAS REGISTRY Database [registered as chemical library (Registry numbers: 352225-16-2, 332389-23-8, 332389-24-9, 332389-25-0, 443105-83-7, 443105-73-5, 443105-51-9, 443105-46-2, 443105-41-7, 443105-34-8, 443105-88-2, 443105-78-0, 443105-56-4, 432536-58-8]

### Disclosure of the Invention

### Object to be Solved by the Invention

An object of the present invention is to provide a therapeutic and/or prophylactic agent for a solid tumor (for example, a tumor of chest such as lung cancer, breast cancer, thymoma or mesothelioma, gastrointestinal cancer such as gastric cancer, esophageal cancer, hepatic cancer, pancreatic cancer, bile duct cancer or gallbladder cancer, a tumor of female genitalia such as ovarian cancer, germ cell tumor, choriocarcinoma, vulvar cancer, uterine cancer, vaginal cancer or uterine sarcoma, a tumor of male genitalia such as prostate cancer, penile cancer or testicular tumor, a tumor of urinary organ such as bladder cancer, renal cancer or renal pelvic-ureteral cancer, a tumor of cranial nerve such as brain tumor, hypophyseal tumor, glial tumor, acoustic schwannoma or neuroblastoma, head and neck cancer such as oral cancer, pharyngeal cancer, laryngeal cancer, nasal sinus cancer or thyroid cancer, retinoblastoma, mediastinal tumor, skin cancer, bone tumor, soft tissue tumor, or the like) comprising a thiadiazoline derivative or a pharmaceutically acceptable salt thereof as an active ingredient, to provide an optically active thiadiazoline derivative useful as a therapeutic and/or prophylactic agent for a solid tumor (for example, a tumor of chest such as lung cancer, breast cancer, thymoma or mesothelioma, gastrointestinal cancer such as colon cancer, gastric cancer, esophageal cancer, hepatic cancer, pancreatic cancer, bile duct cancer or gallbladder cancer, a tumor of female genitalia such as ovarian cancer, germ cell tumor, choriocarcinoma, vulvar cancer, uterine cancer, vaginal cancer or uterine sarcoma, a tumor of male genitalia such as prostate cancer, penile cancer or testicular tumor, a tumor of urinary organ such as bladder cancer, renal cancer or renal pelvic-ureteral cancer, a tumor of cranial nerve such as brain tumor, hypophyseal tumor, glial tumor, acoustic schwannoma or neuroblastoma, head and neck cancer such as oral cancer, pharyngeal cancer, laryngeal cancer, nasal sinus cancer or thyroid cancer, retinoblastoma, mediastinal tumor, skin cancer, bone tumor, soft tissue tumor, or the like), and the like.

### Means for Solving the Object

The present invention relates to the following (1) to (44).
(1) A therapeutic and/or prophylactic agent for a solid tumor, which comprises a thiadiazoline derivative represented by the general formula (I): 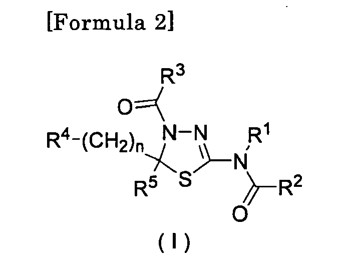 {wherein, n represents an integer of 1 to 3,
   R¹ represents a hydrogen atom,
   R² represents lower alkyl, or
   R¹ and R² are combined together to represent alkylene,
   R³ represents lower alkyl,
   R⁴ represents NHSO₂R⁶ (wherein R⁶ represents lower alkyl which may be substituted with one or two substituents selected from the group consisting of hydroxy, lower alkoxy, amino, hydroxyamino, (lower alkyl)amino, di-(lower alkyl)amino, N-hydroxy(lower alkyl)amino, amino-substituted (lower alkyl)thio, (lower alkyl)amino-substituted (lower alkyl)thio and di-(lower alkyl)amino-substituted (lower alkyl)thio, or lower alkenyl),
   NHR⁷ [wherein R⁷ represents lower alkyl which may be substituted with one or two substituents selected from the group consisting of hydroxy, lower alkoxy, amino, (lower alkyl)amino and di-(lower alkyl)amino, COR⁸ (wherein R⁸ represents lower alkyl which may be substituted with one or two substituents selected from the group consisting of hydroxy, lower alkoxy, amino, (lower alkyl)amino, di-(lower alkyl)amino, carboxy, phenyl, hydroxyphenyl, imidazolyl, guanidyl, methylthio and (lower alkoxy)carbonylamino, a nitrogen-containing aliphatic heterocyclic group which may be substituted with (lower alkoxy)carbonyl or oxo, or lower alkoxy), or a hydrogen atom], or
   CONHR⁹ (wherein R⁹ represents lower alkyl which may be substituted with one or two substituents selected from the group consisting of hydroxy, lower alkoxy, amino, (lower alkyl)amino and di-(lower alkyl)amino), and
   R⁵ represents aryl which may be substituted with one to three substituents selected from the group consisting of halogen, hydroxy, lower alkoxy, nitro, amino, cyano and carboxy}, or a pharmaceutically acceptable salt thereof.

(2) The therapeutic and/or prophylactic agent according to (1), wherein R⁵ is phenyl.
(3) The therapeutic and/or prophylactic agent according to (1) or (2), wherein R³ is methyl, ethyl, isopropyl or tert-butyl.
(4) The therapeutic and/or prophylactic agent according to any one of (1) to (3), wherein R¹ is a hydrogen atom.
(5) The therapeutic and/or prophylactic agent according to any one of (1) to (4), wherein R² is methyl or tert-butyl.
(6) The therapeutic and/or prophylactic agent according to any one of (1) to (3), wherein R¹ and R² are combined together to form trimethylene, or tetramethylene.
(7) The therapeutic and/or prophylactic agent according to any one of (1) to (6), wherein R⁴ is NHSO₂R⁶ (wherein R⁶ has the same meaning as that mentioned above).
(8) The therapeutic and/or prophylactic agent according to any one of (1) to (6), wherein R⁴ is CONHR⁹ (wherein R⁹ has the same meaning as that mentioned above).
(9) The therapeutic and/or prophylactic agent according to any one of (1) to (8), wherein n is 1 or 2.

(10) The therapeutic and/or prophylactic agent according to any one of (1) to (9), wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.
(11) The therapeutic and/or prophylactic agent according to any one of (1) to (9), wherein the solid tumor is a tumor selected from the group consisting of lung cancer, breast cancer, thymoma, mesothelioma, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.

(12) A thiadiazoline derivative represented by the general formula (II): 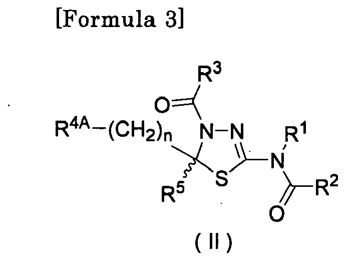 [wherein R¹, R², R³, R⁵, and n have the same meanings as those mentioned above, and R^{4A} represents NHSO₂R⁶ (wherein R⁶ has the same meaning as that mentioned above), NHR^{7A} (wherein R^{7A} represents a hydrogen atom, or lower alkyl which may be substituted with one or two substituents selected from the group consisting of hydroxy, lower alkoxy, amino, (lower alkyl)amino, and di-(lower alkyl)amino), or CONHR⁹ (wherein R⁹ has the same meaning as that mentioned above)], or a pharmaceutically acceptable salt thereof, which shows a negative value as a specific rotation at 20°C for sodium D line (wavelength: 589.3 nm) when the thiadiazoline derivative or the pharmaceutically acceptable salt thereof is dissolved in methanol.

(13) The thiadiazoline derivative or the pharmaceutically acceptable salt thereof according to (12), wherein R⁵ is phenyl.
(14) The thiadiazoline derivative or the pharmaceutically acceptable salt thereof according to (12) or (13), wherein R³ is methyl, ethyl, isopropyl, or tert-butyl.
(15) The thiadiazoline derivative or the pharmaceutically acceptable salt thereof according to any one of (12) to (14), wherein R¹ is a hydrogen atom.
(16) The thiadiazoline derivative or the pharmaceutically acceptable salt thereof according to any one of (12) to (15), wherein R² is methyl, or tert-butyl.
(17) The thiadiazoline derivative or the pharmaceutically acceptable salt thereof according to any one of (12) to (14), wherein R¹ and R² are combined together to form trimethylene or tetramethylene.
(18) The thiadiazoline derivative or the pharmaceutically acceptable salt thereof according to any one of (12) to (17), wherein R^{4A} is NHSO₂R⁶ (wherein R⁶ has the same meaning as that mentioned above).
(19) The thiadiazoline derivative or the pharmaceutically acceptable salt thereof according to any one of (12) to (17), wherein R^{4A} is CONHR⁹ (wherein R⁹ has the same meaning as that mentioned above).
(20) The thiadiazoline derivative or the pharmaceutically acceptable salt thereof according to any one of (12) to (19), wherein n is 1 or 2.

(21) The thiadiazoline derivative or the pharmaceutically acceptable salt thereof according to (12), wherein the thiadiazoline derivative represented by the general formula (II) is a thiadiazoline derivative represented by any one of the following formulas (a) to (q).

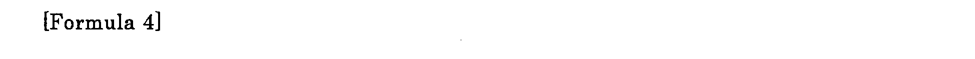

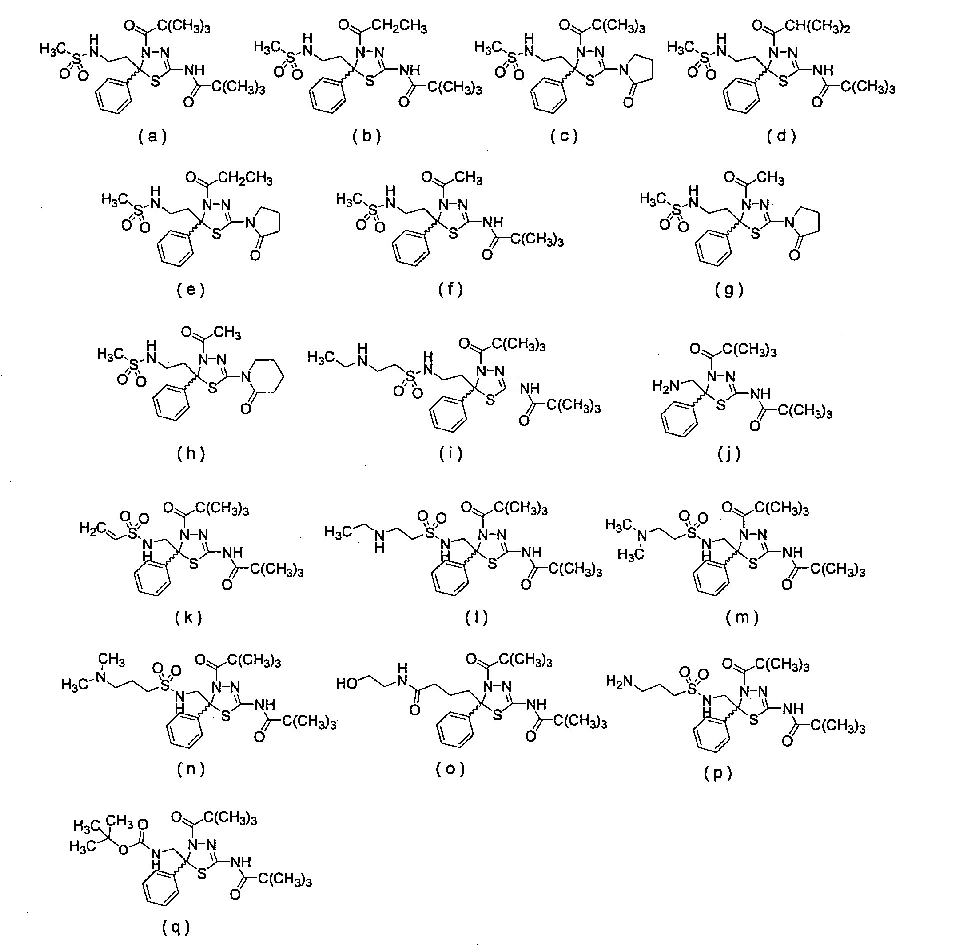

(22) A pharmaceutical composition which comprises the thiadiazoline derivative or the pharmaceutically acceptable salt thereof according to any one of (12) to (21).
(23) A mitotic kinesin Eg5 inhibitor which comprises the thiadiazoline derivative or the pharmaceutically acceptable salt thereof according to any one of (12) to (21).
(24) A therapeutic and/or prophylactic agent for a solid tumor, which comprises the thiadiazoline derivative or the pharmaceutically acceptable salt thereof according to any one of (12) to (21).
(25) The therapeutic and/or prophylactic agent according to (24), wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.
(26) The therapeutic and/or prophylactic agent according to (24), wherein the solid tumor is a tumor selected from the group consisting of lung cancer, breast cancer, thymoma, mesothelioma, colon cancer, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.

(27) A method for preparing a thiadiazoline derivative represented by the general formula (IA), or a salt thereof: 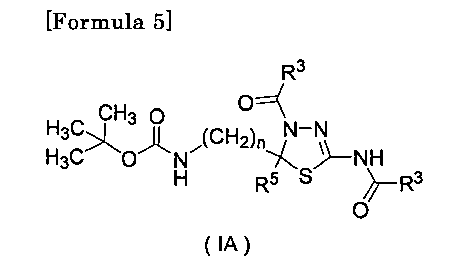 (wherein n, R³, and R⁵ have the same meanings as those mentioned above) as the thiadiazoline derivative, or the salt thereof described in (1) wherein R¹ is a hydrogen atom, R² and R³, which are the same, represent lower alkyl, and R⁴ is tert-butoxycarbonylamino, which comprises (1) the step of reacting a compound represented by the general formula (X), or a salt thereof: 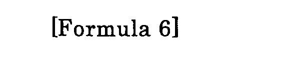 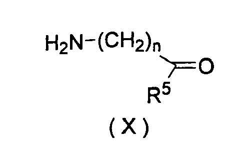 (wherein n and R⁵ have the same meanings as those mentioned above) with di-tert-butyl dicarbonate in a non-hydrophilic solvent in the presence of an aqueous solution containing a base selected from the group consisting of sodium hydrogencarbonate, potassium hydrogencarbonate, sodium carbonate, potassium carbonate, potassium hydroxide, and sodium hydroxide to obtain a compound represented by the general formula (XI): 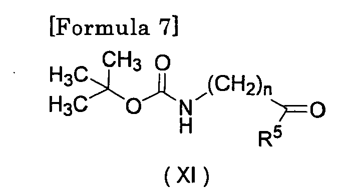 (wherein n and R⁵ have the same meanings as those mentioned above), (2) the step of reacting the compound represented by the above general formula (XI) with thiosemicarbazide in a solvent selected from methanol, ethanol, propanol, 2-propanol, butanol, sec-butanol, and tert-butanol, or in a mixed solvent of any one of these solvents and water in the presence of hydrochloric acid to obtain a compound represented by the general formula (XII): 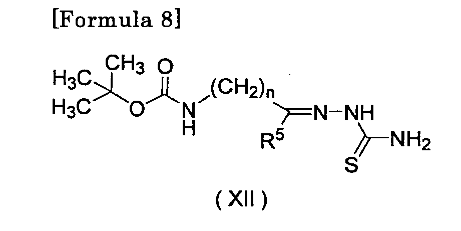 (wherein n and R⁵ have the same meaning as those defined above), and (3) the step of reacting the compound represented by the above general formula (XII) with a compound represented by the formula R³COX (wherein X represent halogen, and R³ has the same meaning as that mentioned above), or a compound represented by the formula (R³CO)₂O (wherein R³ has the same meaning as that mentioned above) in a hydrophilic solvent in the presence of a base.

(28) A method for preparing a thiadiazoline derivative represented by the general formula (IB) or (IIB), or a salt thereof: 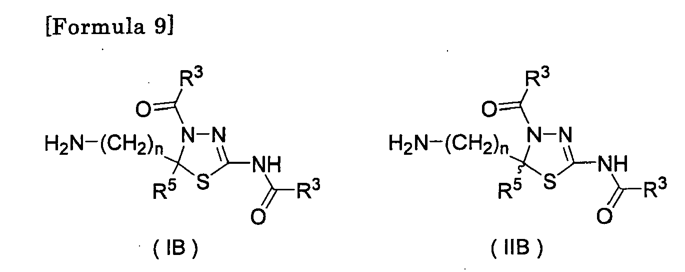 (wherein n, R³, and R⁵ have the same meanings as those mentioned above) as the thiadiazoline derivative, or the salt thereof described in (1) or (12) wherein R¹ is a hydrogen atom, R² and R³, which are the same, represent lower alkyl, and R⁴ or R^{4A} is amino, which comprises the step of treating the compound represented by the general formula (IA), or the salt thereof described in (27) in a solvent selected from the group consisting of methyl acetate, ethyl acetate, propyl acetate, isopropyl acetate, butyl acetate, methanol, ethanol, and dioxane in the presence of hydrogen chloride.

(29) A method for preparing a thiadiazoline derivative represented by the general formula (IIB), or a salt thereof: 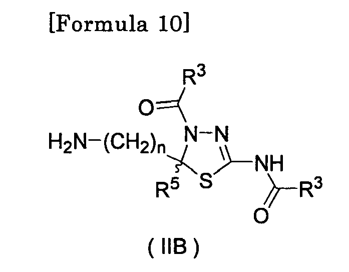 (wherein n, R³, and R⁵ have the same meanings as those mentioned above) as the thiadiazoline derivative, or the salt thereof according to (12) wherein R¹ is a hydrogen atom, R² and R³, which are the same, represent lower alkyl, and R^{4A} is amino, which comprises (1) the step of performing optical resolution of the thiadiazoline derivative represented by the general formula (IA), or the salt thereof described in (27) by high performance liquid chromatography, and (2) the step of treating the resulting thiadiazoline derivative, or the resulting salt thereof in a solvent selected from the group consisting of methyl acetate, ethyl acetate, propyl acetate, isopropyl acetate, butyl acetate, methanol, ethanol, and dioxane in the presence of hydrogen chloride.

(30) A method for preparing a thiadiazoline derivative represented by the general formula (IC) or (IIC), or a salt thereof: 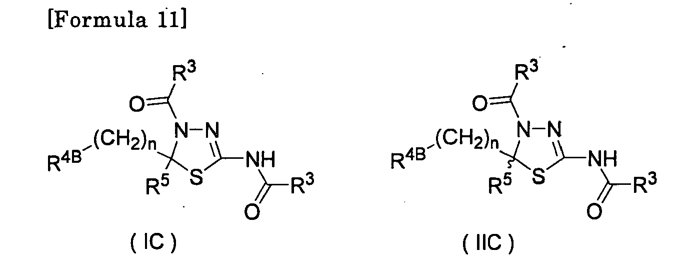 [wherein n, R¹, R³, and R⁵ have the same meanings as those mentioned above, R^{4B} represents NHSO₂R⁶ (R⁶ has the same meaning as that defined above), or NHR⁷ (R⁷ has the same meaning as that defined above)] as the thiadiazoline derivative, or the salt thereof described in any one of (1) to (5), (7), (9), (12) to (16), (18), (20) and (21), wherein R¹ is a hydrogen atom, R² and R³, which are the same, represent lower alkyl, and R⁴ or R^{4A} is NHSO₂R⁶ (R⁶ has the same meaning as that defined above), or NHR⁷ (R⁷ has the same meaning as that defined above),

which comprises using the thiadiazoline derivative represented by the general formula (IB) or (IIB), or the salt thereof described in (28) or (29).
(31) A method for therapeutic and/or prophylactic treatment of a solid tumor, which comprises administering an effective amount of the thiadiazoline derivative or the pharmaceutically acceptable salt thereof described in any one of (1) to (9).
(32) The method according to (31), wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.
(33) The method according to (31), wherein the solid tumor is a tumor selected from the group consisting of lung cancer, breast cancer, thymoma, mesothelioma, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.
(34) A method for inhibiting mitotic kinesin Eg5, which comprises administering an effective amount of the thiadiazoline derivative or the pharmaceutically acceptable salt thereof according to any one of (12) to (21).

(35) A method for therapeutic and/or prophylactic treatment of a solid tumor, which comprises administering an effective amount of the thiadiazoline derivative or the pharmaceutically acceptable salt thereof according to any one of (12) to (21).
(36) The method according to (35), wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.
(37) The method according to (35), wherein the solid tumor is a tumor selected from the group consisting of lung cancer, breast cancer, thymoma, mesothelioma, colon cancer, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.
(38) Use of the thiadiazoline derivative or the pharmaceutically acceptable salt thereof described in any one of (1) to (9) for the manufacture of a therapeutic and/or prophylactic agent for a solid tumor.
(39) The use according to (38), wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.

(40) The use according to (38), wherein the solid tumor is a tumor selected from the group consisting of lung cancer, breast cancer, thymoma, mesothelioma, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.
(41) Use of the thiadiazoline derivative or the pharmaceutically acceptable salt thereof according to any one of (12) to (21) for the manufacture of a mitotic kinesin Eg5 inhibitor.
(42) Use of the thiadiazoline derivative or the pharmaceutically acceptable salt thereof according to any one of (12) to (21) for the manufacture of a therapeutic and/or prophylactic agent for a solid tumor.
(43) The use according to (42), wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.
(44) The use according to (42), wherein the solid tumor is a tumor selected from the group consisting of lung cancer, breast cancer, thymoma, mesothelioma, colon cancer, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.

### Effect of the Invention

According to the present invention, a therapeutic and/or prophylactic agent for a solid tumor (for example, a tumor of chest such as lung cancer, breast cancer, thymoma or mesothelioma, gastrointestinal cancer such as gastric cancer, esophageal cancer, hepatic cancer, pancreatic cancer, bile duct cancer or gallbladder cancer, a tumor of female genitalia such as ovarian cancer, germ cell tumor, choriocarcinoma, vulvar cancer, uterine cancer, vaginal cancer or uterine sarcoma, a tumor of male genitalia such as prostate cancer, penile cancer or testicular tumor, a tumor of urinary organ such as bladder cancer, renal cancer or renal pelvic-ureteral cancer, a tumor of cranial nerve such as brain tumor, hypophyseal tumor, glial tumor, acoustic schwannoma or neuroblastoma, head and neck cancer such as oral cancer, pharyngeal cancer, laryngeal cancer, nasal sinus cancer or thyroid cancer, retinoblastoma, mediastinal tumor, skin cancer, bone tumor, soft tissue tumor, or the like) comprising a thiadiazoline derivative or a pharmaceutically acceptable salt thereof as an active ingredient, an optically active thiadiazoline derivative useful as a therapeutic and/or prophylactic agent for a solid tumor (for example, a tumor of chest such as lung cancer, breast cancer, thymoma or mesothelioma, gastrointestinal cancer such as colon cancer, gastric cancer, esophageal cancer, hepatic cancer, pancreatic cancer, bile duct cancer or gallbladder cancer, a tumor of female genitalia such as ovarian cancer, germ cell tumor, choriocarcinoma, vulvar cancer, uterine cancer, vaginal cancer or uterine sarcoma, a tumor of male genitalia such as prostate cancer, penile cancer or testicular tumor, a tumor of urinary organ such as bladder cancer, renal cancer or renal pelvic-ureteral cancer, a tumor of cranial nerve such as brain tumor, hypophyseal tumor, glial tumor, acoustic schwannoma or neuroblastoma, head and neck cancer such as oral cancer, pharyngeal cancer, laryngeal cancer, nasal sinus cancer or thyroid cancer, retinoblastoma, mediastinal tumor, skin cancer, bone tumor, soft tissue tumor, or the like), and the like can be provided.

### Best Mode for Carrying out the Invention

Hereinafter, compounds represented by the general formula (I) and compounds represented by the general formula (II) are referred to as "Compound (I)" and "Compound (II)", respectively. The compounds having the other formula numbers are referred to in the same manner.
In the definition of each group of the general formulas (I) and (II):
(i) Examples of the lower alkyl and the lower alkyl moiety in the lower alkoxy, the (lower alkyl)amino, the di-(lower alkyl)amino, the (lower alkoxy)carbonyl, the (lower alkoxy)carbonylamino, the N-hydroxy(lower alkyl)amino, the (lower alkyl)amino-substituted (lower alkyl)thio, and the di-(lower alkyl)amino-substituted (lower alkyl)thio include straight or branched alkyl having 1 to 10 carbon atoms, for example, methyl, ethyl, propyl, isopropyl, n-butyl, isobutyl, sec-butyl, tert-butyl, pentyl, isopentyl, neopentyl, hexyl, heptyl, octyl, nonyl, decyl and the like. The two lower alkyl moieties in the di-(lower alkyl)amino and the di-(lower alkyl)amino-substituted (lower alkyl)thio may be the same or different.
(ii) Examples of the lower alkenyl include straight or branched alkenyl having 2 to 10 carbon atoms, for example, vinyl, allyl, 1-propenyl, butenyl, pentenyl, hexenyl, heptenyl, octenyl, nonenyl, decenyl and the like.

(iii) Examples of the aryl include aryl having 6 to 14 carbon atoms, for example, phenyl, naphthyl and the like.
(iv) Examples of the alkylene include straight or branched alkylene having 1 to 10 carbon atoms, for example, methylene, ethylene, trimethylene, tetramethylene, pentamethylene, hexamethylene, heptamethylene, octamethylene, nonamethylene, decamethylene, propylene, ethylethylene, methylmethylene, dimethylmethylene and the like.
(v) Examples of the nitrogen-containing aliphatic heterocyclic group include a 5- or 6-membered monocyclic aliphatic heterocyclic group containing at least one nitrogen atom, a bicyclic or tricyclic condensed aliphatic heterocyclic group comprising 3- to 8-membered rings and containing at least one nitrogen atom and the like, for example, aziridinyl, azetidinyl, pyrrolidinyl, piperidino, piperidinyl, perhydroazepinyl, perhydroazocinyl, imidazolidinyl, pyrazolidinyl, piperazinyl, morpholino, morpholinyl, thiomorpholino, thiomorpholinyl, homopiperazinyl, tetrahydroquinolinyl, tetrahydroisoquinolinyl, dihydroindolinyl, dihydroisoindolinyl and the like.
(vi) Halogen means each atom of fluorine, chlorine, bromine, and iodine.
(vii) The alkylene moieties in the amino-substituted (lower alkyl)thio, the (lower alkyl)amino-substituted (lower alkyl)thio, and the di-(lower alkyl)amino-substituted (lower alkyl)thio have the same meanings as that of the aforementioned (iv) alkylene.

In each group of Compounds (I) and (II):
Preferred examples of R¹ include a hydrogen atom.
Preferred examples of R² include methyl, ethyl, propyl, isopropyl, n-butyl, sec-butyl, tert-butyl and the like, and more preferred examples include methyl, tert-butyl and the like.
Preferred examples of the alkylene formed by R¹ and R² combined together include trimethylene, tetramethylene, pentamethylene and the like.
Preferred examples of R³ include methyl, ethyl, propyl, isopropyl, n-butyl, sec-butyl, tert-butyl and the like, and more preferred examples include methyl, ethyl, isopropyl, tert-butyl and the like.
Preferred examples of R⁴ include NHSO₂R^{6B} [wherein R^{6B} represents methyl, ethyl, propyl, vinyl, aminomethyl, 1-aminoethyl, 2-aminoethyl, 1-aminopropyl, 2-aminopropyl, 3-aminopropyl, methylaminomethyl, 1-(methylamino)ethyl, 2-(methylamino)ethyl, 1-(methylamino)propyl, 2-(methylamino)propyl, 3-(methylamino)propyl, dimethylaminomethyl, 1-(dimethylamino)ethyl, 2-(dimethylamino)ethyl, 1-(dimethylamino)propyl, 2-(dimethylamino)propyl, 3-(dimethylamino)propyl, ethylaminomethyl, 1-(ethylamino)ethyl, 2-(ethylamino)ethyl, 1-(ethylamino)propyl, 2-(ethylamino)propyl, 3-(ethylamino)propyl, diethylaminomethyl, 1-(diethylamino)ethyl, 2-(diethylamino)ethyl, 1-(diethylamino)propyl, 2-(diethylamino)propyl, 3-(diethylamino)propyl, propylaminomethyl, 2-(propylamino)ethyl, 3-(propylamino)propyl, isopropylaminomethyl, 2-(isopropylamino)ethyl, 3-(isopropylamino)propyl, vinyl, aminomethylthiomethyl, aminoethylthiomethyl, methylaminomethylthiomethyl, dimethylaminoethylthiomethyl, aminomethylthioethyl, aminoethylthioethyl, methylaminomethylthioethyl, methylaminoethylthioethyl, dimethylaminomethylthioethyl, dimethylaminoethylthioethyl, aminomethylthiopropyl, aminoethylthiopropyl or the like], NHR^{7B} [wherein R^{7B} represents a hydrogen atom, methyl, ethyl, propyl, isopropyl, n-butyl, aminomethyl, 1-aminoethyl, 2-aminoethyl, 1-aminopropyl, 2-aminopropyl, 3-aminopropyl, methylaminomethyl, 1-(methylamino)ethyl, 2-(methylamino)ethyl, 1-(methylamino)propyl, 2-(methylamino)propyl, 3-(methylamino)propyl, dimethylaminomethyl, 1-(dimethylamino)ethyl, 2-(dimethylamino)ethyl, 1-(dimethylamino)propyl, 2-(dimethylamino)propyl, 3-(dimethylamino)propyl, ethylaminomethyl, 1-(ethylamino)ethyl, 2-(ethylamino)ethyl, 3-(ethylamino)propyl, diethylaminomethyl, 1-(diethylamino)ethyl, 2-(diethylamino)ethyl, 3-(diethylamino)propyl, propylaminomethyl, 2-(propylamino)ethyl, 3-(propylamino)propyl, isopropylaminomethyl, 2-(isopropylamino)ethyl, 3-(isopropylamino)propyl or the like], NHCOR^{8B} (wherein R^{8B} represents methyl, ethyl, propyl, n-butyl, sec-butyl, tert-butyl, n-pentyl, aminomethyl, methylaminomethyl, dimethylaminomethyl, aminoethyl, methylaminoethyl, dimethylaminoethyl, aminopropyl, methylaminopropyl, dimethylaminopropyl, pyrrolidinyl, 2-oxopyrrolidinyl, methoxy, ethoxy, n-butoxy, sec-butoxy, tert-butoxy or the like], CONHR^{9B} [wherein R^{9B} represents methyl, ethyl, propyl, isopropyl, n-butyl, 2-hydroxyethyl, 2-hydroxypropyl, 3-hydroxypropyl, 2-hydroxy-n-butyl, 3-hydroxy-n-butyl, 4-hydroxy-n-butyl, 2-hydroxy-1-(hydroxymethyl)ethyl, 2-hydroxy-1-methylethyl, aminomethyl, 1-aminoethyl, 2-aminoethyl, 1-aminopropyl, 2-aminopropyl, 3-aminopropyl, methylaminomethyl, 1-(methylamino)ethyl, 2-(methylamino)ethyl, 1-(methylamino)propyl, 2-(methylamino)propyl, 3-(methylamino)propyl, dimethylaminomethyl, 1-(dimethylamino)ethyl, 2-(dimethylamino)ethyl, 1-(dimethylamino)propyl, 2-(dimethylamino)propyl, 3-(dimethylamino)propyl, ethylaminomethyl, 1-(ethylamino)ethyl, 2-(ethylamino)ethyl, 3-(ethylamino)propyl, diethylaminomethyl, 1-(diethylamino)ethyl, 2-(diethylamino)ethyl, 3-(diethylamino)propyl, propylaminomethyl, 2-(propylamino)ethyl, 3-(propylamino)propyl, isopropylaminomethyl, 2-(isopropylamino)ethyl, 3-(isopropylamino)propyl or the like] and the like, more preferred examples include NHSO₂R^{6B} (wherein R^{6B} has the same meaning as that mentioned above), NHCOR^{8B} (wherein R^{8B} has the same meaning as that mentioned above), CONHR^{9B} (wherein R^{9B} has the same meaning as that mentioned above) and the like, still more preferred examples include NHSO₂R^{6B} (wherein R^{6B} has the same meaning as that mentioned above), NHCOR^{8BB} (wherein R^{8BB} represents methoxy, ethoxy, n-butoxy, sec-butoxy, tert-butoxy or the like), CONHR^{9B} (wherein R^{9B} has the same meaning as that mentioned above) and the like, and still further preferred examples include NHSO₂R^{6B} (wherein R^{6B} has the same meaning as that mentioned above), NHCOR^{8BB} (wherein R^{8BB} has the same meaning as that mentioned above) and the like.
Preferred examples of R⁵ include phenyl and the like.
n is preferably 1 or 2.

As Compounds (I) and (II), preferred are those having a combination of substituents selected from the preferred substituents mentioned above per group. For example, preferred are those compounds wherein R¹ is a hydrogen atom, R² is methyl, ethyl, propyl, isopropyl, n-butyl, sec-butyl, tert-butyl or the like, or R¹ and R² are combined together to represent trimethylene, tetramethylene, pentamethylene or the like, R³ is methyl, ethyl, propyl, isopropyl, n-butyl, sec-butyl, tert-butyl or the like, R⁴ is NHSO₂R^{6B} (wherein R^{6B} has the same meaning as that mentioned above), NHR^{7B} (wherein R^{7B} has the same meaning as that mentioned above), NHCOR^{8B} (wherein R^{8B} has the same meaning as that mentioned above), CONHR^{9B} (wherein R^{9B} has the same meaning as that mentioned above) or the like, R⁵ is phenyl, and n is 1 or 2, more preferred are those compounds wherein R¹ is a hydrogen atom, R² is methyl, tert-butyl or the like, or R¹ and R² are combined together to represent trimethylene, tetramethylene or the like, R³ is methyl, ethyl, isopropyl, tert-butyl or the like, R⁴ is NHSO₂R^{6B} (wherein R^{6B} has the same meaning as that mentioned above), NHCOR^{8B} (wherein R^{8B} has the same meaning as that mentioned above), CONHR^{9B} (wherein R^{9B} has the same meaning as that mentioned above) or the like, R⁵ is phenyl, and n is 1 or 2, still more preferred are those compounds wherein R¹ is a hydrogen atom, R² is tert-butyl or the like, or R¹ and R² are combined together to represent trimethylene, tetramethylene or the like, R³ is methyl, ethyl, isopropyl, tert-butyl or the like, R⁴ is NHSO₂R^{6B} (wherein R^{6B} has the same meaning as that mentioned above), NHCOR^{8BB} (wherein R^{8BB} has the same meaning as that mentioned above), CONHR^{9B} (wherein R^{9B} has the same meaning as that mentioned above), R⁵ is phenyl, and n is 1 or 2, and further preferred are those compounds wherein R¹ is a hydrogen atom, R² is tert-butyl or the like, or R¹ and R² are combined together to represent trimethylene, tetramethylene or the like, R³ is methyl, ethyl, isopropyl, tert-butyl or the like, R⁴ is NHSO₂R^{6B} (wherein R^{6B} has the same meaning as that mentioned above), NHCOR^{8BB} (wherein R^{8BB} has the same meaning as that mentioned above) or the like, R⁵ is phenyl, and n is 1 or 2.

Further, as Compound (I), preferred are those compounds showing a negative value as a specific rotation at 20°C for sodium D line (wavelength: 589.3 nm) when they are dissolved in methanol.
Furthermore, in Compounds (I) and (II), the asymmetric center to which R⁵ binds is preferably in the R-configuration when n is 1, or the asymmetric center to which R⁵ binds is preferably in the S-configuration when n is 2 or 3. Namely, Compounds (I) and (II) are preferably compounds having the steric configuration represented by the following formula (Z).

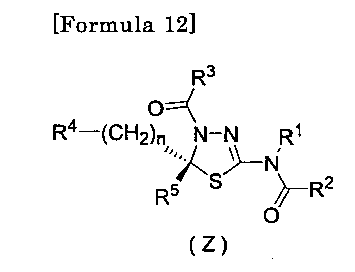

Examples of the pharmaceutically acceptable salt of Compound (I) include pharmaceutically acceptable acid addition salts, metal salts, ammonium salts, organic amine addition salts, amino acid addition salts and the like. Examples of the pharmaceutically acceptable acid addition salt of Compound (I) include an inorganic acid salt such as hydrochloride, sulfate and phosphate, an organic acid salt such as acetate, maleate, fumarate and citrate, and the like. Examples of the pharmaceutically acceptable metal salt include an alkali metal salt such as a sodium salt and a potassium salt, an alkaline-earth metal salt such as a magnesium salt and a calcium salt, an aluminium salt, a zinc salt and the like. Examples of the pharmaceutically acceptable ammonium salt include a salt of ammonium, tetramethylammonium or the like. Examples of the pharmaceutically acceptable organic amine addition salt include an addition salt of morpholine, piperidine or the like. Examples of the pharmaceutically acceptable amino acid addition salt include an addition salt of lysine, glycine, phenylalanine, aspartic acid, glutamic acid or the like.
In addition to the pharmaceutically acceptable salt mentioned above, examples of salts of Compound (I) include a trifluoroacetate, a trifluoromethanesulfonate and the like.

Next, the methods of preparing the Compounds (I) and (II) are described as follows.

### Preparing method 1

Compound (I) can be prepared by the methods described in WO2003/051854, WO2004/092147, WO2004/111024 and the like.

### Preparing method 2

Compound (II) can be prepared by subjecting Racemate (Ia) which can be obtained by the methods described in WO2003/051854, WO2004/092147, WO2004/11102 and the like as Compound (I) wherein R⁴ is R^{4A} to preparative high performance liquid chromatography using, for example, a column for optical isomer separation [for example, CHIRALPAK AD (Daicel Chemical Industries, Ltd.)] to separate each optical isomer. 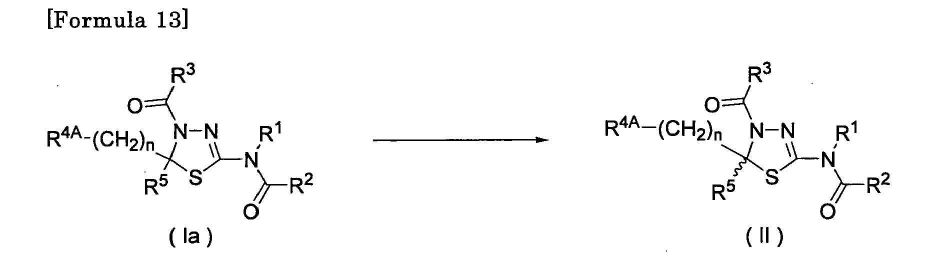 (wherein R¹, R², R³, R⁵, R^{4A} and n have the same meanings as those mentioned above, respectively)

### Preparing method 3

Compound (II) can also be prepared in accordance with the following steps. 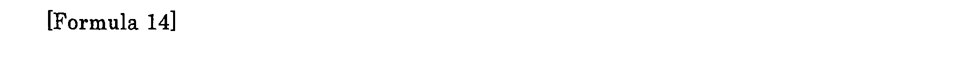 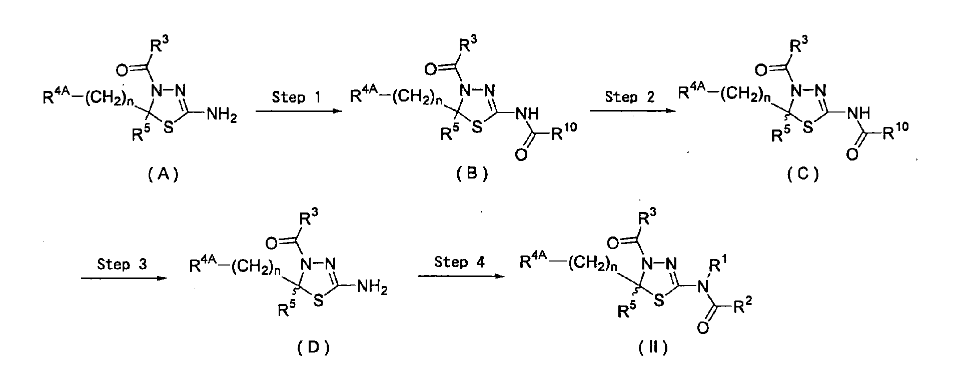 (wherein R¹, R², R³, R⁵, R^{4A}, and n have the same meanings as those mentioned above, respectively, and R¹⁰ represents an optically active substituent having one asymmetric center, for example, optically active C₁₋₁₀ alkyl, optically active hydroxy-substituted C₁₋₁₀ alkyl, optically active C₁₋₁₀ alkoxy-substituted C₁₋₁₀ alkyl, optically active phenyl-substituted C₁₋₁₀ alkyl, optically active naphthyl-substituted C₁₋₁₀ alkyl or the like, and examples of the C₁₋₁₀ alkyl and the C₁₋₁₀ alkyl moiety of the C₁₋₁₀ alkoxy include the groups exemplified for the lower alkyl mentioned above.)

The compound (A; racemate) obtained by the methods described in WO2003/051854, WO2004/092147, WO2004/111024 or the like is reacted with an optically active acylating agent [R¹⁰COX (wherein R¹⁰ has the same meaning as that mentioned above, and X represents chlorine atom, bromine atom, iodine atom or the like); (R¹⁰CO)₂O (wherein R¹⁰ has the same meaning as that mentioned above), or the like, for example, (R)-(-)-2-phenylpropionyl chloride, (S)-(+)-2-phenylpropionyl chloride and the like] according to, for example, the method described in Shin-Jikken-Kagaku-Koza Vol. 14, p.1142 (Maruzen, 1978) or the like to obtain a compound (B; mixture of diastereomers) (Step 1). Next, the diastereomers of Compound (B) obtained are separated by silica gel column chromatography, recrystallization, or other means to obtain a compound (C; one diastereomer) (Step 2). Then, Compound (C) obtained is treated with a reducing agent such as sodium borohydride, or the like according to, for example, the method described in WO2003/051854 or the like and thereby converted into Compound (D) (Step 3), and finally, Compound (D) can be, for example, acylated according to, for example, the method described in WO2003/051854 or the like to obtain Compound (II) (Step 4).

### Preparing method 4

Among Compound (II), Compound (IIa) wherein n is 1, and R^{4A} is NHSO₂R⁶ (wherein R⁶ has the same meaning as that mentioned above) or NHR^{7A} (wherein R^{7A} has the same meaning as that mentioned above) can also be prepared in accordance with the following steps. 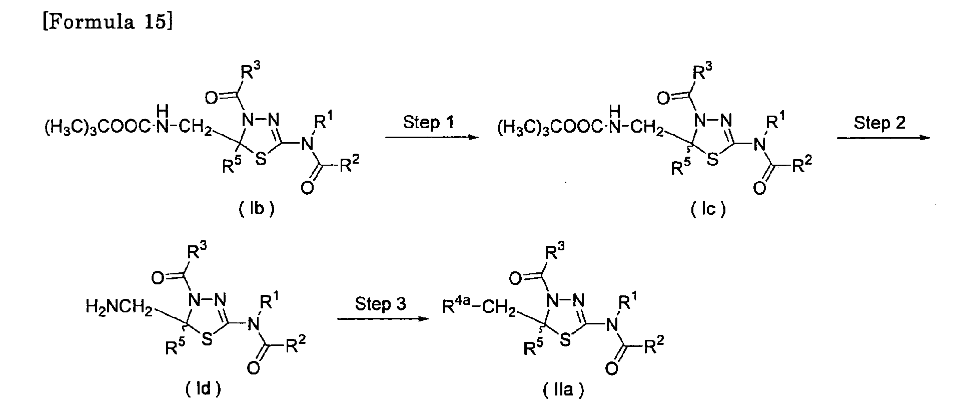 (wherein R^{4a} represents NHSO₂R⁶ (wherein R⁶ has the same meaning as that mentioned above) or NHR^{7A} (wherein R^{7A} has the same meaning as that mentioned above), and R¹, R², R³ and R⁵ have the same meanings as those mentioned above, respectively)

The compound (Ib; racemate) obtained by the method described in WO2003/051854, WO2004/092147, WO2004/11102 or the like is subjected to preparative high performance liquid chromatography using a column for optical isomer separation [for example, CHIRALPAK AD (Daicel Chemical Industries, Ltd.)] to obtain a compound (Ic; one enantiomer) (Step 1). Next, Compound (Ic) obtained is treated with an acid such as hydrochloric acid and trifluoroacetic acid according to, for example, the method described in WO2004/111024 or the like and thereby converted into Compound (Id) (Step 2), and then sulfonylation, alkylation and the like of Compound (Id) can be performed according to, for example, the method described in WO2004/11102 or the like to prepare Compound (IIa) (Step 3).

### Preparing method 5

Among Compound (I), Compound (IA) wherein R¹ is a hydrogen atom, R² and R³, which are the same, represent lower alkyl, and R⁴ is tert-butoxycarbonylamino can also be prepared in accordance with the following steps. 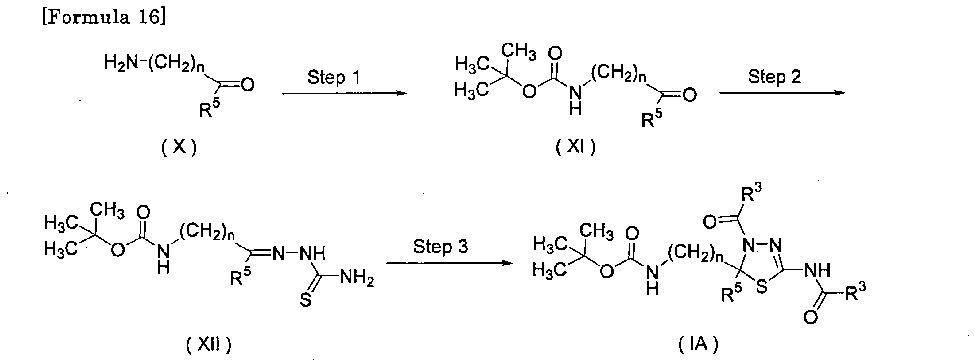 (wherein n, R¹, R³ and R⁵ have the same meaning as those mentioned above, respectively)

### Step 1

Compound (XI) can be prepared by the reaction of Compound (X) with di-tert-butyl dicarbonate in a suitable solvent in the presence of a base.
Specifically, for example, Compound (XI) can be prepared by dissolving Compound (X) in a suitable solvent, adding di-tert-butyl dicarbonate and then a base, and allowing them to react at a temperature preferably between 0°C and 80°C, more preferably between 0°C and 40°C, for 5 minutes to 72 hours, preferably 30 minutes to 4 hours.
Di-tert-butyl dicarbonate is preferably used in an amount of 1 to 10 equivalents, more preferably 1 to 3 equivalents, still more preferably 1 to 1.2 equivalents, to Compound (X).
Examples of the solvent include, for example, hydrophilic solvents such as methanol, ethanol, acetonitrile, dioxane, N,N-dimethylformamide (DMF), N,N-dimethylacetamide (DMA), N-methylpyrrolidone (NMP) and pyridine, non-hydrophilic organic solvents such as dichloromethane, chloroform, 1,2-dichloroethane, toluene, methyl acetate, ethyl acetate, propyl acetate, isopropyl acetate, butyl acetate, diethyl ether, tetrahydrofuran (THF), and 1,2-dimethoxyethane (DME); water and the like, and they can be used alone or as a mixture. Preferred examples include non-hydrophilic organic solvents, or mixed solvents of a non-hydrophilic organic solvent and water, more preferred examples include organic solvents such as methyl acetate, ethyl acetate, propyl acetate, isopropyl acetate and butyl acetate, and mixed solvents of these organic solvents and water, and still more preferred examples include mixed solvents of ethyl acetate and water (2:1 to 1:2, preferably 4:3 to 3:4, more preferably 5:4 to 1:1, still more preferably 1:1). Further, the total amount of the solvent used is, for example, such an amount that the concentration of Compound (X) should become 10 to 600 g/L, preferably 20 to 200 g/L, more preferably 30 to 80 g/L.

Examples of the base include, for example, sodium hydrogencarbonate, potassium hydrogencarbonate, sodium carbonate, potassium carbonate, potassium hydroxide, sodium hydroxide, lithium hydroxide, sodium methoxide, potassium tert-butoxide, triethylamine, diisopropylethylamine, N-methylmorpholine, pyridine, 1,8-diazabicyclo[5.4.0]-7-undecene (DBU) and the like, preferred examples include sodium hydrogencarbonate, potassium hydrogencarbonate, sodium carbonate, potassium carbonate, potassium hydroxide, sodium hydroxide and the like, and more preferred examples include sodium hydrogencarbonate, potassium carbonate and the like. The base is preferably used in a large excess amount, more preferably in an amount of 1 to 30 equivalents, still more preferably 1 to 5 equivalents, further preferably 1 to 1.2 equivalents, to Compound (X). The base is preferably dissolved in a suitable volume of water, and slowly added as an aqueous solution at a concentration of, for example, 1 to 6 mol/L, preferably 1.5 to 2.5 mol/L, to a solution dissolving Compound (X) and di-tert-butyl dicarbonate with vigorous stirring at a temperature preferably between 0°C and 40°C, more preferably between 0°C and 10°C.
Compound (X) can be obtained as a commercial product, or according to the methods described in, for example, J. Med. Chem., Vol. 25, p.1045 (1982); Synthesis, Vol. 28, p.615 (1990) and the like.

### Step 2

Compound (XII) can be prepared by the reaction of Compound (XI) obtained in Step 1 mentioned above with thiosemicarbazide in a suitable solvent.
Specifically, Compound (XII) can be prepared by dissolving Compound (XI) obtained in Step 1 mentioned above in a suitable solvent, adding dropwise a solution of thiosemicarbazide in aqueous hydrochloric acid preferably at a temperature between -10°C and 60°C, more preferably between 0°C and 20°C, stirring the mixture preferably at room temperature, for 5 minutes to 72 hours, preferably 30 minutes to 4 hours, and then for 30 minutes to 24 hours, preferably 30 minutes to 4 hours, under ice cooling, collecting deposited solid, washing and drying the resulting solid.
Examples of the solvent include, for example, hydrophilic solvents such as methanol, ethanol, propanol, 2-propanol, butanol, sec-butanol, tert-butanol, acetonitrile, dioxane, DMF, DMA, NMP and pyridine, non-hydrophilic solvents such as dichloromethane, chloroform, 1,2-dichloroethane, toluene, ethyl acetate, diethyl ether, THF and DME, water and the like, and they are used alone or as a mixture. Preferred examples include hydrophilic solvents or mixed solvents of a hydrophilic solvent and water, more preferred examples include methanol, ethanol, propanol, 2-propanol, butanol, sec-butanol, tert-butanol, mixed solvents of these and water and the like, and still more preferred examples include methanol, ethanol, mixed solvents of these and water and the like. A mixed solvent with water is most preferred, and a mixed solvent of methanol or ethanol and water (for example, 9:1 to 1:9, preferably 8:2 to 5:5, more preferably 7:3 to 6:4 (methanol or ethanol:water) is especially preferred. The amount of the solvent used is, for example, such an amount that the concentration of Compound (XI) should become 50 to 600 g/L, preferably 80 to 300 g/L, more preferably 100 to 200 g/L.

Thiosemicarbazide is preferably used in an amount of 1 to 5 equivalents, more preferably 1 to 3 equivalents, still more preferably 1.1 to 2.2 equivalents. Moreover, thiosemicarbazide is preferably used as an aqueous solution acidified with hydrochloric acid, and for example, is dissolved in, for example, 0.5 to 12 mol/L, preferably 0.5 to 6 mol/L, more preferably 2 to 3 mol/L of hydrochloric acid at a concentration of, for example, 100 g to 1 kg/L, preferably 150 to 300 g/L, more preferably 190 to 230 g/L, and used.
Furthermore, more preferably, by adding separately prepared crystals of Compound (XII), if needed, when 20 to 90%, preferably 30 to 80%, more preferably 40 to 60%, or total amount of thiosemicarbazide used was added, crystallization of Compound (XII) produced can be accelerated, and the reaction can be performed more efficiently. Depending on the reaction conditions, stability of Compound (XII) dissolved in the solvent may not be sufficient, and it is preferred that Compound (XII) produced should be immediately crystallized from the reaction solution.

Under the aforementioned preferred reaction conditions, the product (Compound (XII)) deposits as solid in the reaction mixture, and the deposited solid can be collected by, for example, filtration, or other techniques. Further, for washing of the resulting solid, for example, the solvent used for the reaction, water, mixed solvents of these and the like are used, and these washing solvents are preferably cooled before use. It is preferable to perform the washing with ice-cooled water or an ice-cooled mixed solvent of water and methanol (1:2 to 2:1, preferably 1:1).
Drying of the resulting solid is preferably performed, for example, at a temperature between 10°C and 60°C under reduced pressure for 30 minutes to 72 hours.

### Step 3

Compound (IA) can be prepared by the reaction of Compound (XII) with R³COX (wherein R³ and X have the same meaning as those mentioned above), or (R³CO)₂O (wherein R³ has the same meaning as that mentioned above) in a solvent in the presence of a base.
Specifically, for example, Compound (IA) can be prepared by adding Compound (XII) to a suitable solvent, slowly adding R³COX (wherein R³ and X have the same meaning as those mentioned above) or (R³CO)₂O (wherein R³ has the same meaning as that mentioned above) to the mixture in the presence of a base at a temperature preferably between 0°C and 30°C, and allowing them to react at a temperature preferably between 0°C and 60°C, more preferably between 5°C and 40°C, for 5 minutes to 72 hours, preferably 30 minutes to 10 hours. Compound (IA) can be isolated by preferably adding hydrochloric acid to the reaction mixture, removing the aqueous phase, if necessary, then adding water dropwise, collecting the deposited solid, washing and drying the resulting solid.

Examples of the solvent include, for example, hydrophilic solvents such as methanol, ethanol, acetone, methyl ethyl ketone, acetonitrile, propionitrile, dioxane, DMF, DMA, NMP and pyridine, non-hydrophilic solvents such as dichloromethane, chloroform, 1,2-dichloroethane, toluene, ethyl acetate, diethyl ether, THF and DME, water and the like, and they can be used alone or as a mixture. Preferred examples include hydrophilic solvents, more preferred examples include acetonitrile, propionitrile, acetone, methyl ethyl ketone, pyridine and the like, and still more preferred examples include acetonitrile. The amount of the solvent used is, for example, such an amount that the concentration of Compound (XII) should become 30 to 600 g/L, preferably 50 to 300 g/L, more preferably 80 to 120 g/L.
Examples of the base include, for example, potassium acetate, sodium hydrogencarbonate, potassium carbonate, potassium hydroxide, sodium hydroxide, sodium methoxide, potassium tert-butoxide, triethylamine, diisopropylethylamine, N-methylmorpholine, pyridine, DBU and the like, and preferred examples include pyridine and the like. The base is used in an amount of 2 to 12 equivalents, preferably 2.5 to 5 equivalents, to Compound (XII).

Examples of R³COX include, for example, R³COCl, R³COBr and the like, and the reagent is preferably used in an amount of 2 to 10 equivalents, more preferably 2.5 to 3.5 equivalents, to Compound (XII). (R³CO)₂O is preferably used in amount of 2 to 10 equivalents, more preferably 2.5 to 3.5 equivalents, to Compound (XII). These reagents are preferably added dropwise to a mixture of Compound(XII), the base and the solvent with stirring under ice cooling.
For obtaining the deposited solid, for example, filtration and other techniques can be used.
For washing of the resulting solid, for example, water or the solvent used for the reaction, a mixed solvent thereof or the like can be used, which are preferably cooled before use. It is preferable to wash the solid with a cooled mixed solvent of the solvent used for the reaction and water (30:1 to 1:1, preferably 15:1 to 5:1), and successively wash the same with cold water.
Drying of the resulting solid is preferably performed, for example, at a temperature between 10°C and 70°C under reduced pressure for 1 to 72 hours.

### Preparing method 6

Among Compound (II), Compound (IIA) wherein R¹ is a hydrogen atom, R² and R³, which are the same, represent lower alkyl, and R⁴ is tert-butoxycarbonylamino can also be prepared by using Compound (IA) obtained by Preparing method 5 or the like according to, for example, the method described in Preparing method 2. 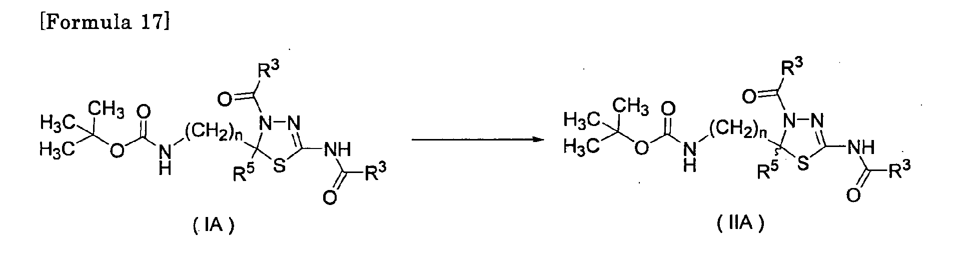 (wherein n, R³ and R⁵ have the same meaning as those mentioned above, respectively)

### Preparing method 7

Among Compounds (I) and (II), Compounds (IB) and (IIB) wherein R¹ is a hydrogen atom, R² and R³, which are the same, represent lower alkyl, and R⁴ is amino can also be prepared in accordance with the following step. 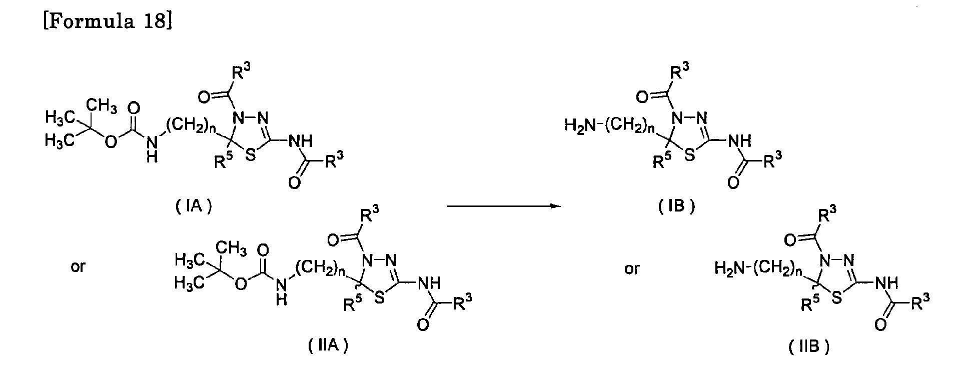 (wherein n, R³ and R⁵ have the same meanings as those mentioned above, respectively)

Compound (IB) or (IIB) can be prepared by treatment of Compound (IA) or (IIA) obtained by Preparing method 1, 2, 3, 5, 6 or the like with an appropriate acid.
Specifically, for example, hydrochloride of Compound (IB) or (IIB) can be prepared by dissolving Compound (IA) or (IIA) obtained by Preparing method 1, 2, 3, 5, 6 or the like in a suitable solvent, if necessary, and treating it with, for example, a solution containing hydrogen chloride. The treatment is preferably performed at a temperature between 0°C to 60°C, more preferably between 5°C and 40°C, for 5 minutes to 72 hours, more preferably 1 to 12 hours, and further stirring for 10 minutes to 4 hours under ice cooling, if necessary. Hydrochloride of Compound (IB) or (IIB) is preferably isolated by, for example, collecting solid deposited in the mixture, washing and drying the solid, if necessary.
Examples of the solution containing hydrogen chloride include, for example, a solution dissolving hydrogen chloride at a concentration of, for example, 1 to 12 mol/L, preferably 1 to 8 mol/L, more preferably 2 to 6 mol/L, in methyl acetate, ethyl acetate, propyl acetate, isopropyl acetate, butyl acetate, methanol, ethanol, dioxane or the like. Preferred examples include, for example, a solution dissolving hydrogen chloride at a concentration of, for example, 1 to 12 mol/L, preferably 1 to 8 mol/L, more preferably 2 to 6 mol/L, in a solvent such as methyl acetate, ethyl acetate, propyl acetate, isopropyl acetate, or butyl acetate, more preferably ethyl acetate, and particularly preferred are 4 mol/L hydrogen chloride in ethyl acetate and the like.

Examples of the solvent for dissolving Compound (IA) or (IIA) include, for example, the same solvents as those for the aforementioned solution containing hydrogen chloride, and specific preferred examples include ethyl acetate and the like.
As the method for obtaining the solid, for example, filtration and other techniques can be used.
Washing of the resulting solid is preferably performed by using the same cooled solvent as that used for the aforementioned solution containing hydrogen chloride, specifically, preferably by using cold ethyl acetate or the like.
Drying of the resulting solid is performed, for example, preferably at a temperature between 10°C and 120°C, more preferably 20°C and 100°C, still more preferably 30°C and 80°C, for 1 to 72 hours, preferably 1 to 24 hours, under reduced pressure.

### Preparing method 8

Among Compound (I), Compounds (ICa), (ICb) and (ICc) wherein R⁴ is NHSO₂R⁶ (wherein R⁶ has the same meaning as that mentioned above), NHR^{7C} (wherein R^{7C} represents lower alkyl which may have 1 or 2 substituents selected from the group consisting of hydroxy, lower alkoxyl, amino, (lower alkyl)amino and di-(lower alkyl)amino, among the groups defined for R⁷), or NHCOR⁸ (wherein R⁸ has the same meaning as that mentioned above) can also be prepared in accordance with the following steps. 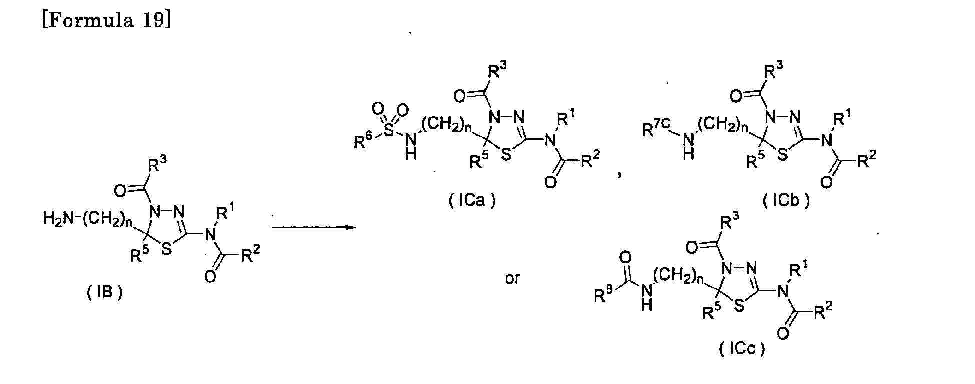 (wherein n, R¹, R², R³, R⁵, R⁶, R^{7C}, and R⁸ have the same meanings as those mentioned above, respectively)
Compound (ICa) can be prepared by the reaction of Compound (IB) obtained by Preparing method 1, 2, 4, 7 or the like with 1 to 20 equivalents, preferably 1 to 5 equivalents, of R⁶SO₂X (wherein R⁶ and X have the same meanings as those mentioned above, respectively), or (R⁶SO₂)₂O (wherein R⁶ has the same meaning as that mentioned above) in a suitable solvent in the presence of 0.5 to 20 equivalents, preferably 1 to 5 equivalents of a base, if necessary, at a temperature between -20°C and 150°C, preferably -10°C and 30°C, for 5 minutes to 72 hours.

Examples of the solvent include, for example, dichloromethane, chloroform, 1,2-dichloroethane, toluene, ethyl acetate, acetonitrile, diethyl ether, THF, DME, dioxane, DMF, DMA, NMP, pyridine and the like, and they can be used alone or as a mixture.
Examples of the base include, for example, sodium hydrogencarbonate, potassium carbonate, potassium hydroxide, sodium hydroxide, sodium methoxide, potassium tert-butoxide, triethylamine, diisopropylethylamine, N-methylmorpholine, pyridine, DBU and the like.
Compound (ICb) can be obtained by the reaction of Compound (IB) obtained by Preparing method 1, 2, 4, 7 or the like with 1 to 20 equivalents of R^{7C}X (wherein R^{7C} and X have the same meanings as those mentioned above, respectively) in a suitable solvent in the presence of 0.5 to 20 equivalents of a base, if necessary, at a temperature between -20°C and 150°C for 5 minutes to 72 hours.
Examples of the solvent include, for example, dichloromethane, chloroform, 1,2-dichloroethane, toluene, ethyl acetate, acetonitrile, diethyl ether, THF, DME, dioxane, DMF, DMA, NMP, pyridine and the like, and they can be used alone or as a mixture.
Examples of the base include, for example, sodium hydrogencarbonate, potassium carbonate, potassium hydroxide, sodium hydroxide, sodium methoxide, potassium tert-butoxide, triethylamine, diisopropylethylamine, N-methylmorpholine, pyridine, DBU and the like.

As an alternative method, Compound (ICb) can be prepared by the reaction of Compound (IB) obtained by Preparing method 1, 2, 4, 7 or the like with preferably 1 to 20 equivalents, more preferably 1 to 5 equivalents, of a ketone or aldehyde corresponding to R^{7C} (for example, formaldehyde when R^{7C} is methyl, acetaldehyde when R^{7C} is ethyl, acetone when R^{7C} is isopropyl, and the like) in a suitable solvent in the presence of preferably 1 to 20 equivalents, more preferably 1 to 5 equivalents of a reducing agent, and preferably 1 to 20 equivalents, more preferably 1 to 5 equivalents of an acid at a temperature between -20°C and 150°C for 5 minutes to 72 hours.
Examples of the reducing agent include, for example, sodium borohydride, sodium triacetoxyborohydride, sodium cyanoborohydride and the like.
Examples of the acid include, for example, hydrochloric acid, acetic acid, trifluoroacetic acid and the like.
Examples of the solvent include, for example, methanol, ethanol, dichloromethane, chloroform, 1,2-dichloroethane, toluene, ethyl acetate, acetonitrile, diethyl ether, THF, DME, dioxane, DMF, DMA, NMP, water and the like, and they can be used alone or as a mixture.

Compound (ICc) can be obtained by the reaction of Compound (IB) obtained by Preparing method 1, 2, 4, 7 or the like with 1 to 20 equivalents of R⁸COX (wherein R⁸ and X have the same meanings as those mentioned above, respectively) or (R⁸CO)₂O (wherein R⁸ has the same meaning as that mentioned above) without solvent or in a suitable solvent in the presence of 0.5 to 20 equivalents of a base, if necessary, at a temperature between -20°C and 150°C for 5 minutes to 72 hours.
Examples of the solvent include, for example, dichloromethane, chloroform, 1,2-dichloroethane, toluene, ethyl acetate, acetonitrile, diethyl ether, THF, DME, dioxane, DMF, DMA, NMP, pyridine and the like, and they can be used alone or as a mixture.
Examples of the base include, for example, sodium hydrogencarbonate, potassium carbonate, potassium hydroxide, sodium hydroxide, sodium methoxide, potassium tert-butoxide, triethylamine, diisopropylethylamine, N-methylmorpholine, pyridine, DBU and the like.
By performing the same procedures as those mentioned above using Compound (IIB) obtained by Preparing method 2, 7 or the like instead of Compound (IB), Compounds (ICa) and (ICb) having the same configuration as that of Compound (IIB) can be obtained.

### Preparing method 9

Among Compound (I), Compound (ID) wherein R⁴ is NHSO₂CH₂CH₂R^{4B} (wherein R^{4B} represents amino, hydroxyamino, (lower alkyl)amino, di-(lower alkyl)amino, N-hydroxy(lower alkyl)amino, amino-substituted (lower alkyl)thio, (lower alkyl)amino-substituted (lower alkyl)thio or di-(lower alkyl)amino-substituted (lower alkyl)thio among the substituents of the lower alkyl defined for R⁶) can also be prepared in accordance with the following steps. 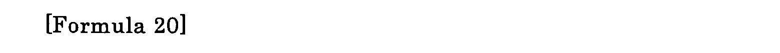 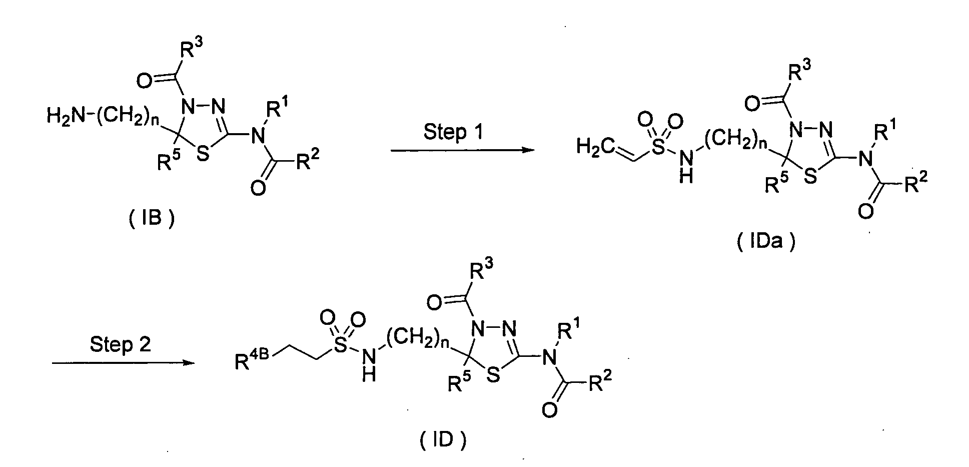 (wherein n, R¹, R², R³, R⁵ and R^{4B} have the same meanings as those mentioned above, respectively)

### Step 1

Compound (IDa) can be prepared by the reaction of Compound (IB) obtained by Preparing method 1, 2, 4, 7 or the like with 1 to 20 equivalents, preferably 1 to 5 equivalents of ClCH₂CH₂SO₂Cl without solvent or in a suitable solvent in the presence of preferably 1 to 20 equivalents of a base, if necessary, at a temperature between -20°C and 150°C, preferably -10°C and 30°C, for 5 minutes to 72 hours, preferably 5 minutes to 5 hours. Compound (IB) can also preferably be used as an acid addition salt such as hydrochloride, and in such a case, the base is preferably used in an amount of 2 equivalents or more.
Examples of the solvent include, for example, dichloromethane, chloroform, 1,2-dichloroethane, toluene, ethyl acetate, acetonitrile, diethyl ether, THF, DME, dioxane, DMF, DMA, NMP, N,N'-dimethylimidazolidinone (DMI), pyridine and the like, and they can be used alone or as a mixture. Ethyl acetate, acetonitrile and the like are particularly preferred.
Examples of the base include, for example, sodium hydrogencarbonate, potassium carbonate, potassium hydroxide, sodium hydroxide, sodium methoxide, potassium tert-butoxide, triethylamine, diisopropylethylamine, N-methylmorpholine, pyridine, N-methylpiperidine, N,N'-dimethylpiperazine, DBU and the like.

### Step 2

Compound (ID) can be prepared by the reaction of Compound (IDa) obtained in Step 1 mentioned above with 1 equivalent to large excess amount, preferably 5 to 100 equivalents, more preferably 10 to 20 equivalents of R^{4C}R^{4D}NH (wherein R^{4C} and R^{4D} are the same or different, and represent a hydrogen atom, hydroxy or the lower alkyl moiety in the lower alkylamino, di-(lower alkyl)amino or N-hydroxy(lower alkyl)amino among the substituents of the lower alkyl defined for R⁶), or R^{4E}SH (wherein R^{4E} represents amino-substituted lower alkyl, (lower alkyl)amino-substituted lower alkyl, and di-(lower alkyl)amino-substituted lower alkyl in the amino-substituted (lower alkyl)thio, the (lower alkyl)amino-substituted (lower alkyl)thio and the di-(lower alkyl)amino-substituted (lower alkyl)thio among the substituents of the lower alkyl defined for R⁶) without solvent or in a suitable solvent in the presence of 1 to 10 equivalent a base, if necessary, at a temperature between -10°C and 150°C, preferably -10°C and 40°C, for 5 minutes to 72 hours.
Examples of the solvent include, for example, methanol, ethanol, propanol, 2-propanol, butanol, dichloromethane, chloroform, 1,2-dichloroethane, toluene, ethyl acetate, acetonitrile, diethyl ether, THF, DME, dioxane, DMF, DMA, NMP, pyridine, water and the like, and they can be used alone or as a mixture. Methanol, ethanol and the like and a mixed solvent of these and water are preferred.
Examples of the base include, for example, sodium hydrogencarbonate, potassium carbonate, potassium hydroxide, sodium hydroxide, sodium methoxide, potassium tert-butoxide, triethylamine, diisopropylethylamine, N-methylmorpholine, pyridine, DBU and the like.

Among Compounds (I) and (II), stereoisomers such as geometrical isomers and optical isomers, regioisomers, tautomers and the like may be existed. Including these isomers, all possible isomers and the mixtures thereof can be used for the therapeutic and/or prophylactic agent for a solid tumor of the present invention, and all these possible isomers and the mixtures thereof fall within the scope of the thiadiazoline derivative of the present invention.
To obtain a salt of Compound (I) or (II), when Compound (I) or (II) is obtained as a salt form, the salt, per se, may be purified. When Compound (I) or (II) is obtained as a free form, Compound (I) or (II) may be dissolved or suspended in an appropriate solvent, and added an appropriate acid or base to form a salt, and then be isolated and purified.
In addition, Compound (I) or (II) or a pharmaceutically acceptable salt thereof may exist in the form of adducts with water or various solvents. These adducts can also be used for the therapeutic and/or prophylactic agent for a solid tumor of the present invention, and fall within the scope of the thiadiazoline derivative of the present invention.
Specific examples of Compounds (I) and (II) are shown in Tables 1 and 2. However, Compounds (I) and (II) are not limited to these examples.

[Table 1]

**Table 1**

| | | | | | | |
|---|---|---|---|---|---|---|
| 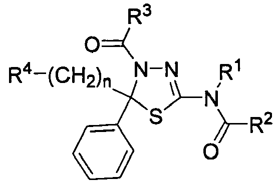 | | | | | | |

| Ref. Ex. No. | Compound No. | n | R¹ | R² | R³ | R⁴ |
|---|---|---|---|---|---|---|
| 1 | 1 | 3 | H | C(CH₃)₃ | C(CH₃)₃ | NHCH₂CH₂CH₂OH |
| 2 | 2 | 3 | H | C(CH₃)₃ | C(CH₃)₃ | NHCH₂CH₂N(CH₃)₂ |
| 3 | 3 | 2 | H | C(CH₃)₃ | C(CH₃)₃ | NHSO₂CH₃ |
| 4 | 4 | 2 | H | C(CH₃)₃ | CH₂CH₃ | NHSO₂CH₃ |
| 5 | 5 | 2 | CH₂CH₂CH₂ | | C(CH₃)₃ | NHSO₂CH₃ |
| 6 | 6 | 2 | H | C(CH₃)₃ | CH(CH₃)₂ | NHSO₂CH₃ |
| 7 | 7 | 2 | CH₂CH₂CH₂ | | CH₂CH₃ | NHSO₂CH₃ |
| 8 | 8 | 3 | H | C(CH₃)₃ | C(CH₃)₃ | CONHCH₂CH₂OH |
| 9 | 9 | 1 | H | C(CH₃)₃ | C(CH₃)₃ | NHSO₂CH₂CH₂NHCH₂CH₃ |
| 10 | 10 | 1 | H | C(CH₃)₃ | C(CH₃)₃ | NHSO₂CH=CH₂ |
| 11 | 11 | 1 | H | C(CH₃)₃ | C(CH₃)₃ | NH₂ |
| 12 | 12 | 1 | H | C(CH₃)₃ | C(CH₃)₃ | NHSO₂CH₂CH₂N(CH₃)₂ |
| 13 | 13 | 1 | H | C(CH₃)₃ | C(CH₃)₃ | NHSO₂CH₂CH₂CH₂N(CH₃)₂ |
| 14 | 14 | 2 | H | C(CH₃)₃ | C(CH₃)₃ | NHSO₂CH₂CH₂NHCH₂CH₃ |
| 15 | 15 | 1 | H | C(CH₃)₃ | C(CH₃)₃ | NHSO₂CH₂CH₂NHOH |
| 16 | 16 | 1 | H | C(CH₃)₃ | C(CH₃)₃ | NHSO₂CH₂CH₂N(OH)CH₂CH₃ |
| 17 | 17 | 1 | H | C(CH₃)₃ | C(CH₃)₃ | NHSO₂CH₂CH₂SCH₂CH₂NH₂ |
| 18 | 18 | 1 | H | C(CH₃)₃ | C(CH₃)₃ | NHSO₂CH₂SCH₂CH₂NH₂ |
| 19 | 19 | 2 | CH₂CH₂CH₂CH₂ | | CH₃ | NHSO₂CH₃ |

[Table 2]

**Table 2**

| | | | | | | |
|---|---|---|---|---|---|---|
| 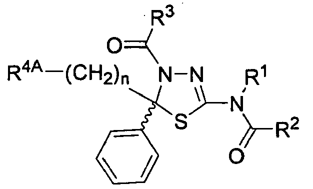 | | | | | | |

| Ex. No. | Compound No. | n | R¹ | R² | R³ | R^{4A} |
|---|---|---|---|---|---|---|
| 15 | a | 2 | H | C(CH₃)₃ | C(CH₃)₃ | NHSO₂CH₃ |
| 16 | b | 2 | H | C(CH₃)₃ | CH₂CH₃ | NHSO₂CH₃ |
| 17 | c | 2 | CH₂CH₂CH₂ | | C(CH₃)₃ | NHSO₂CH₃ |
| 18 | d | 2 | H | C(CH₃)₃ | CH(CH₃)₂ | NHSO₂CH₃ |
| 19 | e | 2 | CH₂CH₂CH₂ | | CH₂CH₃ | NHSO₂CH₃ |
| 20 | f | 2 | H | C(CH₃)₃ | CH₃ | NHSO₂CH₃ |
| 21* | g | 2 | CH₂CH₂CH₂ | | CH₃ | NHSO₂CH₃ |
| 22 | h | 2 | CH₂CH₂CH₂CH₂ | | CH₃ | NHSO₂CH₃ |
| 23* | i | 2 | H | C(CH₃)₃ | C(CH₃)₃ | NHSO₂CH₂CH₂NHCH₂CH₃ |
| 24* | j | 1 | H | C(CH₃)₃ | C(CH₃)₃ | NH₂ |
| 25* | k | 1 | H | C(CH₃)₃ | C(CH₃)₃ | NHSO₂CH=CH₂ |
| 26 | l | 1 | H | C(CH₃)₃ | C(CH₃)₃ | NHSO₂CH₂CH₂NHCH₂CH₃ |
| 27 | m | 1 | H | C(CH₃)₃ | C(CH₃)₃ | NHSO₂CH₂CH₂N(CH₃)₂ |
| 28* | p | 1 | H | C(CH₃)₃ | C(CH₃)₃ | NHSO₂CH₂CH₂CH₂NH₂ |
| 29 | n | 1 | H | C(CH₃)₃ | C(CH₃)₃ | NHSO₂CH₂CH₂CH₂N(CH₃)₂ |
| 30* | o | 3 | H | C(CH₃)₃ | C(CH₃)₃ | CONHCH₂CH₂OH |
| 32 | q | 1 | H | C(CH₃)₃ | C(CH₃)₃ | NHCOOC(CH₃)₃ |

| | | | | | | |
|---|---|---|---|---|---|---|
| *Specific rotation was not determined. | | | | | | |

Next, pharmacological activities of Compounds (I) and (II) will be specifically explained by the following test examples.

### Test Example 1: Cell growth inhibition tests against lung cancer cells, ovarian cancer cells and colon cancer cells

As cancer cell lines, human lung cancer A549 cells (ATCC No. CCL-185), human ovarian cancer SK-OV-3 cells (ATCC No. HTB-77), and human colon cancer HCT 116 cells (ATCC No. CCL-247) were used. For the culture of A549 cells, Nutrient Mixture F-12K medium (Invitrogen, catalog No. 21127-022) containing 10% fetal bovine serum (Invitrogen, catalog No. 10099-141), 100 units/mL penicillin (Invitrogen, catalog No. 15140-122), and 100 µg/mL streptomycin (Invitrogen, catalog No. 15140-122) was used. For the culture of SK-OV-3 cells and HCT 116 cells, McCoy's 5A medium (Invitrogen, catalog No. 16600-082) containing 10% fetal bovine serum (Invitrogen, catalog No. 10099-141), 100 units/mL penicillin (Invitrogen, catalog No. 15140-122), and 100 µg/mL streptomycin (Invitrogen, catalog No. 15140-122) was used. The cells were cultured at 37°C in a 5% carbon dioxide atmosphere.

A549 cells (1000 cells/well), SK-OV-3 cells (2000 cells/well), or HCT 116 cells (1000 cells/well) were seeded in each well of 96-well plates (Nunc, catalog No. 167008), and cultured overnight. Test compounds diluted stepwise were added, and the cells were further cultured for 72 hours (final volume: 100 µL/well). Fifty µL XTT labeling mixture of Cell Proliferation Kit II (XTT) (Roche Diagnostics, catalog No. 1465015) was added to each well, and the plates were incubated at 37°C. After 1 to 3 hours, absorbance at 490 nm (reference wavelength: 655 nm) was measured with a plate reader (Molecular Device, SpectraMax 340PC³⁸⁴). Growth ratios of the cells in the wells treated with the test compounds were calculated based on the growth ratio of the cells in the control well treated with solvent (dimethyl sulfoxide (DMSO)) for 72 hours, which was defined as 100%. From a plot of test compound concentrations and the cell growth ratios at the concentrations, the concentration of 50% growth inhibition, the GI₅₀ value, was calculated.

Compounds a, b, d, e, h, i, j, l, m, n and o showed growth inhibitory activities less than 0.1 µmol/L in terms of the GI₅₀ value against the human colon cancer cell line HCT 116. Compounds 1, 2, a, b, d, e, h, i, j, l, m, n and o showed growth inhibitory activities less than 10 µmol/L in terms of the GI₅₀ value against the human lung cancer cell line A549 and the human ovarian cancer cell line SK-OV-3. From these results, it is considered that Compounds (I) and (II) show cell growth inhibitory activity against human lung cancer cells, human ovarian cancer cells, and human colon cancer cells, namely, they are useful as therapeutic and/or prophylactic agents for lung cancer, ovarian cancer, and colon cancer.

### Test Example 2: Cell growth inhibition tests against pancreatic cancer cells, cervical cancer cells and breast cancer cells

Cell growth inhibitory activities of Compounds (I) and (II) against pancreatic cancer cells, cervical cancer cells and breast cancer cells can be measured in the same manner as that of Test Example 1 by using the human pancreatic cancer cell line MIA PaCa-2 (ATCC No. CRL-1420), the human cervical cancer cell line HeLa (ATCC No. CCL-2) and the human breast cancer cell line T-47D (ATCC No. HTB-133). That is, it can be confirmed that Compounds (I) and (II) are useful as therapeutic and/or prophylactic agents for pancreatic cancer, uterine cancer and breast cancer.
From the above, it is considered that Compounds (I) and (II) are useful as therapeutic and/or prophylactic agents for tumors of chest, digestive organs, female genital organs and the like, i.e., solid tumors.

### Test Example 3: Cell growth inhibition tests against pancreatic cancer cells, cervical cancer cells, breast cancer cells, prostate cancer cells, skin cancer cells, head and neck cancer cells, renal cancer cells and liver cancer cells

As cancer cell lines, human pancreatic cancer MIA PaCa-2 cells (JCRB No. 0070), human cervical cancer HeLa cells (ATCC No. CCL-2), human breast cancer MDA-MB-468 cells (ATCC No. HTB-132), human prostate cancer DU 145 cells (ATCC No. HTB-81), human skin cancer SK-MEL-28 cells (ATCC No. HTB-72), human head and neck cancer KB cells (JCRB No. 9027), human renal cancer 786-O cells (ATCC No. CRL-1932), and human liver cancer Hep G2 cells (ATCC No. HB-8065) were used. The cells were cultured at 37°C in a 5% carbon dioxide atmosphere (except for the human breast cancer MDA-MB-468 cells, which were cultured under the condition at 37°C) by using the mediums mentioned below, respectively.

[Table 3]

**Table 3**

| Cell | Medium |
|---|---|
| Human pancreatic cancer MIA PaCa-2 cell | Minimum Essential Medium (Invitrogen, catalog No. 11095-080) containing 10% fetal bovine serum (Invitrogen, catalog No. 10099-141), 0.1 mmol/L MEM Non-Essential Amino Acids Solution (Invitrogen, catalog No. 11140-050), 100 units/mL penicillin (Invitrogen, catalog No. 15140-122) and 100 µg/mL streptomycin (Invitrogen, catalog No. 15140-122) |
| Human cervical cancer HeLa cell | Minimum Essential Medium (Invitrogen, catalog No. 11095-080) containing 10% fetal bovine serum (Invitrogen, catalog No. 10099-141), 0.1 mmol/L MEM Non-Essential Amino Acids Solution (Invitrogen, catalog No. 11140-050), 100 units/mL penicillin (Invitrogen, catalog No. 15140-122) and 100 µg/mL streptomycin (Invitrogen, catalog No. 15140-122) |
| Human breast cancer MDA-MB-468 cell | Leibovitz's L-15 Medium (Invitrogen, catalog No. 11415-064) containing 10% fetal bovine serum (Invitrogen, catalog No. 10099-141), 100 units/mL penicillin (Invitrogen, catalog No. 15140-122) and 100 µg/mL streptomycin (Invitrogen, catalog No. 15140-122) |
| Human prostate cancer DU 145 cell | Minimum Essential Medium (Invitrogen, catalog No. 11095-080) containing 10% fetal bovine serum (Invitrogen, catalog No. 10099-141), 0.1 mmol/L MEM Non-Essential Amino Acids Solution (Invitrogen, catalog No. 11140-050), 1 mmol/L Sodium Pyruvate Solution (Invitrogen, catalog No. 11360-070), 100 units/mL penicillin (Invitrogen, catalog No. 15140-122) and 100 µg/mL streptomycin (Invitrogen, catalog No. 15140-122) |
| Human skin cancer SK-MEL-28 cell | Minimum Essential Medium (Invitrogen, catalog No. 11095-080) containing 10% fetal bovine serum (Invitrogen, catalog No. 10099-141), 0.1 mmol/L MEM Non-Essential Amino Acids Solution (Invitrogen, catalog No. 11140-050), 100 units/mL penicillin (Invitrogen, catalog No. 15140-122) and 100 µg/mL streptomycin (Invitrogen, catalog No. 15140-122) |
| Human head and neck cancer KB cell | Minimum Essential Medium (Invitrogen, catalog No. 11095-080) containing 10% fetal bovine serum (Invitrogen, catalog No. 10099-141), 0.1 mmol/L MEM Non-Essential Amino Acids Solution (Invitrogen, catalog No. 11140-050), 100 units/mL penicillin (Invitrogen, catalog No. 15140-122) and 100 µg/mL streptomycin (Invitrogen, catalog No. 15140-122) |
| Human renal cancer 786-O cell | RPMI 1640 Medium (Invitrogen, catalog No. 11875-093) containing 10% fetal bovine serum (Invitrogen, catalog No. 10099-141), 10 mmol/L HEPES Buffer Solution (Invitrogen, catalog No. 15630-080), 1 mmol/L Sodium Pyruvate Solution (Invitrogen, catalog No. 11360-070), 4.5 g/L D-(+)-Glucose Solution (Sigma, catalog No. G8769), 100 units/mL penicillin (Invitrogen, catalog No. 15140-122) and 100 µg/mL streptomycin (Invitrogen, catalog No. 15140-122) |
| Human liver cancer Hep G2 cell | Minimum Essential Medium (Invitrogen, catalog No. 11095-080) containing 10% fetal bovine serum (Invitrogen, catalog No. 10099-141), 0.1 mmol/L MEM Non-Essential Amino Acids Solution (Invitrogen, catalog No. 11140-050), 1 mmol/L Sodium Pyruvate Solution (Invitrogen, catalog No. 11360-070), 100 units/mL penicillin (Invitrogen, catalog No. 15140-122) and 100 µg/mL streptomycin (Invitrogen, catalog No. 15140-122) |

In the same manner as that in Test Example 1, the cells were seeded (500 to 4000 cells/well, respectively) in each well of 96-well plates (Nunc, catalog No. 167008), and growth ratios of the cells treated with test compounds were calculated. The measurement of absorbance was performed at 1.5 to 3 hours after the addition of the XTT labeling mixture. From a plot of test compound concentrations and the cell growth ratios at the concentrations, the concentration of 50% growth inhibition, the GI₅₀ value, was calculated.
As the results, (1) Compounds 1, 2, a, b, d, e, h, i, l, m, n and o showed growth inhibitory activities less than 10 µmol/L in terms of the GI₅₀ value against the human pancreatic cancer MIA PaCa-2 cells, (2) Compounds 1, 2, a, b, d, e, h, i, l, m, n and o showed growth inhibitory activities less than 10 µmol/L in terms of the GI₅₀ value against the human cervical cancer HeLa cells, (3) Compounds 1, 2, a, b, d, e, h, i, l, m, n and o showed growth inhibitory activities less than 10 µmol/L in terms of the GI₅₀ value against the human breast cancer MDA-MB-468 cells, (4) Compounds 1, 2, a, b, d, e, h, i, l, m, n and o showed growth inhibitory activities less than 10 µmol/L in terms of GI₅₀ value against the human prostate cancer DU 145 cells, (5) Compounds 1, 2, a, b, d, e, h, i, l, m, n and o showed growth inhibitory activities less than 10 µmol/L in terms of GI₅₀ value against the human skin cancer SK-MEL-28 cells, (6) Compounds 1, 2, a, b, d, e, h, i, l, m, n and o showed growth inhibitory activities less than 10 µmol/L in terms of GI₅₀ value against the human head and neck cancer KB cells, (7) Compounds 1, 2, a, b, d, e, h, i, l, m, n and o showed growth inhibitory activities less than 10 µmol/L in terms of GI₅₀ value against the human renal cancer 786-O cells, and (8) Compounds 1, 2, a, d, e, i, l, m, n and o showed growth inhibitory activities less than 10 µmol/L in terms of GI₅₀ value against the human liver cancer Hep G2 cells.

From these results, it was considered that Compounds (I) and (II) had cell growth inhibitory activity against human pancreatic cancer cells, human cervical cancer cells, human breast cancer cells, human prostate cancer cells, human skin cancer cells, human head and neck cancer cells, human renal cancer cells, and human liver cancer cells. Namely, it is considered that Compounds (I) and (II) are useful as therapeutic and/or prophylactic agents for pancreatic cancer, cervical cancer, breast cancer, prostate cancer, skin cancer, head and neck cancer, renal cancer, and liver cancer.
From the above, it is considered that Compounds (I) and (II) are useful as therapeutic and/or prophylactic agents against tumors of chest, gastrointestinal organs, female genitalia, male genitalia, urinary organs, head and neck, skin and the like, i.e., solid tumors.

### Test Example 4: Eg5 enzyme inhibition test

A recombinant human Eg5 motor domain protein was prepared by referring to the literature [Biochemistry, Vol. 35, p.2365 (1996)]. A plasmid expressing the motor domain of human Eg5 was constructed, and transformed into *Escherichia coli* BL21 (DE3). The transformant was cultured at 25°C, and when the OD₆₀₀ reached 0.74, isopropyl-β-D-thiogalactoside was added at a final concentration of 0.5 mmol/L. The transformant was further cultured for 4 hours, and then the culture medium was centrifuged to collect the cells. The cells were suspended in a buffer and ultrasonicated, and then the sonicated solution was centrifuged to recover the supernatant. The supernatant was purified by cation exchange column chromatography to obtain a partially purified sample. Furthermore, the partially purified sample was purified by gel filtration column chromatography to obtain a finally purified sample.

Measurement of the ATPase activity of Eg5 was carried out by referring to the literatures [EMBO Journal, Vol. 13, p.751 (1994); Proc. Natl. Acad. Sci. USA, Vol. 89, p.4884 (1992)]. The following two kinds of solutions were prepared: Solution A consisting of 25 mmol/L piperazine N,N'-bis(ethanesulfonate) (PIPES)/KOH (pH 6.8), 1 mmol/L ethylene glycol-bis(2-aminoethyl ether)tetraacetic acid (EGTA), 2 mmol/L MgCl₂, 1 mmol/L dithiothreitol (DTT), 5 µmol/L paclitaxel, 167 µg/mL bovine serum albumin (BSA), 41.7 µg/mL tubulin (Cytoskeleton, Catalog No. TL238), 333 µmol/L MESG substrate (2-amino-6-mercapto-7-methylpurine riboside) (Molecular Probes, Catalog No. E-6646), 1.67 U/mL purine nucleoside phosphorylase (Molecular Probe, Catalog No. E-6646) and 1.33 µg/mL of the human Eg5 motor domain purified sample, and Solution B consisting of 25 mmol/L piperazine N,N'-bis(ethanesulfonate) (PIPES)/KOH (pH 6.8), 1 mmol/L ethylene glycol-bis(2-aminoethyl ether)tetraacetic acid (EGTA), 2 mmol/L MgCl₂, 1 mmol/L dithiothreitol (DTT), 5 µmol/L paclitaxel and 2.5 mmol/L ATP. Solution A was dispensed into each well of a 96-well plate as 45 µL portions. Solution B was used to serially dilute a test compound. The diluted test compound solutions in a volume of 30 µL were mixed with Solution A added beforehand in each well of the 96-well plate to start the enzymatic reaction. The enzymatic reaction was performed at 30°C for 30 minutes. Absorbance at 360 nm, which serves as an index of the ATPase activity, was measured using a plate reader (Molecular Device, SpectraMax 340PC³⁸⁴). The absorbance observed in the presence of Eg5 and absence of the test compound was defined 100%, and the absorbance observed in the absence of both Eg5 and the test compound was defined 0%. The relative activity was calculated to calculate IC₅₀ value.

Compounds 3, 4, 6, 7, 20, 14, 9, 8, a, b, d, e, h, i, l, o and the like inhibited the ATPase activity of Eg5 in a concentration-dependent manner. Inhibition ratios (IC₅₀) of Compound a, b, d, e, h, i, l, o and the like on the ATPase activity of Eg5 were less than 0.1 µmol/L. These compounds showed stronger inhibitory activities compared with those of Compounds 3, 4, 6, 7, 20, 14, 9, 8 and the like, which are respectively corresponding racemic mixtures. That is, it was considered that Compound (II) showing a negative value as a specific rotation in methanol at 20°C for sodium D line (wavelength: 589.3 nm) showed more potent inhibition on Eg5 than that of the racemic mixture thereof, and therefore it was suggested that such a compound showed stronger antitumor activity.

Compound (I) or (II), or a pharmaceutically acceptable salt thereof can be administered alone. However, usually, Compound (I) or (II), or a pharmaceutically acceptable salt thereof is preferably provided in various pharmaceutical preparations. Furthermore, these pharmaceutical preparations are used for animals and humans.
The pharmaceutical preparations according to the present invention may comprise Compound (I) or (II), or a pharmaceutically acceptable salt thereof alone as an active ingredient. Alternatively, the pharmaceutical preparations may comprise a mixture of Compound (I) or (II), or a pharmaceutically acceptable salt thereof with other arbitrary medicinal ingredient(s). Furthermore, these pharmaceutical preparations are prepared by mixing the active ingredient(s) with one or more pharmaceutically acceptable carrier(s) and then employing any method well-known in the technical field of pharmaceutics.
As for administration routes, it is preferred to select the most effective route of administration. Examples of the administration routes include oral administration and parenteral administration such as intravenous administration and the like.
As for the dosage form, for example, tablets, injections and the like are included.
For example, the tablet suitable for oral administration can be prepared with, for example, excipients such as lactose and mannitol; disintegrants such as starch; lubricants such as magnesium stearate; binders such as hydroxypropylcellulose; surfactants such as a fatty acid ester; plasticizers such as glycerol; and the like.

Preparations suitable for parenteral administration preferably comprise a sterilized aqueous preparation containing the active compound and being isotonic to blood of a recipient. For example, when an injection is prepared, a solution for injection is prepared by using a carrier consisting of a salt solution, glucose solution, a mixture of salt solution and glucose solution, or the like.
Also in these parenteral preparations, one or more kinds of auxiliary components selected from excipients, disintegrants, lubricants, binders, surfactants, plasticizers, diluents which are exemplified for the oral administration, preservatives, flavors and the like may be added.
Compound (I) or (II), or a pharmaceutically acceptable salt thereof is generally administered systemically or locally in the form of an oral or parenteral preparation when used for the aforementioned purpose. The dose and the frequency of administration may vary depending on the administration form, the age and body weight of a patient, nature and severity of the condition to be treated, and the like. When oral administration is performed, generally 0.01 to 1,000 mg/kg, preferably 0.05 to 500 mg/kg per single administration for an adult may be administered once a day or a few times a day, or once every several days to 1 or 2 weeks. When parenteral administration such as intravenous administration is performed, 0.001 to 1,000 mg/kg, preferably 0.01 to 300 mg/kg, per single administration for an adult may be administered once a day or a few times a day, or once every several days to 1 to 3 weeks. Examples of the administration method also include rapid intravenous injection, continuous intravenous administration for 1 to 24 hours a day, and the like. However, the dose and the frequency of administration may vary depending on the aforementioned various conditions and the like.

The therapeutic and/or prophylactic agent for a solid tumor of the present invention exhibits superior therapeutic and/or prophylactic effect for a solid tumor, and furthermore, Compound (I) or (II), or a pharmaceutically acceptable salt can be used also in combination with one or more kinds of other pharmaceutical ingredients as described above.
Examples of the other pharmaceutical ingredients used in combination include, for example, low molecular weight compounds, medicaments comprising proteins, nucleic acids or the like, and specific examples include the pharmaceutical ingredients described in Rinsho Shuyo-Gaku (Clinical Oncology), 3rd edition, edited by Japanese Society of Medicinal Oncology (2003) and the like.

Examples of the low molecular weight compounds include, for example, DNA alkylating agents (for example, cyclophosphamide, ifosfamide, melphalan, dacarbazine, procarbazine, nimustine, carmustine, lomustine, estramustine, busulfan, thiotepa and the like); DNA synthesis inhibitors (for example, bleomycin, peplomycin, mitomycin C, mitoxantrone, actinomycin D and the like); platinum preparation type DNA crosslinking agents (for example, cisplatin, carboplatin, oxaliplatin, nedaplatin and the like); antimetabolites (for example, 5-fluorouracil, tegafur, capecitabine, methotrexate, gemcitabine, fludarabine, cytarabine, cladribine, mercaptopurine, hydroxycarbamide and the like); topoisomerase I inhibitors (for example, irinotecan, topotecan, nogitecan and the like); topoisomerase II inhibitors (for example, doxorubicin, daunorubicin, epirubicin, etoposide and the like); tubulin agonists (for example, vincristine, vinblastine, vindesine, vinorelbine, paclitaxel, docetaxel, epothilone and the like); hormone antagonists (for example, tomoxifen, goserelin, leuprorelin, flutamide and the like); aromatase inhibitors (for example, anastrozole, fadrozole, letrozole, exemestane and the like); immunomodulators (for example, gold thiomalate, D-penicillamine, bucillamine, thalidomide and the like); immunosuppressants (for example, azathioprine, mizoribine, ciclosporin and the like); steroidal antiinflammatory agents (for example, hydrocortisone, prednisolone, dexamethasone and the like); non-steroidal anti-inflammatory agents (for example, aspirin, indomethacin, celecoxib and the like); antihistamines (for example, chlorpheniramine, clemastine and the like); differentiation inducers (for example, tretinoin, bexarotene, arsenic and the like); proteasome inhibitors (for example, bortezomib and the like); ubiquitin ligase inhibitors [for example, Nutlin (Science, Vol. 303, p.844 (2004)) and the like]; tyrosine kinase inhibitors {for example, EGFR inhibitors (for example, gefitinib, erlotinib and the like), Abl inhibitors (for example, imatinib and the like), VEGFR inhibitors [for example, ZD6474 (Cancer Res., Vol. 62, p.4645 (2002)) and the like], FGFR inhibitors [for example, PD 173074 (EMBO J., Vol. 17, p.5896 (1998)) and the like], PDGFR inhibitors [for example, SU11248 (Clin. Cancer Res.), Vol. 9, p.327 (2003)) and the like], Flt3 inhibitors [for example, MLN518 (Cancer Cell, Vol. 1, p.421 (2002)) and the like], IGF-1R inhibitors [for example, NVP-AEW541 (Cancer Cell, Vol. 5, p.231 (2004)) and the like]}; adenosine deaminase inhibitors (for example, pentostatin and the like); Hsp90 inhibitors [for example, radicicol, 17-allylamino-17-demethoxygeldanamycin (Cancer Chemother. Pharmacol., Vol. 42, p.273 (1998)) and the like]; neovascularization inhibitors [for example, SU6668 (Cancer Res.), Vol. 60, p.4152 (2000)) and the like]; blood vessel target agents (for example, combretastatin A4 and the like); histone deacetylase inhibitors [for example, SAHA (Proc. Natl. Acad. Sci. USA, Vol. 95, p.3003 (1998)) and the like]; matrix metalloprotease inhibitors (for example, marimastat and the like); prenyltransferase inhibitors [for example, R115777 (Cancer Res., Vol. 61, p.131 (2001)) and the like]; bisphosphonate preparations (for example, pamidronate, zoledronate and the like); serine/threonine kinase inhibitors {for example, Raf inhibitors [for example, BAY 43-9006 (Cancer Res., Vol. 64, p.7099 (2004)) and the like], mTOR inhibitors (for example, rapamycin and the like), aurora inhibitors [for example, VX-680 (Nat. Med., Vol. 10, p.262 (2004)) and the like], PKC/CHK1 inhibitors [for example, UCN-01 (J. Antibiot.), Vol. 40, p.1782 (1987)) and the like] and the like}; mitotic kinesin inhibitors [for example, Eg5 inhibitors (for example, SB-715992 (WO2001/98278, WO2003/070701) and the like) and the like] and the like, and further include derivatives of these compounds.

Examples of the medicaments comprising of proteins include, for example, cytokines, antibodies and the like.
Examples of the cytokines include, for example, interferons α, β, and γ; tumor necrosis factor (TNF)-α; lymphotoxin; interleukins-1, 2, 3, 4, 7, 8, 12, 15, 18 and 21; granulocyte colony stimulating factor (G-CSF); macrophage colony stimulating factor (M-CSF); granulocyte and macrophage colony-stimulating factor (GM-CSF); interferon-y-inducing protein-10 (IP-10); fractalkine and the like. Moreover, protein preparations comprising growth hormone receptor antagonists and the like are also included.
The antibodies are not particularly limited so long as an antibody against an antigen expressed in tumor cells or involved in formation of pathological conditions of tumors such as proliferation and metastasis of tumor cells, is chosen. Examples include, for example, antibodies against interleukin-6 (IL-6) receptor, GD2, GD3, GM2, HER2, CD20, CD22, CD33, CD52, MAGE, HM1.24, parathyroid hormone-related protein (PTHrP), basic fibroblast growth factor, fibroblast growth factor 8, basic fibroblast growth factor receptor, fibroblast growth factor 8 receptor, epidermal growth factor receptor (EGFR), epithelium cell adhesion molecule (EpCAM), insulin-like growth factor, insulin-like growth factor receptor, prostate-specific membrane antigen (PSMA), endothelial cell growth factor, endothelial cell growth factor receptor and the like. Specific examples of the aforementioned antibodies, not limiting the scope of the present invention, include, for example, the antibody described in Anticancer Res., Vol. 18, p.1217 (1998) as the anti-IL-6 receptor antibody, antibody described in Anticancer Res., Vol. 13, p.331 (1993) as the anti-GD2 antibody, antibody described in Cancer Immunol. Immunother., Vol. 36, p.260 (1993) as the anti-GD3 antibody, antibody described in Cancer Res., Vol. 54, p.1511 (1994) as the anti-GM2 antibody, antibody described in Proc. Natl. Acad. Sci. USA, Vol. 89, p.4285 (1992) as the anti-HER2 antibody, antibody described in Blood, Vol. 83, p.435 (1994) as the anti-CD20 antibody, antibody described in Semmin. Oncol., Vol. 30, p.253 (2003) as the anti-CD22 antibody, antibody described in J. Clin. Oncol., Vol. 19, p.3244 (2001) as the anti-CD33 antibody, antibody described in Blood, Vol. 82, p.807 (1993) as the anti-CD52 antibody, antibody described in British J. Cancer, Vol. 83, p.493, (2000) as the anti-MAGE antibody, antibody described in Molecular Immunol., Vol. 36, p.387 (1999) as the anti-HM1.24 antibody, antibody described in Cancer, Vol. 88, p.2909 (2000) as the anti-parathyroid hormone-related protein antibody, antibody described in Proc. Natl. Acad. Sci. USA, Vol. 86, p.9911 (1989) as the anti-fibroblast growth factor 8 antibody, antibody described in J. Biol. Chem., Vol. 265, p.16455 (1990) as the anti-fibroblast growth factor 8 receptor antibody, antibody described in Cancer Res., Vol. 59, p.1236 (1999) as the anti-epidermal growth factor receptor antibody, antibody described in Proc. Natl. Acad. Sci. USA, Vol. 76, p.1438 (1979) as the anti-epithelium cell adhesion-molecule antibody, antibody described in J. Neurosci. Res., Vol. 40, p.647 (1995) as the anti-insulin-like growth factor antibody, antibody described in J. Neurosci. Res., Vol. 40, p.647 (1995) as the anti-insulin-like growth factor receptor antibody, antibody described in J. Urology, Vol. 160, p.2396 (1998) as the anti-prostate-specific membrane antigen antibody, antibody described in Cancer Res., Vol. 57, p.4593 (1997) as the anti-endothelial cell growth factor antibody, antibody described in Oncogene, Vol. 19, p.2138 (2000) as the anti-endothelial cell growth factor receptor antibody, and the like.

More specifically, examples include, for example, Herceptin, Rituxan, Campath, Avastin, Bexxar, LymphoCide, Mylotarg, Panorex, Zevalin [Nat. Rev. Cancer, Vol. 1, p.118 (2001)] and the like.
Examples of the medicament consisting of nucleic acids include, for example, antisense, small interfering RNA (siRNA), ribozyme and the like. The nucleic acids are not particularly limited so long as a nucleic acid having a sequence complementary to a gene involved in formation of pathological conditions of tumors such as proliferation and metastasis of tumor cells is chosen. Examples include nucleic acids having sequences complementary to gene sequences targeted by the aforementioned low molecular weight compounds or proteins.

When Compound (I) or (II), or a pharmaceutically acceptable salt and another pharmaceutical ingredient are used in combination, Compound (I) or (II), or a pharmaceutically acceptable salt and the other pharmaceutical ingredient may be simultaneously administered, or they may be separately administered at an interval. Doses of these ingredients may be similar to clinically used doses, and vary depending on object of administration, administration route, type of disease, combination of pharmaceutical ingredient and the like.
When Compound (I) or (II), or a pharmaceutically acceptable salt and another pharmaceutical ingredient are used in combination, dosage forms are not particularly limited, and it is sufficient that Compound (I) or (II), or a pharmaceutically acceptable salt and another pharmaceutical ingredient are combined. For example, preparations prepared to contain these ingredients may be used or administered as a single preparation (mixture) or a combination of two or more preparations. When they are administered as a combination of two or more preparations, they may be simultaneously administered, or separately administered at an interval. These preparations are preferably used in the form of, for example, tablet, injection or the like. These preparations are prepared by any methods well known in the field of pharmaceutics as described above.

When they are administered as a combination of two or more preparations, for example, (a) a first component containing Compound (I) or (II), or a pharmaceutically acceptable salt, and (b) a second component containing another pharmaceutical ingredient may be prepared as separate preparations and prepared as a kit, and this kit may be used to administer the components simultaneously or separately at an interval to the same object via the same route or different routes.
Examples of the kits include those consisting of, for example, two or more containers (e.g., vial, bag, and the like) and contents thereof, of which container materials and forms are not particularly limited so long as denaturation of the components as contents by external temperature or light, or leakage of the contents are not caused during storage, and having such forms that the first and second components as the contents can be administered via separate routes (e.g., tubes) or the same route. Specifically, examples include a kit comprising tablets, injections and the like.

By use of the combination of Compound (I) or (II), or a pharmaceutically acceptable salt and one or more other pharmaceutical ingredients, improvement of the therapeutic and/or prophylactic effect for solid tumors, amelioration of side effects and the like can be expected.
As another embodiment of the present invention, administration of Compound (I) or (II), or a pharmaceutically acceptable salt and other medical practices can also be used in combination.
Although the other medical practices used in combination are not particularly limited, examples include, for example, surgical therapy, endoscopic therapy, radiotherapy, corpuscular radiation therapy, laser radiation therapy, immunotherapy, bone marrow transplantation, heat therapy, gene therapy [Rinsho Shuyo-Gaku (Clinical Oncology), 3rd edition, edited by Japanese Society of Medicinal Oncology (2003)] and the like.
By use of the combination of administration of Compound (I) or (II), or a pharmaceutically acceptable salt and other medical practices, improvement of the therapeutic and/or prophylactic effect for solid tumors, amelioration of side effects and the like can be expected.

### Examples

The present invention will be explained in detail with reference to the following examples and reference examples.
The spectra of proton nuclear magnetic resonance (¹H NMR) used in Examples were measured at 270 or 300 MHz, and exchangeable hydrogen may not always be clearly observed depending on the compound and the measurement conditions. For the descriptions of the multiplicity of signals, those generally applied are used, and the symbol "br" represents an apparent broad signal.

### [Example 1]

### Tablets (Compound 3)

Tablets having the following composition are prepared in a conventional manner. Compound 3 (40 g), lactose (286.8 g) and potato starch (60 g) are mixed, and 10% aqueous solution of hydroxypropylcellulose (120 g) is added to the mixture. Resulting mixture is kneaded, granulated and dried in a conventional manner, and then the granules are sized to obtain granules for tablet pressing. Magnesium stearate (1.2 g) is added to the granules for tablet pressing and mixed. Tablet formation is performed by using a compressing machine having a punch of 8 mm a diameter (Kikusui, RT-15) to obtain tablets (containing 20 mg/tablet of active ingredient).

**[Table 4]**

| Formulation | |
|---|---|
| Compound 3 | 20 mg |
| Lactose | 143.4 mg |
| Potato starch | 30 mg |
| Hydroxypropylcellulose | 6 mg |
| Magnesium stearate | 0.6 mg |
| | 200 mg |

### [Example 2]

### Tablets (Compound 4)

Tablets having the following composition are prepared in a conventional manner. Compound 4 (40 g), lactose (286.8 g) and potato starch (60 g) are mixed, and 10% aqueous solution of hydroxypropylcellulose (120 g) is added to the mixture. Resulting mixture is kneaded, granulated and dried in a conventional manner, and then the granules are sized to obtain granules for tablet pressing. Magnesium stearate (1.2 g) is added to the granules for tablet pressing and mixed. Tablet formation is performed by using a compressing machine having a punch of 8 mm a diameter (Kikusui, RT-15) to obtain tablets (containing 20 mg/tablet of active ingredient).

**[Table 5]**

| Formulation | |
|---|---|
| Compound 4 | 20 mg |
| Lactose | 143.4 mg |
| Potato starch | 30 mg |
| Hydroxypropylcellulose | 6 mg |
| Magnesium stearate | 0.6 mg |
| | 200 mg |

### [Example 3]

### Tablets (Compound 7)

Tablets having the following composition are prepared in a conventional manner. Compound 7 (40 g), lactose (286.8 g) and potato starch (60 g) are mixed, and 10% aqueous solution of hydroxypropylcellulose (120 g) is added to the mixture. Resulting mixture is kneaded, granulated and dried in a conventional manner, and then the granules are sized to obtain granules for tablet pressing. Magnesium stearate (1.2 g) is added to the granules for tablet pressing and mixed. Tablet formation is performed by using a compressing machine having a punch of 8 mm a diameter (Kikusui, RT-15) to obtain tablets (containing 20 mg/tablet of active ingredient).

**[Table 6]**

| Formulation | |
|---|---|
| Compound 7 | 20 mg |
| Lactose | 143.4 mg |
| Potato starch | 30 mg |
| Hydroxypropylcellulose | 6 mg |
| Magnesium stearate | 0.6 mg |
| | 200 mg |

### [Example 4]

### Injection (Compound 3)

Injection having the following composition is prepared in a conventional manner. Compound 3 (1 g) and D-mannitol (5 g) are added to distilled water for injection and mixed, and hydrochloric acid and aqueous sodium hydroxide are added to the mixture to adjust to pH 7, and then the total volume is made 1000 mL with distilled water for injection. The resulting mixture is aseptically filled in glass vials in a volume of 2 mL each to obtain injection (containing 2 mg/vial of the active ingredient).

**[Table 7]**

| Formulation | |
|---|---|
| Compound 3 | 2 mg |
| D-Mannitol | 10 mg |
| Hydrochloric acid | Optimum amount |
| Aqueous sodium hydroxide | Optimum amount |
| Distilled water for injection | Optimum amount |
| | 2.00 mL |

### [Example 5]

### Injection (Compound 9)

Injection having the following composition is prepared in a conventional manner. Compound 9 (1 g) and D-mannitol (5 g) are added to distilled water for injection and mixed, and hydrochloric acid and aqueous sodium hydroxide are added to the mixture to adjust to pH 7, and then the total volume is made 1000 mL with distilled water for injection. The resulting mixture is aseptically filled in glass vials in a volume of 2 mL each to obtain injection (containing 2 mg/vial of the active ingredient).

**[Table 8]**

| Formulation | |
|---|---|
| Compound 9 | 2 mg |
| D-Mannitol | 10 mg |
| Hydrochloric acid | Optimum amount |
| Aqueous sodium hydroxide | Optimum amount |
| Distilled water for injection | Optimum amount |
| | 2.00 mL |

### [Example 6]

### Injection (Compound 12)

Injection having the following composition is prepared in a conventional manner. Compound 12 (1 g) and D-mannitol (5 g) are added to distilled water for injection and mixed, and hydrochloric acid and aqueous sodium hydroxide are added to the mixture to adjust to pH 7, and then the total volume is made 1000 mL with distilled water for injection. The resulting mixture is aseptically filled in glass vials in a volume of 2 mL each to obtain injection (containing 2 mg/vial of the active ingredient).

**[Table 9]**

| Formulation | |
|---|---|
| Compound 12 | 2 mg |
| D-Mannitol | 10 mg |
| Hydrochloric acid | Optimum amount |
| Aqueous sodium hydroxide | Optimum amount |
| Distilled water for injection | Optimum amount |
| | 2.00 mL |

### [Example 7]

### Tablets (Compound a)

Tablets having the following composition are prepared in a conventional manner. Compound a (40 g), lactose (286.8 g) and potato starch (60 g) are mixed, and 10% aqueous solution of hydroxypropylcellulose (120 g) is added to the mixture. Resulting mixture is kneaded, granulated and dried in a conventional manner, and then the granules are sized to obtain granules for tablet pressing. Magnesium stearate (1.2 g) is added to the granules for tablet pressing and mixed. Tablet formation is performed by using a compressing machine having a punch of 8 mm a diameter (Kikusui, RT-15) to obtain tablets (containing 20 mg/tablet of active ingredient).

**[Table 10]**

| Formulation | |
|---|---|
| Compound a | 20 mg |
| Lactose | 143.4 mg |
| Potato starch | 30 mg |
| Hydroxypropylcellulose | 6 mg |
| Magnesium stearate | 0.6 mg |
| | 200 mg |

### [Example 8]

### Tablets (Compound d)

Tablets having the following composition are prepared in a conventional manner. Compound d (40 g), lactose (286.8 g) and potato starch (60 g) are mixed, and 10% aqueous solution of hydroxypropylcellulose (120 g) is added to the mixture. Resulting mixture is kneaded, granulated and dried in a conventional manner, and then the granules are sized to obtain granules for tablet pressing. Magnesium stearate (1.2 g) is added to the granules for tablet pressing and mixed. Tablet formation is performed with a compressing machine having a punch of 8 mm a diameter (Kikusui, RT-15) to obtain tablets (containing 20 mg/tablet of active ingredient).

**[Table 11]**

| Formulation | |
|---|---|
| Compound d | 20 mg |
| Lactose | 143.4 mg |
| Potato starch | 30 mg |
| Hydroxypropylcellulose | 6 mg |
| Magnesium stearate | 0.6 mg |
| | 200 mg |

### [Example 9]

### Tablets (Compound e)

Tablets having the following composition are prepared in a conventional manner. Compound e (40 g), lactose (286.8 g) and potato starch (60 g) are mixed, and 10% aqueous solution of hydroxypropylcellulose (120 g) is added to the mixture. Resulting mixture is kneaded, granulated and dried in a conventional manner, and then the granules are sized to obtain granules for tablet pressing. Magnesium stearate (1.2 g) is added to the granules for tablet pressing and mixed. Tablet formation is performed with a compressing machine having a punch of 8 mm a diameter (Kikusui, RT-15) to obtain tablets (containing 20 mg/tablet of active ingredient).

**[Table 12]**

| Formulation | |
|---|---|
| Compound e | 20 mg |
| Lactose | 143.4 mg |
| Potato starch | 30 mg |
| Hydroxypropylcellulose | 6 mg |
| Magnesium stearate | 0.6 mg |
| | 200 mg |

### [Example 10]

### Tablets (Compound 1)

Tablets having the following composition are prepared in a conventional manner. Compound 1 (40 g), lactose (286.8 g) and potato starch (60 g) are mixed, and 10% aqueous solution of hydroxypropylcellulose (120 g) is added to the mixture. Resulting mixture is kneaded, granulated and dried in a conventional manner, and then the granules are sized to obtain granules for tablet pressing. Magnesium stearate (1.2 g) is added to the granules for tablet pressing and mixed. Tablet formation is performed with a compressing machine having a punch of 8 mm a diameter (Kikusui, RT-15) to obtain tablets (containing 20 mg/tablet of active ingredient).

**[Table 13]**

| Formulation | |
|---|---|
| Compound 1 | 20 mg |
| Lactose | 143.4 mg |
| Potato starch | 30 mg |
| Hydroxypropylcellulose | 6 mg |
| Magnesium stearate | 0.6 mg |
| | 200 mg |

### [Example 11]

### Tablets (Compound m)

Tablets having the following composition are prepared in a conventional manner. Compound m (40 g), lactose (286.8 g) and potato starch (60 g) are mixed, and 10% aqueous solution of hydroxypropylcellulose (120 g) is added to the mixture. Resulting mixture is kneaded, granulated and dried in a conventional manner, and then the granules are sized to obtain granules for tablet pressing. Magnesium stearate (1.2 g) is added to the granules for tablet pressing and mixed. Tablet formation is performed with a compressing machine having a punch of 8 mm a diameter (Kikusui, RT-15) to obtain tablets (containing 20 mg/tablet of active ingredient).

**[Table 14]**

| Formulation | |
|---|---|
| Compound m | 20 mg |
| Lactose | 143.4 mg |
| Potato starch | 30 mg |
| Hydroxypropylcellulose | 6 mg |
| Magnesium stearate | 0.6 mg |
| | 200 mg |

### [Example 12]

### Injection (Compound a)

Injection having the following composition is prepared in a conventional manner. Compound a (1 g) and D-mannitol (5 g) are added to distilled water for injection and mixed, and hydrochloric acid and aqueous sodium hydroxide are added to the mixture to adjust to pH 7, and then the total volume is made 1000 mL with distilled water for injection. The resulting mixture is aseptically filled in glass vials in a volume of 2 mL each to obtain injection (containing 2 mg/vial of the active ingredient).

**[Table 15]**

| Formulation | |
|---|---|
| Compound a | 2 mg |
| D-Mannitol | 10 mg |
| Hydrochloric acid | Optimum amount |
| Aqueous sodium hydroxide | Optimum amount |
| Distilled water for injection | Optimum amount |
| | 2.00 mL |

### [Example 13]

### Injection (Compound 1)

Injection having the following composition is prepared in a conventional manner. Compound 1 (1 g) and D-mannitol (5 g) are added to distilled water for injection and mixed, and hydrochloric acid and aqueous sodium hydroxide are added to the mixture to adjust to pH 7, and then the total volume is made 1000 mL with distilled water for injection. The resulting mixture is aseptically filled in glass vials in a volume of 2 mL each to obtain injection (containing 2 mg/vial of the active ingredient).

**[Table 16]**

| Formulation | |
|---|---|
| Compound 1 | 2 mg |
| D-Mannitol | 10 mg |
| Hydrochloric acid | Optimum amount |
| Aqueous sodium hydroxide | Optimum amount |
| Distilled water for injection | Optimum amount |
| | 2.00 mL |

### [Example 14]

### Injection (Compound m)

Injection having the following composition is prepared in a conventional manner. Compound m (1 g) and D-mannitol (5 g) are added to distilled water for injection and mixed, and hydrochloric acid and aqueous sodium hydroxide are added to the mixture to adjust to pH 7, and then the total volume is made 1000 mL with distilled water for injection. The resulting mixture is aseptically filled in glass vials in a volume of 2 mL each to obtain injection (containing 2 mg/vial of the active ingredient).

**[Table 17]**

| Formulation | |
|---|---|
| Compound m | 2 mg |
| D-Mannitol | 10 mg |
| Hydrochloric acid | Optimum amount |
| Aqueous sodium hydroxide | Optimum amount |
| Distilled water for injection | Optimum amount |
| | 2.00 mL |

### [Example 15]

Compound a: (-)-N-[4-(2,2-Dimethylpropionyl)-5-(2-methanesulfonylaminoethyl)-5-phenyl-4,5-dihyd ro-1,3,4-thiadiazol-2-yl]-2,2-dimethylpropanamide Step 1: (S)-(+)-2-Phenylpropionic acid (4.88 g, 32.5 mmol) was dissolved in dichloromethane (20 mL), and thionyl chloride (30 mL) was added thereto, then the mixture was stirred at room temperature for 4 hours. The mixture was concentrated under reduced pressure, and the resulting residue was dissolved in dichloromethane (10 mL) (dichloromethane solution). Next, N-{2-[5-amino-3-(2,2-dimethylpropionyl)-2-phenyl-2,3-dihydro-1,3,4-thiadiazol-2-yl]ethyl}methanesulfonamide (4.93 g, 12.8 mmol) obtained according to the method described in WO2003/051854 was dissolved in dichloromethane (15 mL) and pyridine (3.1 mL), and the aforementioned dichloromethane solution was added. After the mixture was stirred at room temperature for 1.5 hours, water was added, and the mixture was extracted with chloroform. The organic layer was washed with 1 mol/L hydrochloric acid, water and saturated brine, dried over anhydrous sodium sulfate, and concentrated under reduced pressure. To the residue were added chloroform (50 mL) and diisopropyl ether (10 mL), and the mixture was stirred. The deposited powder was collected by filtration, and purified by silica gel column chromatography (chloroform/acetone/n-hexane/ethyl acetate = 9/1/1/1, 9/1/6.5/3.5, 9/1/7/3, and then 9/1/5/5) repeatedly to give one diastereomer of N-[4-(2,2-dimethylpropionyl)-5-(2-methanesulfonylaminoethyl)-5-phenyl-4,5-dihydro-1,3,4-thiadiazol-2-yl]-2-phenylpropanamide (2.48 g, 38%) as a fraction eluted first and another diastereomer of N-[4-(2,2-dimethylpropionyl)-5-(2-methanesulfonylaminoethyl)-5-phenyl-4,5-dihydro-1,3,4-thiadiazol-2-yl]-2-phenylpropanamide (2.80 g, 43%) as a fraction eluted later.

One diastereomer of N-[4-(2,2-dimethylpropionyl)-5-(2-methanesulfonylaminoethyl)-5-phenyl-4,5-dihydro-1,3,4-thiadiazol-2-yl]-2-phenylpropanamide that eluted first:
¹H NMR (270 MHz, CDCl₃) δ (ppm): 1.26 (s, 9H), 1.53 (d, J = 7.1 Hz, 3H), 2.60 (m, 1H), 2.93 (s, 3H), 3.20 (m, 1H), 3.36 (m, 1H), 3.57 (m, 1H), 3.67 (q, J = 7.1 Hz, 1H), 4.45 (br t, 1H), 7.20-7.49 (m, 10H), 7.75 (s, 1H).
APCI-MS m/z: 515 (M-H)⁻.
Another diastereomer of N-[4-(2,2-dimethylpropionyl)-5-(2-methanesulfonylaminoethyl)-5-phenyl-4,5-dihydro-1,3,4-thiadiazol-2-yl]-2-phenylpropanamide that eluted later: ¹H NMR (270 MHz, CDCl₃) δ (ppm): 1.25 (s, 9H), 1.51 (d, J = 7.1 Hz, 3H), 2.56 (m, 1H), 2.96 (s, 3H), 3.23 (m, 1H), 3.37 (m, 1H), 3.62 (m, 1H), 3.63 (q, J = 7.1 Hz, 1H), 4.67 (br t, J = 5.9 Hz, 1H), 7.17-7.52 (m, 10H), 7.99 (s, 1H).
APCI-MS m/z: 515 (M-H)⁻.

Step 2: The one diastereomer of N-[4-(2,2-dimethylpropionyl)-5-(2-methanesulfonylaminoethyl)-5-phenyl-4,5-dihydro-1,3,4-thiadiazol-2-yl]-2-phenylpropanamide (2.28 g, 4.41 mmol) eluted first obtained in Step 1 mentioned above was dissolved in methanol (100 mL), and cerium chloride heptahydrate (1.64 g, 4.41 mmol) and sodium borohydride (6.68 g, 0.176 mmol) were added, then the mixture was stirred at room temperature for 40 minutes. The mixture was further stirred at room temperature for 2 hours with adding sodium borohydride (20.04 g, 0.5297 mmol) and methanol (250 mL), divided into 3 portions, respectively, to the mixture, and then concentrated under reduced pressure. To the residue were added ethyl acetate and 1 mol/L hydrochloric acid, and the mixture was extracted with ethyl acetate. The organic layer was washed with saturated brine, dried over anhydrous sodium sulfate, and concentrated under reduced pressure. The residue was purified by silica gel column chromatography (chloroform/acetone/n-hexane/ethyl acetate = 9/1/7/3 -> 9/1/5/5).
This procedure was repeatedly performed, and the resulting crude product (0.802 g, 2.09 mmol in total) was dissolved in a mixed solvent of ethanol (20 mL) and n-hexane (200 mL). Then the deposited solid was filtered off, and the filtrate was concentrated to give optically active N-{2-[5-amino-3-(2,2-dimethylpropionyl)-2-phenyl-2,3-dihydro-1,3,4-thiadiazol-2-yl]ethyl}methanesulfonamide (0.647 g, 23%).

Step 3: The optically active N-{2-[5-amino-3-(2,2-dimethylpropionyl)-2-phenyl-2,3-dihydro-1,3,4-thiadiazol-2-yl]ethyl}methanesulfonamide (90 mg, 0.23 mmol) obtained in Step 2 mentioned above was dissolved in dichloromethane (4 mL), and pyridine (0.224 mL, 2.77 mmol) and trimethylacetyl chloride (0.288 mL, 2.33 mmol) were added, then the mixture was stirred at room temperature for 3.5 hours. To the reaction mixture were added water and 1 mol/L hydrochloric acid, and the mixture was extracted with ethyl acetate. The organic layer was washed with saturated brine, dried over anhydrous sodium sulfate, and concentrated under reduced pressure. After the residue was purified by silica gel column chromatography (n-hexane/ethyl acetate = 3/1 -> 2/1), to the resulting syrup were added ethanol and then n-hexane. The supernatant was separated by decantation to give the deposited solid. Subsequently, to the solid was added diisopropyl ether, and the mixture was stirred to pulverize the resulting solid and thereby give Compound a {(-)-N-[4-(2,2-dimethyl-propionyl)-5-(2-methanesulfonylaminoethyl)-5-phenyl-4,5-dihydro-1,3,4-thiadiazol-2-yl]-2,2-dimethylpropanamide} (60 mg, 55%).
¹H NMR (300 MHz, CDCl₃) δ (ppm): 1.30 (s, 9H), 1.34 (s, 9H), 2.56-2.65 (m, 1H), 2.94 (s, 3H), 3.21-3.44 (m, 2H), 3.58-3.70 (m, 1H), 4.45 (br s, 1H), 7.28-7.37 (m, 5H), 7.97 (br s, 1H).
APCI-MS m/z: 467 (M-1)⁻.
Melting point: 204.0-206.0°C.
Specific rotation: A solution of the resulting compound in methanol gave a negative value as a specific rotation for sodium D line (wavelength: 589.3 nm) at 20°C.

### [Example 16]

Compound b: (-)-N-[5-(2-Methanesulfonylaminoethyl)-5-phenyl-4-propionyl-4,5-dihydro-1,3,4-thiadiazol-2-yl]-2,2-dimethylpropanamide
Step 1: In the same manner as that in Step 1 of Example 15, from N-[2-(5-amino-2-phenyl-3-propionyl-2,3-dihydro-1,3,4-thiadiazol-2-yl)ethyl]methanesulfonamide (10.7 g, 30.0 mmol) obtained according to the method described in WO2003/051854, and (R)-(-)-2-phenylpropionyl chloride prepared from (R)-(-)-2-phenylpropionic acid (10.5 g, 69.9 mmol) and thionyl chloride, N-[5-(2-methanesulfonylaminoethyl)-5-phenyl-4-propionyl-4,5-dihydro-1,3,4-thiadiazol-2-yl]-2-phenylpropanamide was obtained as a diastereomer mixture (13.3 g, 92%). A part of this mixture (3.89 g, 7.96 mmol) was purified by silica gel column chromatography (chloroform/acetonitrile/n-hexane/ethyl acetate = 9/1/1/1) to give one diastereomer of N-[5-(2-methanesulfonylaminoethyl)-5-phenyl-4-propionyl-4,5-dihydro-1,3,4-thiadiazol-2-yl]-2-phenylpropanamide (0.861 g, 22%) as a fraction that eluted later, and another diastereomer of N-[5-(2-methanesulfonylaminoethyl)-5-phenyl-4-propionyl-4,5-dihydro-1,3,4-thiadiazol-2-yl]-2-phenylp ropanamide (0.802 g, 20%) as a fraction that eluted first.

Step 2: In the same manner as that in Step 2 of Example 15, from the one diastereomer of N-[5-(2-methanesulfonylaminoethyl)-5-phenyl-4-propionyl-4,5-dihydro-1,3,4-thiadiazol-2-yl]-2-phenylpropanamide (4.41 g, 9.03 mmol) eluted later obtained in Step 1 mentioned above, cerium chloride heptahydrate (3.37 g, 9.05 mmol) and sodium borohydride (3.42 g, 90.5 mmol), optically active N-[2-(5-amino-2-phenyl-3-propionyl-2,3-dihydro-1,3,4-thiadiazol-2-yl)ethyl]methanesulfonamide (2.16 g, 67%) was obtained.
Step 3: In the same manner as that in Step 3 of Example 15, from the optically active N-[2-(5-amino-2-phenyl-3-propionyl-2,3-dihydro-1,3,4-thiadiazol-2-yl)ethyl]methanesulfonamide (0.0480 g, 0.135 mmol) obtained in Step 2 mentioned above, pyridine (32.7 µL, 0.405 mmol) and trimethylacetyl chloride (41.7 µL, 0.338 mmol), Compound b {(-)-N-[5-(2-methanesulfonylaminoethyl)-5-phenyl-4-propionyl-4,5-dihydro-1,3,4-thiadiazol-2-yl]-2,2-dimethylpropanamide} (0.0504 g, 84%) was obtained.
¹H NMR (270 MHz, CDCl₃) δ (ppm): 1.13 (t, J = 6.0 Hz, 3H), 1.28 (s, 9H), 2.66 (m, 3H), 2.97 (s, 3H), 3.35 (m, 2H), 3.61 (m, 1H), 4.58 (br s, 1H), 7.32 (m, 5H), 8.08 (br s, 1H). APCI-MS m/z: 441 (M+1)⁺.
Melting point: 107.0- 110.0°C.
Specific rotation: A solution of the resulting compound in methanol gave a negative value as a specific rotation for sodium D line (wavelength: 589.3 nm) at 20°C.

### [Example 17]

### Compound c: (-)-N-{2-[3-(2,2-Dimethylpropionyl)-5-(2-oxopyrrolidin-1-yl)-2-phenyl-2,3-dihydro-1,3,4-thiadiazol-2-yl]ethyl}methanesulfonamide

The optically active N-{2-[5-amino-3-(2,2-dimethylpropionyl)-2-phenyl-2,3-dihydro-1,3,4-thiadiazol-2-yl]et hyl}methanesulfonamide (0.647 g, 1.68 mmol) obtained in Step 2 of Example 15 was dissolved in dichloromethane (25 mL), and pyridine (0.41 mL, 5.1 mmol) and 4-bromobutyryl chloride (0.49 mL, 4.2 mmol) were added, then the mixture was stirred at room temperature for 2 hours. To the reaction mixture was added water, and the mixture was extracted with chloroform. The organic layer was washed with 0.5 mol/L hydrochloric acid and brine, dried over anhydrous sodium sulfate, and concentrated under reduced pressure. The residue was dissolved in dimethyl sulfoxide (DMSO, 6 mL), and sodium acetate (0.331 g, 4.04 mmol) was added, then the mixture was heated to 100°C over 14 minutes with stirring. After cooling, to the mixture was added water, and the mixture was extracted with ethyl acetate. The organic layer was washed with brine, dried over anhydrous sodium sulfate, and concentrated under reduced pressure. The residue was purified by flash column chromatography (n-hexane/ethyl acetate = 3/1 -> 1/1), and recrystallized from acetone to give Compound c {(-)-N-{2-[3-(2,2-dimethylpropionyl)-5-(2-oxopyrrolidin-1-yl)-2-phenyl-2,3-dihydro-1,3,4-thiadiazol-2-yl] ethyl}methanesulfonamide} (0.649 g, 85%).
¹H NMR (270 MHz, CDCl₃) δ (ppm): 1.34 (s, 9H), 2.23 (m, 2H), 2.56 (m, 2H), 2.61 (m, 1H), 2.97 (s, 3H), 3.27 (m, 1H), 3.40 (m, 1H), 3.63 (m, 1H), 3.98 (m, 2H), 4.01 (br t, J = 3.5 Hz, 1H), 7.20-7.37 (m, 5H).
APCI-MS m/z: 453 (M+1)⁺.
Melting point: 107.0-110.0°C.
Specific rotation: A solution of the resulting compound in methanol gave a negative value as a specific rotation for sodium D line (wavelength: 589.3 nm) at 20°C.

### [Example 18]

### Compound d: (-)-N-[4-Isobutyryl-5-(2-methanesulfonylaminoethyl)-5-phenyl-4,5-dihydro-1, 3,4-thiadiazol-2-yl]-2,2-dimethylpropanamide

### Step 1:

N-[4-Isobutyryl-5-(2-methanesulfonylaminoethyl)-5-phenyl-4,5-dihydro-1,3,4-thiadiaz ol-2-yl]-2,2-dimethylpropanamide (2.32 g, 5.10 mmol) obtained according to the method described in WO2003/051854 was subjected to preparative high performance liquid chromatography (HPLC) [column: CHIRALPAK AD (Daicel Chemical Industries, Ltd.], elution solvent: 12% isopropyl alcohol/n-hexane, flow rate: 6 mL/minute, column temperature: 25°C.] to give fractions for retention times of 10.2 minutes and 11.2 minutes. Among them, the fraction of 11.2 minutes was concentrated, and the residue was recrystallized from n-pentane and ethanol to give Compound d {(-)-N-[4-isobutyryl-5-(2-methanesulfonylaminoethyl)-5-phenyl-4,5-dihydro-1,3,4-thiadiazol-2-yl]-2,2-dimethylpropanamide} (0.707 g, 30%) as white crystals.
¹H NMR (270 MHz, CDCl₃) δ (ppm): 1.15 (2 x d, J = 7.0 Hz, 6H), 1.29 (s, 9H), 2.57-2.67 (m, 1H), 2.96 (s, 3H), 3.23-3.44 (m, 3H), 3.37-3.68 (m, 1H), 4.46 (br s, 1H), 7.25-7.38 (m, 5H), 8.00 (br s, 1H).
APCI-MS m/z: 453 (M-1)⁻.
Melting point: 162.0-164.0°C.
Specific rotation: A solution of the resulting compound in methanol gave a negative value as a specific rotation for sodium D line (wavelength: 589.3 nm) at 20°C.

### [Example 19]

### Compound e:(-)-N-{2- [5-(2-Oxopyrrolidin-1-yl)-2-phenyl-3-propionyl-2,3-dihydro-1,3,4-thiadiazol-2-yl]ethyl}methanesulfonamide

The optically active N- [2-(5-amino-2-phenyl-3-propionyl-2,3-dihydro-1,3,4-thiadiazol-2-yl)ethyl]methanesu lfonamide (1.01 g, 2.83 mmol) obtained in Step 2 of Example 16 and pyridine (330 µL, 4.08 mmol) were dissolved in dichloromethane (40 mL), and 4-bromobutyryl chloride (390 µL, 3.40 mmol) was added at 0°C, then the mixture was stirred at room temperature for 2 hours. To the mixture was added 1 mol/L hydrochloric, and the mixture was extracted with chloroform. The organic layer was dried over anhydrous sodium sulfate, and concentrated under reduced pressure. To the residue were added DMSO (10 mL) and sodium acetate (560 mg, 6.83 mmol), and the mixture was stirred at 100°C for 5 minutes. After cooling, water and 1 mol/L hydrochloric acid were added, and the mixture was extracted with ethyl acetate. The organic layer was dried over anhydrous sodium sulfate, and concentrated under reduced pressure. The residue was purified by silica gel column chromatography (chloroform/methanol = 20/1) to give Compound e {(-)-N-{2-[5-(2-oxopyrrolidin-1-yl)-2-phenyl-3-propionyl-2,3-dihydro-1,3,4-thiadiazol-2-yl]ethyl}methanesulfonamide} (878 mg, 73%).
¹H NMR (270 MHz, CDCl₃) δ (ppm): 1.15 (t, J = 6.6 Hz, 3H), 2.22 (m, 2H), 2.55-2.67 (m, 3H), 2.94 (s, 3H), 3.31-3.47 (m, 4H), 3.61 (m, 1H), 3.91-3.98 (m, 2H), 5.0 (br s, 1H), 7.20-7.35 (m, 5H).
APCI-MS m/z: 423 (M-1)⁻.
Melting point: 188.0-191.0°C.
Specific rotation: A solution of the resulting compound in methanol gave a negative value as a specific rotation for sodium D line (wavelength: 589.3 nm) at 20°C.

### [Example 20]

### Compound f:

### (-)-N-[4-Acetyl-5-(2-methanesulfonylaminoethyl)-5-phenyl-4,5-dihydro-1,3,4-thiadiazol-2-yl] -2,2-dimethylpropanamide

Step 1: Methanesulfonamide (0.476 g, 5.00 mmol) was dissolved in N,N-dimethylformamide (DMF, 10 mL), and 60% sodium hydride (0.275 g, 5.00 mmol) was added at 0°C, then the mixture was stirred at the same temperature for 20 minutes. Subsequently, to the mixture was added 3-chloropropiophenone (843 mg, 5.00 mol), and the mixture was stirred at the same temperature for 2 hours, and then further stirred at room temperature for 15 hours. To the mixture was added water, and the mixture was extracted with ethyl acetate. The organic layer was washed with brine, dried over anhydrous sodium sulfate, and concentrated under reduced pressure. The residue was purified by silica gel column chromatography (chloroform/methanol = 20/1) to give N-methanesulfonyl-3-aminopropiophenone (240 mg, 21%).
Subsequently, in the same manner as that of the method described in WO2003/051854, N-methanesulfonyl-3-aminopropiophenone=thiosemicarbazone (219 mg, 45%) was obtained from N-methanesulfonyl-3-aminopropiophenone (388 mg, 1.71 mmol) obtained above and thiosemicarbazide (156 mg, 1.71 mmol).

Step 2: N-Methanesulfonyl-3-aminopropiophenone=thiosemicarbazone (9.83 g, 32.7 mmol) obtained in Step 1 mentioned above was dissolved in acetic anhydride (38 mL), and the solution was stirred at 130°C for 10 minutes, and further stirred at 70°C for 2 hours, and then at room temperature for 5 hours. The deposited solid was collected by filtration to give N-[4-acetyl-5-(2-methanesulfonylaminoethyl)-5-phenyl-4,5-dihydro-1,3,4-thiadiazol-2-yl]acetamide (11.3 g, 73%).
Step 3: In the same manner as that of the method described in WO2003/051854, from N-[4-acetyl-5-(2-methanesulfonylaminoethyl)-5-phenyl-4,5-dihydro-1,3,4-thiadiazol-2-yl]acetamide (5.22 g, 13.6 mmol) obtained in Step 2 mentioned above, sodium borohydride (5.14 g, 136 mmol), and cerium chloride heptahydrate (5.07 g, 13.6 mmol), N-[2-(3-acetyl-5-amino-2-phenyl-2,3-dihydro-1,3,4-thiadiazol-2-yl)ethyl]methanesulfonamide was obtained.
Next, (R)-(-)-2-phenylpropionyl chloride prepared from (R)-(-)-2-phenylpropionic acid (4.65 g, 3.10 mmol) and thionyl chloride (30 mL), and N-[2-(3-acetyl-5-amino-2-phenyl-2,3-dihydro-1,3,4-thiadiazol-2-yl)ethyl]methanesulfonamide obtained above were treated in pyridine (5.0 mL, 61.8 mmol) in the same manner as that in Step 1 of Example 15, and the resultant was purified by silica gel column chromatography (chloroform/n-hexane/ethyl acetate/methanol = 20/3/2/1) to give one diastereomer of N-[4-acetyl-5-(2-methanesulfonylaminoethyl)-5-phenyl-4,5-dihydro-1,3,4-thiadiazol-2-yl]-2-phenylpropanamide (0.75 g, 12%) as a fraction eluted first, and another diastereomer of N-[4-acetyl-5-(2-methanesulfonylaminoethyl)-5-phenyl-4,5-dihydro-1,3,4-thiadiazol-2-yl]-2-phenylpropanamide (0.82 g, 13%) as a fraction eluted later.

Step 4: In the same manner as that in Step 2 of Example 15, from another diastereomer of N-[4-acetyl-5-(2-methanesulfonylaminoethyl)-5-phenyl-4,5-dihydro-1,3,4-thiadiazol-2-yl]-2-phenylpropanamide (0.632 g, 1.33 mmol) eluted later obtained in Step 3 mentioned above, cerium chloride heptahydrate (0.496 g, 1.33 mmol) and sodium borohydride (0.503 g, 13.3 mmol), optically active N-[2-(3-acetyl-5-amino-2-phenyl-2,3-dihydro-1,3,4-thiadiazol-2-yl)ethyl]methanesulfonamide (232 mg, 51%) was obtained.
Step 5: In the same manner as that in Step 3 of Example 15, from the optically active N- [2-(3-acetyl-5-amino-2-phenyl-2,3-dihydro-1,3,4-thiadiazol-2-yl)ethyl]-methanesulfonamide (0.0393 g, 0.115 mmol) obtained in Step 4 mentioned above, pyridine (44.7 µL, 0.552 mmol) and trimethylacetyl chloride (56.7 µL, 0.460 mmol), Compound f {(-)-N-[4-acetyl-5-(2-methanesulfonylaminoethyl)-5-phenyl-4,5-dihydro-1,3,4-thiadiazol-2-yl]-2,2-dimethylpropanamide} (0.0420 g, 86%) was obtained.
¹H NMR (270 MHz, CDCl₃) δ (ppm): 1.28 (s, 9H), 2.30 (s, 3H), 2.55-2.68 (m, 1H), 2.97 (s, 3H), 3.30-3.43 (m, 2H), 3.59-3.68 (m, 1H), 4.44 (br s, 1H), 7.27-7.39 (m, 5H), 8.00 (br s, 1H).
APCI-MS m/z: 425 (M-1)⁻.
Melting point: 187.0-190.0°C.
Specific rotation: A solution of the resulting compound in methanol gave a negative value as a specific rotation for sodium D line (wavelength: 589.3 nm) at 20°C.

### [Example 21]

### Compound g: N-{2-[3-Acetyl-5-(2-oxopyrrolidin-1-yl)-2-phenyl-2,3-dihydro-1,3,4-thiadiazol-2-yl]ethyl}methanesulfonamide

In the same manner as that in Example 17, from the optically active N-[2-(3-acetyl-5-amino-2-phenyl-2,3-dihydro-1,3,4-thiadiazol-2-yl)ethyl]methanesulfonamide (0.0300 g, 0.0876 mmol) obtained in Step 4 of Example 20, pyridine (33.6 µL, 0.420 mmol), 4-bromobutyryl chloride (40.6 µL, 0.350 mmol) and sodium acetate (0.0575 g, 0.701 mmol), Compound g {N-{2-[3-acetyl-5-(2-oxopyrrolidin-1-yl)-2-phenyl-2,3-dihydro-1,3,4-thiadiazol-2-yl]ethyl}methanesulfonamide} (0.0301 g, 84%) was obtained.
¹H NMR (270 MHz, CDCl₃) δ (ppm): 2.15 (m, 2H), 2.33 (s, 3H), 2.50-2.67 (m, 3H), 2.97 (s, 3H), 3.31-3.44 (m, 2H), 3.60-3.65 (m, 1H), 3.87-3.97 (m, 2H), 4.46 (br s, 1H), 7.24-7.38 (m, 5H).
APCI-MS m/z: 409 (M-1)⁻.
Melting point: 137.0-140.0°C.

### [Example 22]

### Compound h: (-)-N-{2-[3-Acetyl-5-(2-oxopiperidino)-2-phenyl-2,3-dihydro-1,3,4-thiadiazol-2-yl]ethyl}methanesulfonamide

In the same manner as that in Example 17, from the optically active N-[2-(3-acetyl-5-amino-2-phenyl-2, 3-dihydro-1,3,4-thiadiazol-2-yl)ethyl]methanesulfonamide (0.0260 g, 0.0759 mmol) obtained in Step 4 of Example 20, pyridine (29.3 µL, 0.365 mmol), 5-bromovaleryl chloride (40.7 µL, 0.304 mmol) and sodium acetate (0.0498 g, 0.607 mmol), Compound h {(-)-N-{2-[3-acetyl-5-(2-oxopiperidino)-2-phenyl-2,3-dihydro-1,3,4-thiadiazol-2-yl]ethyl}methanesulfonamide} (0.0241 g, 75%) was obtained.
¹H NMR (270 MHz, CDCl₃) δ (ppm): 1.82-1.98 (m, 4H), 2.33 (s, 3H), 2.52-2.62 (m, 3H), 2.95 (s, 3H), 3.27-3.38 (m, 2H), 3.59-3.70 (m, 1H), 3.84-3.92 (m, 2H), 4.62 (br s, 1H), 7.23-7.37 (m, 5H).
APCI-MS m/z: 423 (M-1)⁻.
Melting point: 169.0-171.0°C.
Specific rotation: A solution of the resulting compound in methanol gave a negative value as a specific rotation for sodium D line (wavelength: 589.3 nm) at 20°C.

### [Example 23]

### Compound i: N-{4-(2,2-Dimethylpropionyl)-5-[2-(2-ethylaminoethanesulfonylamino)ethyl]-5-phenyl-4,5-dihydro-1,3,4-thiadiazol-2-yl}-2,2-dimethylpropanamide

Compound 14 {N-{4-(2,2-dimethylpropionyl-5-[2-(2-ethylaminoethanesulfonyl-amino)ethyl]-5-phenyl-4,5-dihydro-1,3,4-thiadiazol-2-yl}-2,2-dimethylpropanamide} obtained in Reference Example 14 (0.15 g, 0.29 mmol) was subjected to preparative high performance liquid chromatography (HPLC) [column: CHIRALCEL OD, ϕ 20 x 250 mm (Daicel Chemical Industries, Ltd.), elution solvent: hexane/ethanol = 80/20 (containing 0.1% diethylamine), flow rate: 6.0 mL/minute] to give a fraction for a retention time of 9.0 minutes among fractions for retention times of 7.5 minutes and 9.0 minutes. The resulting fraction was concentrated to give Compound i {N-{4-(2,2-dimethylpropionyl)-5-[2-(2-ethylaminoethanesulfonylamino)ethyl]-5-phenyl-4,5-dihydro-1,3,4-thiadiazol-2-yl}-2,2-dimethylpropanamide} (33 mg, 22% as a white solid. ¹H NMR (270 MHz, CDCl₃) δ (ppm): 1.11 (t, J = 7.1 Hz, 3H), 1.30 (s, 9H), 1.33 (s, 9H), 2.67 (q, J = 7.1 Hz, 2H), 2.53-2.70 (m, 1H), 3.00-3.76 (m, 8H), 7.22-7.38 (m, 5H), 7.92 (br s, 1H).
APCI-MS m/z: 526 (M+H)⁺.
[Example 24]

### Compound j:

### N-[5-Aminomethyl-4-(2,2-dimethylpropionyl)-5-phenyl-4,5-dihydro-1,3,4-thiadiazol-2-yl]-2,2-dimethylpropanamide

Step 1: [3-(2,2-Dimethylpropionyl)-5-(2,2-dimethylpropionylamino)-2-phenyl-2,3-dihydro-1,3,4-thiadiazol-2-ylmethyl]carbamic acid tert-butyl ester obtained according to the method described in WO2004/092147 was subjected to high performance liquid chromatography (HPLC) [column: CHIRALPAK AD ϕ 4.6 x 250 mm (Daicel Chemical Industries, Ltd.), elution solvent: hexane/ethanol = 80/20, flow rate: 1.0 mL/minute], and a fraction for a retention time of 5.76 minutes was collected among fractions for retention times of 4.63 minutes and 5.76 minutes to give optically active [3-(2,2-dimethylpropionyl)-5-(2,2-dimethylpropionylamino)-2-phenyl-2,3-dihydro-1,3,4-thiadiazol-2-ylmethyl]carbamic acid tert-butyl ester.
Step 2: The optically active [3-(2,2-dimethylpropionyl)-5-(2,2-dimethylpropionylamino)-2-phenyl-2,3-dihydro-1,3,4-thiadiazol-2-ylmethyl]carbamic acid tert-butyl ester (5.91 g, 12.4 mmol) obtained in Step 1 mentioned above was dissolved in ethyl acetate (20 mL), and 1 mol/L hydrogen chloride/ethyl acetate solution (40 mL) was added, then the mixture was stirred at room temperature for 1 hour. The deposited crystals were collected by filtration, and the resulting crystals were dried under reduced pressure with heating to give hydrochloride of Compound j {N-[5-aminomethyl-4-(2,2-dimethylpropionyl)-5-phenyl-4,5-dihydro-1,3,4-thiadiazol-2-yl]-2,2-dimethylpropanamide} (4.72 g, 92%).
APCI-MS m/z: 377(M+H)⁺.
Melting point: 175.0-182.0°C.

### [Example 25]

### Compound k: N-[4-(2,2-Dimethylpropionyl)-5-ethenesulfonylaminomethyl-5-phenyl-4,5-dihydro-1,3,4-thiadiazol-2-yl]-2,2-dimethylpropanamide

The hydrochloride of Compound j {N-[5-aminomethyl-4-(2,2-dimethylpropionyl)-5-phenyl-4,5-dihydro-1,3,4-thiadiazol-2-yl]-2,2-dimethylpropanamide} (0.502 g, 1.22 mmol) obtained in Example 24 was dissolved in ethyl acetate (20 mL), and 2-chloroethanesulfonyl chloride (0.203 mL, 1.22 mmol) was added, then the mixture was stirred at room temperature for 2 minutes. The mixture was cooled to 0°C, and triethylamine (0.680 mL, 4.88 mmol) was added, then the mixture was stirred at the same temperature for 30 minutes. To the mixture were added water and 1.0 mol/L hydrochloric acid, and the mixture was extracted with chloroform. The organic layer was washed with water and brine, dried over anhydrous sodium sulfate, and concentrated under reduced pressure. The residue was purified by preparative silica gel thin layer chromatography (hexane/ethyl acetate = 3/2) to give Compound k {N-[4-(2,2-dimethylpropionyl)-5-ethenesulfonylaminomethyl-5-phenyl-4,5-dihydro-1,3,4-thiadiazol-2-yl]-2,2-dimethylpropanamide} (0.408 g, 72%).
¹H NMR (300 MHz, CDCl₃) δ (ppm): 1.29 (s, 9H), 1.33 (s, 9H), 3.85 (dd, J = 13.5, 4.8 Hz, 1H), 4.49 (dd, J = 13.5, 8.1 Hz, 1H), 5.29 (br s, 1H), 5.93 (br d, J = 9.9 Hz, 1H), 6.27 (br d, J = 16.5 Hz, 1H), 6.53 (br dd, J = 16.4, 9.6 Hz, 1H), 7.27-7.34 (m, 5H), 8.06 (br s, 1H). APCI-MS m/z: 466 (M)⁺.

### [Example 26]

### Compound 1: (-)-N-[4-(2,2-Dimethylpropionyl)-5-(2-ethylaminoethanesulfonylaminomethyl)-5-phenyl-4,5-dihydro-1,3,4-thiadiazol-2-yl]-2,2-dimethylpropanamide

Compound k {N-[4-(2,2-dimethylpropionyl)-5-ethenesulfonylaminomethyl-5-phenyl-4,5-dihydro-1,3,4-thiadiazol-2-yl]-2,2-dimethylpropanamide} (1.50 g, 3.21 mmol) obtained in Example 25 was dissolved in acetonitrile (60 mL), and 70% aqueous ethylamine (13.9 mL) was added, then the mixture was stirred at room temperature for 1 hour. The mixture was concentrated under reduced pressure, and the resulting residue was dissolved in ethanol. To the solution was added water, and the deposited solid was collected by filtration to give Compound 1 {(-)-N-[4-(2,2-dimethylpropionyl)-5-(2-ethylaminoethanesulfonylaminomethyl)-5-phenyl-4,5-dihydro-1,3,4-thiadiazol-2-yl]-2,2-dimethylpropanamide} (0.830 g, 51%).
¹H NMR (300 MHz, CDCl₃) δ (ppm): 1.09 (t, J= 7.0 Hz, 3H), 1.28 (s, 9H), 1.34 (s, 9H), 2.63 (q, J= 7.0 Hz, 2H), 3.03-3.12 (m, 2H), 3.16-3.24 (m, 2H), 4.02 (d, J= 13.2 Hz, 1H), 4.58 (d, *J*= 13.2 Hz, 1H), 7.27-7.35 (m, 6H), 8.02 (br s, 1H).
APCI-MS m/z: 512 (M+1)⁺.
Melting point: 169.0-171.0°C.
Specific rotation: A solution of the resulting compound in methanol gave a negative value as a specific rotation for sodium D line (wavelength: 589.3 nm) at 20°C.

### [Example 27]

### Compound m: (-)-N-[5-(2-Dimethylaminoethanesulfonylaminomethyl)-4-(2,2-dimethylpropionyl)-5-phenyl-4,5-dihydro-1,3,4-thiadiazol-2-yl]-2,2-dimethylpropanamide

Step 1: In the same manner as that in Example 26, from N-[4-(2,2-dimethylpropionyl)-5-ethenesulfonylaminomethyl-5-phenyl-4,5-dihydro-1,3,4-thiadiazol-2-yl]-2,2-dimethylpropanamide (0.05 g, 0.11 mmol) obtained according to the method described in WO2003/051854 and a 2 mol/L dimethylamine/methanol solution (0.10 mL), N-[5-(2-dimethylaminoethanesulfonylaminomethyl)-4-(2,2-dimethylpropionyl)-5-phenyl-4,5-dihydro-1,3,4-thiadiazol-2-yl]-2,2-dimethylpropanamide (0.02 g, 35%) was obtained.

Step 2: N-[5-(2-Dimethylaminoethanesulfonylaminomethyl)-4-(2,2-dimethylpropionyl)-5-phenyl-4,5-dihydro-1,3,4-thiadiazol-2-yl]-2,2-dimethylpropanamide (50 mg) obtained in Step 1 mentioned above was subjected to preparative high performance liquid chromatography (HPLC) [column: CHIRALPAK AD ϕ 20 x 250 mm (Daicel Chemical Industries, Ltd.), elution solvent: n-hexane/ethanol = 91/9, flow rate: 5.0 mL/minute], and fractions for retention times of 22 minutes and 33 minutes were collected, respectively. Among them, the fraction of 33 minutes was concentrated to give Compound m {(-)-N-[5-(2-dimethylaminoethanesulfonylaminomethyl)-4-(2,2-dimethylpropionyl)-5-phenyl-4,5-dihydro-1,3,4-thiadiazol-2-yl]-2,2-dimethylpropanamide} (17 mg, 34%).
¹H NMR (300 MHz, CDCl₃) δ (ppm): 1.28 (s, 9H), 1.34 (s, 9H), 2.25 (s, 6H), 2.73 (br q, J = 6.3 Hz, 1H), 2.84 (br q, J = 6.2 Hz, 1H), 3.18 (br t, J = 6.6 Hz, 2H), 4.02 (d, J = 13.2 Hz, 1H), 4.58 (d, J = 13.2 Hz, 1H), 5.85 (br s, 1H), 7.27-7.35 (m, 5H), 8.02 (br s, 1H).
APCI-MS m/z: 512 (M+1)⁺.
Melting point: 101.0-104.0°C.
Specific rotation: A solution of the resulting compound in methanol gave a negative value as a specific rotation for sodium D line (wavelength: 589.3 nm) at 20°C.

### [Example 28]

### Compound p: N-[5-(3-Aminopropanesulfonylaminomethyl)-4-(2,2-dimethylpropionyl)-5-phenyl-4,5-dihydro-1,3,4-thiadiazol-2-yl]-2,2-dimethylpropanamide

Step 1: The hydrochloride of Compound j {N-[5-aminomethyl-4-(2,2-dimethylpropionyl)-5-phenyl-4,5-dihydro-1,3,4-thiadiazol-2-yl]-2,2-dimethylpropanamide} (1.00 g, 2.42 mmol) obtained in Example 24 was suspended in dichloromethane (25 mL), and triethylamine (1.35 mL, 9.69 mmol) and 3-chloropropanesulfonyl chloride (0.442 mL, 3.63 mmol) were added under ice cooling, then the mixture was stirred at room temperature for 22 hours. To the mixture were added water and 1 mol/L hydrochloric acid, and the mixture was extracted with chloroform. The organic layer was washed with saturated aqueous sodium hydrogencarbonate and brine, dried over anhydrous sodium sulfate, and concentrated under reduced pressure. The residue was triturated with a mixed solvent of diisopropyl ether and ethyl acetate to give optically active N-[5-(3-chloropropanesulfonylaminomethyl)-4-(2,2-dimethylpropionyl)-5-phenyl-4,5-dihydro-1,3,4-thiadiazol-2-yl]-2,2-dimethylpropanamide (0.880 g, 70%).
¹H NMR (270 MHz, CDCl₃) δ (ppm): 1.29 (s, 9H), 1.35 (s, 9H), 2.25 (m, 2H), 3.22 (m, 2H), 3.63 (m, 2H), 4.01 (dd, J = 5.1, 13.7 Hz, 1H), 4.60 (dd, J = 8.0, 13.7 Hz, 1H), 5.19 (dd, J = 5.1, 8.0 Hz, 1H), 7.23-7.41 (m, 5H), 7.94 (s, 1H).
ESI-MS m/z: 515, 517 (M-H)⁻.

Step 2: The optically active N-[5-(3-chloropropanesulfonylaminomethyl)-4-(2,2-dimethylpropionyl)-5-phenyl-4,5-dihydro-1,3,4-thiadiazol-2-yl]-2,2-dimethylpropanamide (1.50 g, 2.90 mmol) obtained in Step 1 mentioned above, sodium iodide (8.69 g, 58.0 mmol) and sodium azide (1.89 g, 29.0 mmol) were suspended in DMF (20 mL), and the suspension was stirred at 90°C for 4 hours. To the mixture was added water, and the mixture was extracted with ethyl acetate. The organic layer was washed with brine, dried over anhydrous sodium sulfate, and concentrated under reduced pressure. The residue was triturated with diethyl ether to give optically active N-[5-(3-azidopropanesulfonylaminomethyl)-4-(2,2-dimethylpropionyl)-5-phenyl-4,5-dihydro-1,3,4-thiadiazol-2-yl]-2,2-dimethylpropanamide (1.82 g).
Next, the resulting optically active N-[5-(3-azidopropanesulfonylaminomethyl)-4-(2,2-dimethylpropionyl)-5-phenyl-4,5-dihydro-1,3,4-thiadiazol-2-yl]-2,2-dimethylpropanamide was dissolved in THF (53 mL), and water (10.6 mL) and triphenylphosphine (1.24 g, 4.73 mmol) were added, then the mixture was stirred at room temperature for 16 hours. The mixture was concentrated under reduced pressure, and water and saturated aqueous sodium hydrogencarbonate were added, then the mixture was extracted with ethyl acetate. The organic layer was extracted with aqueous hydrochloric acid, and the aqueous layer was made basic by adding saturated aqueous sodium hydrogencarbonate, and then extracted with ethyl acetate. The resulting organic layer was concentrated under reduced pressure to give Compound p {N-[5-(3-aminopropanesulfonylaminomethyl)-4-(2,2-dimethylpropionyl)-5-phenyl-4,5-dihydro-1,3,4-thiadiazol-2-yl]-2,2-dimethylpropanamide} (1.29 g, 89%). ¹H NMR (300 MHz, CDCl₃) δ (ppm): 1.29 (s, 9H), 1.33 (s, 9H), 1.96 (m, 2H), 2.85 (t, J = 6.6 Hz, 2H), 3.19 (t, J = 7.5 Hz, 2H), 3.99 (d, J = 13.7 Hz, 1H), 4.61 (d, J = 13.7 Hz, 1H), 7.24-7.39 (m, 5H).
APCI-MS m/z: 498 (M+H)⁺.

### [Example 29]

### Compound n: (-)-N-[5-(3-Dimethylaminopropanesulfonylaminomethyl)-4-(2,2-dimethylpropionyl)-5-phenyl-4,5-dihydro-1,3,4-thiadiazol-2-yl]-2,2-dimethylpropanamide

Compound p {N-[5-(3-aminopropanesulfonylaminomethyl)-4-(2,2-dimethylpropionyl)-5-phenyl-4,5-dihydro-1,3,4-thiadiazol-2-yl]-2,2-dimethylpropanamide} (1.00 g, 2.01 mmol) obtained in Example 28 was dissolved in dichloroethane (40 mL), and 37% aqueous formalin (1.63 mL, 0.201 mmol), acetic acid (1.15 mL, 20.1 mmol) and sodium triacetoxyborohydride (4.26 g, 20.1 mmol) were added, then the mixture was stirred at room temperature for 13 hours. To the mixture were added water and saturated aqueous sodium hydrogencarbonate, and the mixture was extracted with chloroform. The organic layer was washed with brine, dried over anhydrous sodium sulfate, and concentrated under reduced pressure. The residue was purified by silica gel column chromatography (chloroform/methanol = 9/1 -> 4/1 -> 7/3) to give Compound n {(-)-N-[5-(3-dimethylaminopropanesulfonylaminomethyl)-4-(2,2-dimethylpropionyl)-5-phenyl-4,5-dihydro-1,3,4-thiadiazol-2-yl]-2,2-dimethylpropanamide} (0.910 mg, 86%).
¹H NMR (270 MHz, CDCl₃) δ (ppm): 1.29 (s, 9H), 1.33 (s, 9H), 1.96 (m, 2H), 2.20 (s, 6H), 2.36 (t, J = 6.7 Hz, 2H), 3.12 (m, 2H), 3.96 (d, J = 13.4 Hz, 1H), 4.59 (m, 1H), 5.57 (br, 1H), 7.23-7.38 (m, 5H), 7.96 (br, 1H).
APCI-MS m/z: 526 (M+H)⁺.
Melting point: 92.0-95.0°C. Specific rotation: A solution of the resulting compound in methanol gave a negative value as a specific rotation for sodium D line (wavelength: 589.3 nm) at 20°c.

### [Example 30]

### Compound o: 4-[3-(2,2-Dimethylpropionyl)-5-(2,2-dimethylpropionylamino)-2-phenyl -2,3-dihydro-1,3,4-thiadiazol-2-yl]-N-(2-hydroxyethyl)butanamide

Step 1: In the same manner as that of the method described in to WO2003/051854, from 4-[3-(2,2-dimethylpropionyl)-5-(2,2-dimethylpropionylamino)-2-phenyl-2,3-dihydro-1,3,4-thiadiazol-2-yl]butanoic acid methyl ester (11.2 g, 25.9 mmol) obtained according to the method described in WO2003/051854 and sodium borohydride (2.94 g, 77.6 mmol), 4-[5-amino-3-(2,2-dimethylpropionyl)-2-phenyl-2,3-dihydro-1,3,4-thiadiazol-2-yl]butanoic acid methyl ester (1.54 g, 17%) was obtained. APCI-MS m/z: 364 (M+H)⁺.

Step 2: In the same manner as that in Step 1 of Example 15, from 4-[5-amino-3-(2,2-dimethylpropionyl)-2-phenyl-2,3-dihydro-1, 3,4-thiadiazol-2-yl]butanoic acid methyl ester (1.54 g, 4.24 mmol) obtained in Step 1 mentioned above, (S)-(+)-2-phenylpropionic acid (1.99 g, 13.2 mmol), thionyl chloride (20 mL) and pyridine (1.80 mL, 22.0 mmol), a diastereomer mixture was obtained. The resulting diastereomer mixture was purified by silica gel column chromatography (chloroform/acetone = 60/12) to give one diastereomer of N-[3-(2,2-dimethylpropionyl)-2-phenyl-5-(2-phenylpropionylamino)-2,3-dihydro-1,3,4-thiadiazol-2-yl]butanoic acid methyl ester (0.679 g, 32%) as a fraction eluted first.
¹H NMR (300 MHz, CDCl₃) δ (ppm): 1.24 (s, 9H), 1.54 (d, J = 8.0 Hz, 3H), 1.42-1.67 (m, ¹H), 1.99-2.15 (m, ¹H), 2.20-2.32 (m, ¹H), 2.38-2.46 (m, 2H), 3.03-3.16 (m, ¹H),
3.62-3.71 (m, 1H), 3.67 (s, 3H), 7.18-7.47 (m, 10H), 7.64 (br s, 1H).
APCI-MS m/z: 496 (M+H)⁺.

Step 3: Sodium hydroxide (0.240 g, 6.01 mmol) was dissolved in water (4.0 mL), and dioxane (8.0 mL) was added, then the mixture was stirred. To the resulting solution was added the one diastereomer of N-[3-(2,2-dimethylpropionyl)-2-phenyl-5-(2-phenylpropionylamino)-2,3-dihydro-1,3,4-thiadiazol-2-yl]butanoic acid methyl ester (0.992 g, 2.00 mmol) obtained in Step 2 mentioned above, and the mixture was stirred at room temperature for 5 hours. To the mixture were added 1 mol/L hydrochloric acid (20 mL) and water (30 mL), and deposited white solid was collected by filtration. The resulting solid was washed with water and diisopropyl ether, and dried under reduced pressure to give 4-[3-(2,2-dimethylpropionyl)-2-phenyl-5-(2-phenyl-propionylamino)-2,3-dihydro-1,3,4-thiadiazol-2-yl]butanoic acid (9.60 g, 99%).
APCI-MS m/z: 481 (M+H)⁺.

Step 4: To 4-[3-(2,2-dimethylpropionyl)-2-phenyl-5-(2-phenylpropionylamino)-2,3-dihydro-1,3,4-thiadiazol-2-yl]butanoic acid (1.03 g, 2.14 mmol) obtained above were added oxalyl chloride (0.223 mL, 2.57 mmol) and DMF (17 µL, 0.214 mmol) at 0°C, and the mixture was stirred at the same temperature for 1 hour. The mixture was concentrated under reduced pressure, to the residue was added dichloromethane (20 mL), and the mixture was stirred at 0°C. Then, ethanolamine (1.2 mL, 21.4 mmol) was added to the mixture, and the mixture was stirred at room temperature for 3 hours. To the mixture were added 1 mol/L hydrochloric acid (20 mL) and water (30 mL), and the mixture was extracted with chloroform. The organic layer was washed with brine, dried over anhydrous sodium sulfate, and concentrated under reduced pressure. To the resulting residue was added diisopropyl ether, and the deposited white solid was collected by filtration. The resulting solid was washed with water and diisopropyl ether, and dried under reduced pressure to give 4-[3-(2,2-dimethylpropionyl)-2-phenyl-5-(2-phenylpropionylamino)-2,3-dihydro-1,3,4-thiadiazol-2-yl]-N-(2-hydroxyethyl)butanamide (1.10 g, 99%).
APCI-MS m/z: 525 (M+H)⁺.

Step 5: To 4-[3-(2,2-dimethylpropionyl)-2-phenyl-5-(2-phenylpropionylamino)-2,3-dihydro-1,3,4-thiadiazol-2-yl]-N-(2-hydroxyethyl)butanamide (1.21 g, 2.31 mmol) obtained in Step 4 mentioned above was added dichloromethane (20 mL), and the mixture was stirred at 0°C. Then, to the mixture were added pyridine (0.470 mL, 5.77 mmol) and tert-butyldimethylsilyl chloride (869 mg, 5.77 mmol), and the mixture was stirred at room temperature for 3 hours. To the mixture were added 1 mol/L hydrochloric acid (20 mL) and water (30 mL), and the mixture was extracted with chloroform. The organic layer was washed with brine, dried over anhydrous sodium sulfate, and concentrated under reduced pressure. To the resulting residue was added diisopropyl ether, and the deposited white solid was collected by filtration. The resulting solid was washed with water and diisopropyl ether, and dried under reduced pressure to give N-[2-(tert-butyldimethylsiloxy)ethyl]-4-[3-(2,2-dimethylpropionyl)-2-phenyl-5-(2-phenylpropionylamino)-2,3-dihydro-1,3,4-thiadiazol-2-yl]butanamide (1.25 g, 85%).
APCI-MS m/z: 638 (M+H)⁺.

Step 6: In the same manner as that in Step 2 of Example 15, from N-[2-(tert-butyldimethylsiloxy)ethyl]-4-[3-(2,2-dimethylpropionyl)-2-phenyl-5-(2-phenylpropionylamino)-2,3-dihydro-1,3,4-thiadiazol-2-yl]butanamide (0.376 g, 0.588 mmol) obtained in Step 5 mentioned above and sodium borohydride (0.111 g, 2.94 mmol), optically active 4-[5-amino-3-(2,2-dimethylpropionyl)-2-phenyl-2,3-dihydro-1,3,4-thiadiazol-2-yl]-N-[2-(tert-butyldimethylsiloxy)ethyl]butanamide (0.113 g, 38%) was obtained.
¹H NMR (270 MHz, CDCl₃) δ (ppm): 0.03 (s, 3H), 0.07 (s, 3H), 0.86 (s, 9H), 0.90 (s, 9H), 2.15-2.28 (m, 1H), 2.49-2.58 (m, 1H), 2.62-2.82 (m, 2H), 3.07-3.13 (m, 1H), 3.27-3.47 (m, 3H), 3.59-3.72 (m, 2H), 4.21 (br s, 2H), 5.97 (m, 1H), 7.22-7.44 (m, 5H).
APCI-MS m/z: 507 (M+H)⁺.

Step 7: In the same manner as that in Step 3 of Example 15, from the optically active 4-[5-amino-3-(2,2-dimethylpropionyl)-2-phenyl-2,3-dihydro-1,3,4-thiadiazol-2-yl]-N-[2-(tert-butyldimethylsiloxy)ethyl]butanamide (0.0683 g, 0.135 mmol) obtained in Step 6 mentioned above, pyridine (131 µL, 1.62 mmol) and trimethylacetyl chloride (0.166 mL, 1.35 mmol), optically active N-[2-(tert-butyldimethylsiloxy)ethyl]-4-[3-(2,2-dimethylpropionyl)-5-(2,2-dimethylpropionylamino)-2-phenyl-2,3-dihydro-1,3,4-thiadiazol-2-yl] butanamide (68.0 mg, 83%) was obtained.
Step 8: The optically active N-[2-(tert-butyldimethylsiloxy)ethyl]-4-[3-(2,2-dimethylpropionyl)-5-(2,2-dimethylpropionylamino)-2-phenyl-2,3-dihydro-1,3,4-thiadiazol-2-yl] butanamide (71.0 mg, 0.117 mmol) obtained in Step 7 mentioned above was dissolved in THF (1 mL), to the solution was added a 1 mol/L solution of tetrabutylammonium fluoride in THF (0.16 mL), and the mixture was stirred at room temperature for 50 minutes. To the mixture was added water (1 mL), and the mixture was extracted with ethyl acetate. The organic layer was washed with brine, dried over anhydrous sodium sulfate, and concentrated under reduced pressure. The residue was purified by column chromatography (chloroform/methanol = 9/1) to give Compound o {4-[3-(2,2-dimethylpropionyl)-5-(2,2-dimethylpropionylamino)-2-phenyl-2,3-dihydro-1,3,4-thiadiazol-2-yl]-N-(2-hydroxyethyl)butanamide} (47.6 mg, 85%) as a white solid.
¹H NMR (300 MHz, CDCl₃) δ (ppm): 1.28 (s, 9H), 1.33 (s, 9H), 1.56 (m, 1H), 2.22-2.51 (m, 4H), 3.15 (m, 1H), 3.35 (m, 1H), 3.45 (m, 1H), 3.61-3.76 (m, 2H), 6.31 (br s, 1H), 7.41-7.72 (m, 5H), 8.05 (br s, 1H).
APCI-MS m/z: 477 (M+H)⁺.

### [Example 31]

### Preparation of [3-(2,2-dimethylpropionyl)-5-(2,2-dimethylpropionylamino)-2-phenyl-2,3-dihydro-1,3,4-thiadiazol-2-ylmethyl]carbamic acid tert-butyl ester

Step 1: 2-Aminoacetophenone hydrochloride (400 g, 2.33 mol) was dissolved in a mixed solvent of water (2.8 L) and ethyl acetate (3.6 L), and di-tert-butyl dicarbonate (534 g, 2.45 mol) together with ethyl acetate (400 mL) were added under ice cooling. Aqueous potassium carbonate (322 g/1.2 L) was dropped to the solution with vigorously stirring over 1 hour. After the mixture was stirred for 1.5 hours under ice cooling, the temperature was elevated to 30°C, and the mixture was stirred for 1 hour at 30°C. Disappearance of the starting material was confirmed by analysis based on high performance liquid chromatography (HPLC), and then the organic layer was separated and washed with brine (800 mL). The organic layer was concentrated under reduced pressure to give 2-(tert-butoxycarbonylamino)acetophenone (610 g) as a slightly yellow oil. This compound was used for the following step without further purification.
¹H NMR (300 MHz, DMSO-d₆) δ (ppm): 7.96 (br d, *J*=7.4 Hz, 2H), 7.61 (tt, *J*=7.4, 1.6 Hz, 1H), 7.49 (br t, *J*=7.4 Hz, 2H), 5.54 (br s, 1H), 4.66 (d, *J*=4.6 Hz, 2H), 1.48 (s, 9H).

Step 2: 2-(tert-Butoxycarbonylamino)acetophenone (610 g) obtained above was dissolved in methanol (4.0 L), and the solution was cooled on ice. Thiosemicarbazide (425 g, 4.66 mol) was dissolved in diluted hydrochloric acid (concentrated hydrochloric acid (388 mL) and water (1612 mL)), and an about half volume of this solution (1 L) was added dropwise to the aforementioned solution over 10 minutes. Then, seed crystals of 2-(tert-butoxycarbonylamino)acetophenone thiosemicarbazone (400 mg) prepared in Reference Example 20 were added, and then the remaining thiosemicarbazide solution was added dropwise over 30 minutes. The mixture was further stirred at room temperature for 1 hour, and water (2.0 L) was added, then the mixture was stirred at 5°C for 1 hour. The deposited solid was collected by filtration, and washed with cooled 50% aqueous methanol (1.2 L) and then with cold water (800 mL). The resulting solid was dried at 50°C for 24 hours under reduced pressure to give 2-(tert-butoxycarbonylamino)acetophenone thiosemicarbazone as a white solid (694 g, yield: 92.1% (for two steps)).
¹H NMR (300 MHz, DMSO-d₆) δ (ppm): 10.6 (br s, 1H), 8.37 (br s, 1H), 8.03-7.83 (m, 3H), 7.67 (br t, *J*= 4.1 Hz, 1H), 7.42-7.30 (m, 3H), 4.17 (br d, *J*= 4.1 Hz, 2H), 1.38 (s, 9H).

Step 3: 2-(tert-Butoxycarbonylamino)acetophenone thiosemicarbazone obtained above (690 g, 2.24 mol) was suspended in acetonitrile (6.9 L), and pyridine (619 g) was added, then the mixture was cooled on ice. To the mixture was added dropwise pivaloyl chloride (809 g) over 25 minutes. After the mixture was stirred at room temperature for 5.5 hours, 1 mol/L hydrochloric acid (1.2 L) was added, and the mixture was stirred for several minutes, and then the aqueous phase was removed. To the organic layer was added water (690 mL) dropwise over 40 minutes with stirring. The solid deposited during the dropping, and the resulting suspension was further stirred at 5°C for 1 hour. The deposited solid was collected by filtration, and washed with cooled acetonitrile/water (10:1, 2.0 L) and then with cold water (1.4 L). The resulting solid was dried under reduced pressure at 25°C for 32 hours to give the title compound {[3-(2,2-dimethylpropionyl)-5-(2,2-dimethylpropionylamino)-2-phenyl-2,3-dihydro-1,3, 4-thiadiazol-2-ylmethyl]carbamic acid tert-butyl ester} as a white solid (1031 g, yield: 95.4%).
¹H NMR (300 MHz, DMSO-d₆) δ (ppm): 10.89 (s, 1H), 7.40-7.20 (m, 5H), 6.74 (br dd, J= 6.8, 6.1 Hz, 1H), 4.37 (dd, J= 14.5, 6.8 Hz, 1H), 3.98 (dd, J= 14.5, 6.1 Hz, 1H), 1.37 (s, 9H), 1.29 (s, 9H), 1.17 (s, 9H). [Example 32]

### Preparation of [(2R)-3-(2,2-dimethylpropionyl)-5-(2,2-dimethylpropionylamino)-2-phenyl-2,3-dihydro-1,3,4-thiadiazol-2-ylmethyl]carbamic acid tert-butyl ester (Compound q)

[3-(2,2-Dimethylpropionyl)-5-(2,2-dimethylpropionylamino)-2-phenyl-2,3-dihydro-1,3,4-thiadiazol-2-ylmethyl]carbamic acid tert-butyl ester obtained in Example 31 was subjected to high performance liquid chromatography (HPLC) [column: CHIRALPAK AD ϕ 4.6 x 250 mm (Daicel Chemical Industries, Ltd.), elution solvent: hexane/ethanol = 80/20, flow rate: 1.0 mL/minute], and a fraction for a retention time of 5.76 minutes was collected among fractions for retention times of 4.63 minutes and 5.76 minutes to give Compound q {[(2R)-3-(2,2-dimethylpropionyl)-5-(2,2-dimethylpropionylaniino)-2-phenyl-2,3-dihydro-1,3,4-thiadiazol-2-ylmethyl]-carbamic acid tert-butyl ester}.

### [Example 33]

### Preparation of hydrochloride of N-[(5R)-5-aminomethyl-4-(2,2-dimethylpropionyl)-5-phenyl-4,5-dihydro-1,3,4-thiadiazol-2-yl]-2,2-dimethylpropanamide (hydrochloride of Compound j)

Compound q (19.8 g, 41.6 mmol) obtained in Example 32 or the like was dissolved in ethyl acetate (198 mL), and a 4 mol/L hydrogen chloride - ethyl acetate solution (99.2 mL, 397 mmol) was dropped at 25°C over 20 minutes. After the mixture was stirred at room temperature for 9 hours, the mixture was cooled on ice, and further stirred at 4°C for 1 hour. The deposited solid was collected by filtration, washed with cooled ethyl acetate (60 mL), and then dried at 60°C for 22 hours under reduced pressure to give hydrochloride of Compound j as a white solid (16.7 g, yield: 97.1%).
¹H NMR (300 MHz, DMSO-d₆) δ (ppm): 11.19 (s, 1H), 8.34 (br s, 3H), 7.47-7.22 (m, 5H), 4.21 (d, *J*= 13.7 Hz, 1H), 4.08 (d, *J*= 13.7 Hz, 1H), 1.34 (s, 9H), 1.18 (s, 9H).

### Reference Examples 1 to 13 (Compounds 1 to 13)

Compounds 1 to 13 were synthesized according to the method described in WO2003/051854 or WO2004/111024 respectively.

### Reference Example 14

### Compound 14: N-{4-(2,2-Dimethylpropionyl)-5-[2-(2-ethylaminoethanesulfonylamino)ethyl]-5-phenyl-4, 5-dihydro-1,3,4-thiadiazol-2-yl}-2,2-dimethylpropanamide

Step 1: Palladium(II) acetate (125 mg, 0.559 mmol) and triphenylphosphine (317 mg, 1.21 mmol) were dissolved in tetrahydrofuran (THF, 50 mL). To the resulting solution were added N-tert-butoxycarbonyl-B-alanine (2.07 g, 10.9 mmol), phenylboronic acid (1.61 g, 13.2 mmol), distilled water (0.477 mL, 26.5 mmol) and trimethylacetic anhydride (3.23 mL, 15.9 mmol), and the mixture was stirred at 60°C for 24 hours. The mixture was filtered, saturated aqueous sodium hydrogencarbonate was added to the filtrate, and the mixture was extracted with ethyl acetate. The organic layer was washed with brine, dried over anhydrous sodium sulfate, and concentrated under reduced pressure. The residue was purified by silica gel column chromatography (hexane/ethyl acetate = 9/1 -> 4/1) to give (3-oxo-3-phenylpropyl)carbamic acid tert-butyl ester (1.85 g, 68%).

Step 2: (3-Oxo-3-phenylpropyl)carbamic acid tert-butyl ester (513 mg, 2.06 mmol) obtained in Step 1 mentioned above was dissolved in methanol (40 mL). To the resulting solution was added thiosemicarbazide hydrochloride (562 mg, 4.40 mmol), and the mixture was stirred at room temperature for 8 hours. To the mixture was added water, and the mixture was extracted with ethyl acetate. The organic layer was washed with brine, dried over anhydrous sodium sulfate, and concentrated under reduced pressure to give a pale yellow solid (513 mg). Apart of the resulting solid (198 mg) was dissolved in dichloromethane (10 mL). To the resulting solution were added pyridine (0.300 mL, 3.73 mmol) and trimethylacetyl chloride (0.415 mL, 3.37 mmol), and the mixture was stirred at room temperature for 22 hours. To the mixture was added saturated aqueous sodium hydrogencarbonate, and the mixture was further stirred at room temperature for 1 hour, and extracted with ethyl acetate. The organic layer was washed with brine, dried over anhydrous sodium sulfate, and concentrated under reduced pressure. The residue was purified by preparative silica gel thin layer chromatography (n-hexane/ethyl acetate = 2/1) to give {2-[3-(2,2-dimethylpropionyl)-5-(2,2-dimethylpropionylamino)-2-phenyl-2,3-dihydro-1,3,4-thiadiazol-2-yl]ethyl}-carbamic acid tert-butyl ester (319 mg, 100%).
APCI-MS m/z: 491(M+H)⁺.

Step 3: {2-[3-(2,2-Dimethylpropionyl)-5-(2,2-dimethylpropionylamino)-2-phenyl-2,3-dihydro-1,3,4-thiadiazol-2-yl]ethyl}carbamic acid tert-butyl ester (274 mg, 0.557 mmol) obtained in Step 2 mentioned above was dissolved in dichloromethane (10 mL). To the resulting solution was added trifluoroacetic acid (1.0 mL), and the mixture was stirred at room temperature for 3 hours, and then concentrated under reduced pressure. To the residue was added diisopropyl ether, and the mixture was stirred for 3 hours. The deposited white solid was collected by filtration to give trifluoroacetate of N-[5-(2-aminoethyl)-4-(2,2-dimethylpropionyl)-5-phenyl-4,5-dihydro-1,3,4-thiadiazol-2-yl]-2,2-dimethylpropanamide (252 mg, 90%).
APCI-MS m/z: 391(M+H)⁺.

Step 4: The trifluoroacetate of N-[5-(2-aminoethyl)-4-(2,2-dimethylpropionyl)-5-phenyl-4,5-dihydro-1,3,4-thiadiazol-2-yl]-2,2-dimethylpropanamide (0.25 g, 0.53 mmol) obtained in Step 3 mentioned above was dissolved in methanol (5 mL), and the solution was loaded on a column filled with ion exchange silica gel [SCX (Varian, BONDESIL SCX 40 µM)]. After SCX was washed with methanol, a fraction eluted with a 1% hydrogen chloride - methanol solution was collected, and the fraction was concentrated under reduced pressure to give hydrochloride of N-[5-(2-aminoethyl)-4-(2,2-dimethylpropionyl)-5-phenyl-4,5-dihydro-1,3,4-thiadiazol-2-yl]-2,2-dimethylpropanamide (0.19 g) as a white solid.
The hydrochloride obtained above was dissolved in dichloromethane (10 mL), and 2-chloroethanesulfonyl chloride (0.14 mL, 2.2 mmol) and triethylamine (0.62 mL, 4.6 mmol) were added at 0°C, then the mixture was stirred at the same temperature for 4 hours, and then at room temperature for 10 hours. To the mixture was added saturated aqueous sodium hydrogencarbonate, and the mixture was extracted with ethyl acetate. The organic layer was washed with saturated aqueous ammonium chloride and brine, dried over anhydrous sodium sulfate, and concentrated under reduced pressure. The residue was purified by preparative silica gel thin layer chromatography (n-hexane/ethyl acetate = 2/1) to give N-[4-(2,2-dimethylpropionyl)-5-(2-ethenesulfonylaminoethyl)-5-phenyl-4,5-dihydro-1,3,4-thiadiazol-2-yl]-2,2-dimethylpropanamide (0.17 g, 65%).
¹H NMR (300 MHz, CDCl₃) δ (ppm): 1.30 (s, 9H), 1.32 (s, 9H), 2.48-2.62 (m, 1H), 3.10-3.64 (m, 3H), 4.45 (br t, J = 5.7 Hz, 1H), 5.95 (d, J = 9.6 Hz, 1H), 6.26 (d, J = 16.2 Hz, 1H), 6.52 (dd, J = 9.6, 16.2 Hz, 1H), 7.22-7.37 (m, 5H), 7.91 (br s, 1H).

Step 5: N-[4-(2,2-Dimethylpropionyl)-5-(2-ethenesulfonylaminoethyl)-5-phenyl-4,5-dihydro-1,3,4-thiadiazol-2-yl]-2,2-dimethylpropanamide (0.16 g, 0.33 mmol) obtained in Step 4 mentioned above was dissolved in acetonitrile (10 mL), and 70% aqueous ethylamine (1.0 mL, 12 mmol) was added, then the mixture was stirred at room temperature for 3 hours. The reaction mixture was concentrated under reduced pressure, and the residue was purified by preparative silica gel thin layer chromatography (chloroform/methanol/concentrated aqueous ammonia = 100/10/1) to give Compound 14 {N-{4-(2,2-dimethylpropionyl)-5-[2-(2-ethylaminoethanesulfonylamino)ethyl]-5-phenyl-4,5-dihydro-1,3,4-thiadiazol-2-yl}-2,2-dimethylpropanamide} (0.15 g, 86%).

### Reference Example 15

### Compound 15: N-{4-(2,2-Dimethylpropionyl)-5-[2-(hydroxyamino)ethanesulfonylaminomethyl]-5-phenyl-4,5-dihydro-[1,3,4]thiadiazol-2-yl}-2,2-dimethylpropionamide

Compound 10 {N-[4-(2,2-dimethylpropionyl)-5-ethenesulfonylaminomethyl-5-phenyl-4,5-dihydro-1,3,4-thiadiazol-2-yl]-2,2-dimethylpropanamide} (101 mg, 0.216 mmol) obtained in Reference Example 10 was dissolved in acetonitrile (5 mL), and hydroxylamine (containing 50% water, 0.265 mL) was added, then the mixture was stirred at room temperature for 1.5 hours. The reaction mixture was concentrated under reduced pressure, and the resulting residue was purified by preparative thin layer chromatography (chloroform/methanol = 20/1), and then triturated with diisopropyl ether to give Compound 15 {N-{4-(2,2-dimethylpropionyl)-5-[2-(hydroxyamino)ethanesulfonylaminomethyl]-5-phenyl-4,5-dihydro-[1,3,4]thiadiazol-2-yl}-2,2-dimethylpropionamide} (89 mg, 83%).
¹H NMR (300 MHz, CDCl₃) δ (ppm) : 1.29 (s, 9H), 1.34 (s, 9H), 3.01 (br d, J =14.4 Hz, 1H), 3.30-3.70 (m, 3H), 4.04 (dd, J = 10.8, 12.3 Hz, 1H), 4.58 (dd, J = 3.3, 12.3 Hz, 1H), 5.21 (dd, J = 3.3, 10.8 Hz, 1H), 5.27 (br s, 1H), 6.46 (br s, 1H), 7.20-7.41 (m, 5H), 7.94 (br s, 1H).

### Reference Example 16

### Compound 16: N-{4-(2,2-Dimethylpropionyl)-5-[2-(N-ethyl-N-hydroxyamino)ethanesulfonylaminomethyl]-5-phenyl-4,5-dihydro-[1,3,4]thiadiazol-2-yl}-2,2-dimethylpropionamide

Compound 15 {N-{4-(2,2-dimethylpropionyl)-5-[2-(hydroxyamino)ethanesulfonylaminomethyl]-5-phenyl-4,5-dihydro-[1,3,4]thiadiazol-2-yl}-2,2-dimethylpropionamide} (60 mg, 0.12 mmol) obtained in Reference Example 15 was dissolved in 1,2-dichloroethane (2.4 mL), and acetaldehyde (0.095 mL, 1.7 mmol), acetic acid (0.068 mL, 1.2 mmol) and sodium triacetoxyborohydride (256 mg, 1.21 mmol) were added, then the mixture was stirred at room temperature for 10 minutes. To the mixture were added water and saturated aqueous sodium hydrogencarbonate, and the mixture was extracted with chloroform. The organic layer was washed with brine, dried over anhydrous sodium sulfate, and then concentrated under reduced pressure. The residue was purified by preparative thin layer chromatography (chloroform/methanol = 20/1), and then triturated with diisopropyl ether to give Compound 16 {N-{4-(2,2-dimethylpropionyl)-5-[2-(N-ethyl-N-hydroxyamino)ethanesulfonylaminomethyl]-5-phenyl-4,5-dihydro-[1,3,4]thiadiazol-2-yl}-2,2-dimethylpropionamide} (23 mg, 36%).
¹H NMR (300 MHz, CDCl₃) δ (ppm) : 1.09 (t, J = 7.2 Hz, 3H), 1.28 (s, 9H), 1.39 (s, 9H), 2.73-2.90 (m, 3H), 2.90-3.30 (m, 2H), 3.40-3.60 (m, 1H), 4.04 (dd, J = 9.6, 12.9 Hz, 1H), 4.60 (dd, J = 5.1, 12.9 Hz, 1H), 5.50 (br s, 1H), 6.50 (br s, 1H), 7.20-7.40 (m, 5H), 7.93 (br s, 1H).

### Reference Example 17

### Compound 17: N-{5-[2-(2-Aminoethylsulfanyl)ethanesulfonylaminomethyl]-4-(2,2-dimethylpropionyl)-5-phenyl-4,5-dihydro[1,3,4]thiadiazol-2-yl}-2,2-dimethylpropionamide

Step 1: Compound 10 {N-[4-(2,2-dimethylpropionyl)-5-ethenesulfonylaminomethyl-5-phenyl-4,5-dihydro-1,3,4-thiadiazol-2-yl]-2,2-dimethylpropanamide} (1.001 g, 2.145 mmol) obtained in Reference Example 10 was dissolved in methanol (20 mL), and 2-aminoethanethiol hydrochloride (1.230 g, 10.83 mmol) and saturated aqueous sodium hydrogencarbonate (15 mL) were added, then the mixture was stirred at room temperature for 1.5 hours. To the mixture was added water, and the mixture was extracted with ethyl acetate. The organic layer was washed with brine, dried over anhydrous sodium sulfate, and concentrated under reduced pressure. The residue was triturated with diethyl ether and then with a mixed solvent of diethyl ether and ethyl acetate (9/1). The resulting crude product was purified by silica gel column chromatography (chloroform/methanol = 6/1), and triturated with diethyl ether to give free base of Compound 17 {N-{5-[2-(2-aminoethylsulfanyl)ethanesulfonylaminomethyl]-4-(2,2-dimethylpropionyl)-5-phenyl-4,5-dihydro[1,3,4]thiadiazol-2-yl}-2,2-dimethylpropionamide} (756 mg, 65%).
APCI-MS m/z: 544 (M+1)⁺.

Step 2: The free base of Compound 17 (756 mg, 1.39 mmol) obtained in Step 1 mentioned above was dissolved in ethyl acetate (20 mL), and to the solution was added 4 mol/L hydrogen chloride - ethyl acetate solution (0.7 mL) under ice cooling. The reaction mixture was concentrated under reduced pressure, and diethyl ether was added, then the mixture was stirred at room temperature for 30 minutes. Then, the deposited solid was collected by filtration to give hydrochloride of Compound 17 (795 mg, 99%).
¹H NMR (270 MHz, DMSO-d₆) δ (ppm): 1.18 (s, 9H), 1.27 (s, 9H), 2.77 (t, J = 7.1 Hz, 2H), 2.86 (m, 2H), 2.98 (t, J = 7.1 Hz, 2H), 3.37 (m, 2H), 4.00 (d, J = 14.0 Hz, 1H), 4.36 (d, J = 14.0 Hz, 1H), 7.21-7.38 (m, 5H), 8.50 (br, 3H).

### Reference Example 18

### Compound 18: N-{5-[(2-Aminoethylsulfanyl)methanesulfonylaminomethyl]-4-(2,2-dimethylpropionyl)-5-phenyl-4,5-dihydro[1,3,4]thiadiazol-2-yl}-2,2-dimethylpropionamide

Step 1: The hydrochloride of Compound 11 {N-[5-aminomethyl-4-(2,2-dimethylpropionyl)-5-phenyl-4,5-dihydro-1,3,4-thiadiazol-2-yl]-2,2-dimethylpropanamide} (4.00 g, 9.69 mmol) obtained in Reference Example 11 was dissolved in dichloromethane (100 mL), and triethylamine (4.05 mL, 29.1 mmol) and chloromethanesulfonyl chloride (1.12 mL, 12.6 mmol) were added under ice cooling, then the mixture was stirred at room temperature for 4 hours. To the mixture were added water and 1 mol/L hydrochloric acid, and the mixture was extracted with chloroform. The organic layer was washed with brine, dried over anhydrous sodium sulfate, and concentrated under reduced pressure. The residue was triturated with a mixed solvent of chloroform and diisopropyl ether to give N-[5-chloromethanesulfonylaminomethyl-4-(2,2-dimethylpropionyl)-5-phenyl-4,5-dihydro[1,3,4]thiadiazol-2-yl]-2,2-dimethylpropionamide (3.82 g, 92%).
APCI-MS m/z: 489, 491 (M+1)⁺.

Step 2: N-[5-Chloromethanesulfonylaminomethyl-4-(2,2-dimethylpropionyl)-5-phenyl-4,5-dihydro[1,3,4]thiadiazol-2-yl]-2,2-dimethylpropionamide (3.818 g, 7.807 mmol) obtained in Step 1 mentioned above was dissolved in DMF (70 mL), and tert-butyl N-(2-mercaptoethyl)carbamate (13.3 mL, 78.1 mmol) and saturated aqueous sodium hydrogencarbonate (15 mL) were added, then the mixture was stirred at 70°C for 5.5 hours. After cooling, water was added, and the mixture was extracted with ethyl acetate. The organic layer was washed with brine, dried over anhydrous sodium sulfate, and concentrated under reduced pressure. The residue was purified by silica gel column chromatography (n-hexane/ethyl acetate = 9/1 -> 7/3), and then triturated with diisopropyl ether to give [2-({[3-(2,2-dimethylpropionyl)-5-(2,2-dimethylpropionylamino)-2-phenyl-2,3-dihydro[1,3,4]thiadiazol-2-ylmethyl]sulfamoyl}-methylsulfanyl)ethyl]carbamic acid tert-butyl ester (1.926 g, 39%).
APCI-MS m/z: 630 (M+1)⁺.

Step 3: [2-({[3-(2,2-Dimethylpropionyl)-5-(2,2-dimethylpropionylamino)-2-phenyl-2,3-dihydro[1,3,4]thiadiazol-2-ylmethyl]sulfamoyl}methylsulfanyl)ethyl]carbamic acid tert-butyl ester (1.926 g, 3.058 mmol) obtained in Step 2 mentioned above was dissolved in dichloromethane (15 mL), and trifluoroacetic acid (15 mL) was added, then the mixture was stirred at room temperature for 1 hour. After the mixture was concentrated under reduced pressure, to the residue were added water and saturated aqueous sodium hydrogencarbonate, and the mixture was extracted with ethyl acetate. The organic layer was washed with brine, dried over anhydrous sodium sulfate, and concentrated under reduced pressure. The residue was purified by silica gel column chromatography (chloroform/methanol = 9/1 -> chloroform containing ammonia/methanol = 9/1), and then triturated with diisopropyl ether to give free base of Compound 18 {N-{5-[(2-aminoethylsulfanyl)methanesulfonylaminomethyl]-4-(2,2-dimethylpropionyl)-5-phenyl-4,5-dihydro[1,3,4]thiadiazol-2-yl}-2,2-dimethylpropionamide} (1.011 g, 63%).
APCI-MS m/z: 530 (M+1)⁺.
Step 4: In the same manner as that in Step 2 of Reference Example 17, the free base of Compound 18 (515 mg, 0.972 mmol) obtained in Step 3 mentioned above was treated with 4 mol/L hydrogen chloride - ethyl acetate solution (0.5 mL) to give hydrochloride of Compound 18 (490 mg, 89%).
¹H NMR (300 MHz, CDCl₃) δ (ppm): 1.26 (s, 9H), 1.32 (s, 9H), 3.10 (m, 2H), 3.11 (m, 2H), 4.06 (dd, J = 5.4, 14.2 Hz, 1H), 4.15 (d, J = 15.0 Hz, 1H), 4.24 (d, J = 15.0 Hz, 1H), 4.67 (m, 1H), 6.34 (m, 1H), 7.23-7.38 (m, 5H), 8.14 (br, 3H), 8.38 (s, 1H).

### Reference Example 19

### Compound 19: N-{2-[3-Acetyl-5-(2-oxopiperidino)-2-phenyl-2,3-dihydro-1,3,4-thiadiazol-2-yl]ethyl}methanesulfonamide

In the same manner as that in Example 17, from N-[2-(3-acetyl-5-amino-2-phenyl-2,3-dihydro-1,3,4-thiadiazol-2-yl)ethyl]methanesulfonamide (0.150 g, 0.438 mmol) obtained on the way of Step 3 of Example 20, pyridine (51.0 µL, 0.631 mmol), 5-bromovaleryl chloride (70.5 µL, 0.526 mmol) and sodium acetate (0.0498 g, 0.607 mmol), Compound 19 {N-{2-[3-acetyl-5-(2-oxopiperidino)-2-phenyl-2,3-dihydro-1,3,4-thiadiazol-2-yl]ethyl}methanesulfonamide} (0.181 g, 97%) was obtained.
¹H NMR (270 MHz, CDCl₃) δ (ppm): 1.82-1.98 (m, 4H), 2.33 (s, 3H), 2.52-2.62 (m, 3H), 2.95 (s, 3H), 3.27-3.38 (m, 2H), 3.59-3.70 (m, 1H), 3.84-3.92 (m, 2H), 4.62 (br s, 1H), 7.23-7.37 (m, 5H).
APCI-MS m/z: 423 (M-1)⁻.

### Reference Example 20

### Preparation of seed crystals of 2-(tert-butoxycarbonylamino)acetophenone thiosemicarbazone

2-(tert-Butoxycarbonylamino)acetophenone (3.00 g) was dissolved in methanol (21.0 mL). To the solution was added an aqueous solution (water: 9.0 mL) of thiosemicarbazide hydrochloride (3.11 g, 24.4 mmol) at room temperature. After the mixture was stirred at the same temperature for 30 minutes, water (12.0 mL) was added, and the mixture was stirred at room temperature for 20 minutes and then at 0°C for 1 hour. The deposited solid was collected by filtration and washed with cooled 50% aqueous methanol (20 mL). The resulting solid was dried at 40°C under reduced pressure to give seed crystals of 2-(tert-butoxycarbonylamino)acetophenone thiosemicarbazone (3.56 g, yield: 95.1%) as a white solid.

### Industrial Applicability

According to the present invention, a therapeutic and/or prophylactic agent for a solid tumor, and an optically active thiadiazoline derivative useful as a therapeutic and/or prophylactic agent for a solid tumor can be provided.

## Claims

1. A therapeutic and/or prophylactic agent for a solid tumor, which comprises a thiadiazoline derivative represented by the general formula (I): 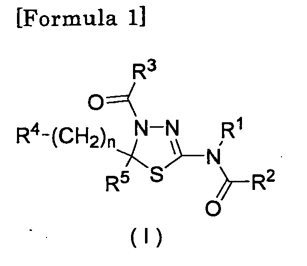 {wherein, n represents an integer of 1 to 3,
R¹ represents a hydrogen atom,
R² represents lower alkyl, or
R¹ and R² are combined together to represent alkylene,
R³ represents lower alkyl,
R⁴ represents NHSO₂R⁶ (wherein R⁶ represents lower alkyl which may be substituted with one or two substituents selected from the group consisting of hydroxy, lower alkoxy, amino, hydroxyamino, (lower alkyl)amino, di-(lower alkyl)amino,
N-hydroxy(lower alkyl)amino, amino-substituted (lower alkyl)thio, (lower alkyl)amino-substituted (lower alkyl)thio and di-(lower alkyl)amino-substituted (lower alkyl)thio, or lower alkenyl),
NHR⁷ [wherein R⁷ represents lower alkyl which may be substituted with one or two substituents selected from the group consisting of hydroxy, lower alkoxy, amino, (lower alkyl)amino and di-(lower alkyl)amino, COR⁸ (wherein R⁸ represents lower alkyl which may be substituted with one or two substituents selected from the group consisting of hydroxy, lower alkoxy, amino, (lower alkyl)amino, di-(lower alkyl)amino, carboxy, phenyl, hydroxyphenyl, imidazolyl, guanidyl, methylthio and (lower alkoxy)carbonylamino, a nitrogen-containing aliphatic heterocyclic group which may be substituted with (lower alkoxy)carbonyl or oxo, or lower alkoxy), or a hydrogen atom], or
CONHR⁹ (wherein R⁹ represents lower alkyl which may be substituted with one or two substituents selected from the group consisting of hydroxy, lower alkoxy, amino, (lower alkyl)amino and di-(lower alkyl)amino), and
R⁵ represents aryl which may be substituted with one to three substituents selected from the group consisting of halogen, hydroxy, lower alkoxy, nitro, amino, cyano and carboxy}, or a pharmaceutically acceptable salt thereof.

2. The therapeutic and/or prophylactic agent according to claim 1, wherein R⁵ is phenyl.

3. The therapeutic and/or prophylactic agent according to claim 1 or 2, wherein R³ is methyl, ethyl, isopropyl or tert-butyl.

4. The therapeutic and/or prophylactic agent according to any one of claims 1 to 3, wherein R¹ is a hydrogen atom.

5. The therapeutic and/or prophylactic agent according to any one of claims 1 to 4, wherein R² is methyl or tert-butyl.

6. The therapeutic and/or prophylactic agent according to any one of claims 1 to 3, wherein R¹ and R² are combined together to form trimethylene or tetramethylene.

7. The therapeutic and/or prophylactic agent according to any one of claims 1 to 6, wherein R⁴ is NHSO₂R⁶ (wherein R⁶ has the same meaning as that mentioned above).

8. The therapeutic and/or prophylactic agent according to any one of claims 1 to 6, wherein R⁴ is CONHR⁹ (wherein R⁹ has the same meaning as that mentioned above).

9. The therapeutic and/or prophylactic agent according to any one of claims 1 to 8, wherein n is 1 or 2.

10. The therapeutic and/or prophylactic agent according to any one of claims 1 to 9, wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.

11. The therapeutic and/or prophylactic agent according to any one of claims 1 to 9, wherein the solid tumor is a tumor selected from the group consisting of lung cancer, breast cancer, thymoma, mesothelioma, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.

12. A thiadiazoline derivative represented by the general formula (II): 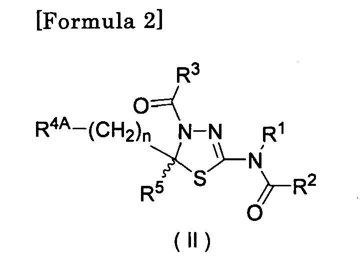 [wherein R¹, R², R³, R⁵, and n have the same meanings as those mentioned above, and R^{4A} represents NHSO₂R⁶ (wherein R⁶ has the same meaning as that mentioned above), NHR^{7A} (wherein R^{7A} represents a hydrogen atom or lower alkyl which may be substituted with one or two substituents selected from the group consisting of hydroxy, lower alkoxy, amino, (lower alkyl)amino and di-(lower alkyl)amino), or CONHR⁹ (wherein R⁹ has the same meaning as that mentioned above)], or a pharmaceutically acceptable salt thereof, which shows a negative value as a specific rotation at 20°C for sodium D line (wavelength: 589.3 nm) when the thiadiazoline derivative or the pharmaceutically acceptable salt thereof is dissolved in methanol.

13. The thiadiazoline derivative or the pharmaceutically acceptable salt thereof according to claim 12, wherein R⁵ is phenyl.

14. The thiadiazoline derivative or the pharmaceutically acceptable salt thereof according to claim 12 or 13, wherein R³ is methyl, ethyl, isopropyl or tert-butyl.

15. The thiadiazoline derivative or the pharmaceutically acceptable salt thereof according to any one of claims 12 to 14, wherein R¹ is a hydrogen atom.

16. The thiadiazoline derivative or the pharmaceutically acceptable salt thereof according to any one of claims 12 to 15, wherein R² is methyl or tert-butyl.

17. The thiadiazoline derivative or the pharmaceutically acceptable salt thereof according to any one of claims 12 to 14, wherein R¹ and R² are combined together to form trimethylene or tetramethylene.

18. The thiadiazoline derivative or the pharmaceutically acceptable salt thereof according to any one of claims 12 to 17, wherein R^{4A} is NHSO₂R⁶ (wherein R⁶ has the same meaning as that mentioned above).

19. The thiadiazoline derivative or the pharmaceutically acceptable salt thereof according to any one of claims 12 to 17, wherein R^{4A} is CONHR⁹ (wherein R⁹ has the same meaning as that mentioned above).

20. The thiadiazoline derivative or the pharmaceutically acceptable salt thereof according to any one of claims 12 to 19, wherein n is 1 or 2.

21. The thiadiazoline derivative or the pharmaceutically acceptable salt thereof according to claim 12, wherein the thiadiazoline derivative represented by the general formula (II) is a thiadiazoline derivative represented by any one of the following formulas (a) to (q).

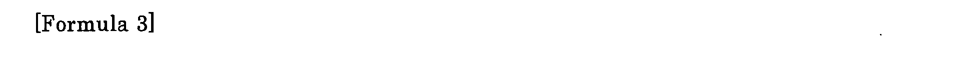

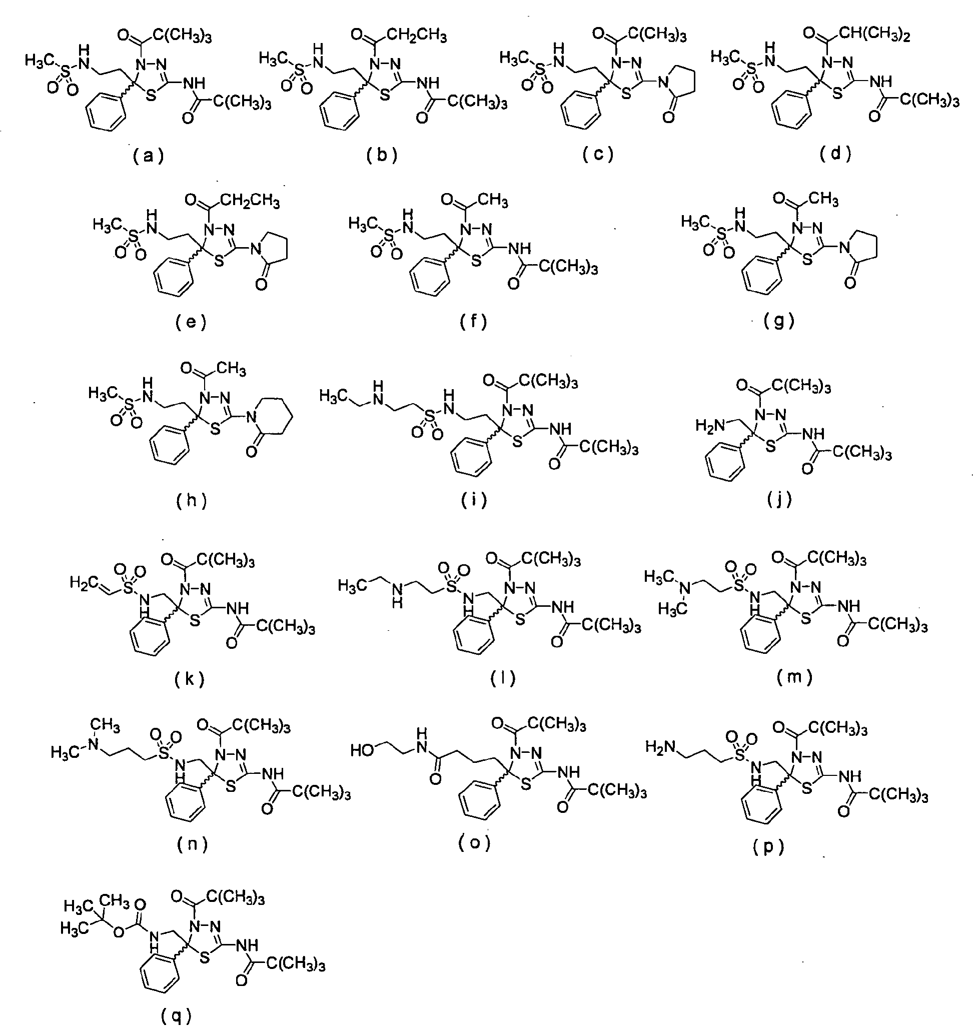

22. A pharmaceutical composition which comprises the thiadiazoline derivative or the pharmaceutically acceptable salt thereof according to any one of claims 12 to 21.

23. A mitotic kinesin Eg5 inhibitor which comprises the thiadiazoline derivative or the pharmaceutically acceptable salt thereof according to any one of claims 12 to 21.

24. A therapeutic and/or prophylactic agent for a solid tumor, which comprises the thiadiazoline derivative or the pharmaceutically acceptable salt thereof according to any one of claims 12 to 21.

25. The therapeutic and/or prophylactic agent according to claim 24, wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.

26. The therapeutic and/or prophylactic agent according to claim 24, wherein the solid tumor is a tumor selected from the group consisting of lung cancer, breast cancer, thymoma, mesothelioma, colon cancer, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.

27. A method for preparing a thiadiazoline derivative represented by the general formula (IA), or a salt thereof: 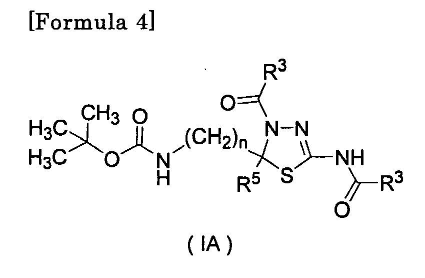 (wherein n, R³, and R⁵ have the same meanings as those mentioned above) as the thiadiazoline derivative, or the salt thereof described in claim 1 wherein R¹ is a hydrogen atom, R² and R³, which are the same, represent lower alkyl, and R⁴ is tert-butoxycarbonylamino, which comprises (1) the step of reacting a compound represented by the general formula (X), or a salt thereof: 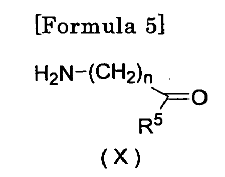 (wherein n and R⁵ have the same meanings as those mentioned above) with di-tert-butyl dicarbonate in a non-hydrophilic solvent in the presence of an aqueous solution containing a base selected from the group consisting of sodium hydrogencarbonate, potassium hydrogencarbonate, sodium carbonate, potassium carbonate, potassium hydroxide and sodium hydroxide to obtain a compound represented by the general formula (XI): 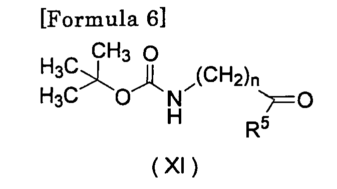 (wherein n and R⁵ have the same meanings as those mentioned above), (2) the step of reacting the compound represented by the above general formula (XI) with thiosemicarbazide in a solvent selected from methanol, ethanol, propanol, 2-propanol, butanol, sec-butanol and tert-butanol, or in a mixed solvent of any one of these solvents and water in the presence of hydrochloric acid to obtain a compound represented by the general formula (XII): 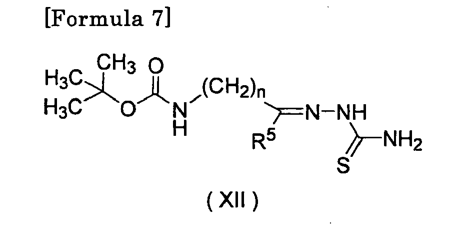 (wherein n and R⁵ have the same meaning as those defined above), and (3) the step of reacting the compound represented by the above general formula (XII) with a compound represented by the formula R³COX (wherein X represent halogen, and R³ has the same meaning as that mentioned above), or a compound represented by the formula (R³CO)₂O (wherein R³ has the same meaning as that mentioned above) in a hydrophilic solvent in the presence of a base.

28. A method for preparing a thiadiazoline derivative represented by the general formula (IB) or (IIB), or a salt thereof: 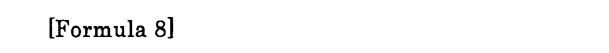 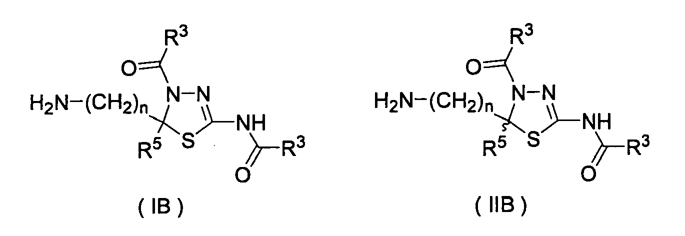 (wherein n, R³, and R⁵ have the same meanings as those mentioned above) as the thiadiazoline derivative, or the salt thereof described in claim 1 or 12 wherein R¹ is a hydrogen atom, R² and R³, which are the same, represent lower alkyl, and R⁴ or R^{4A} is amino, which comprises the step of treating the compound represented by the general formula (IA), or the salt thereof described in claim 27 in a solvent selected from the group consisting of methyl acetate, ethyl acetate, propyl acetate, isopropyl acetate, butyl acetate, methanol, ethanol and dioxane in the presence of hydrogen chloride.

29. A method for preparing a thiadiazoline derivative represented by the general formula (IIB), or a salt thereof: 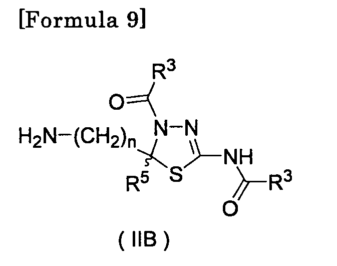 (wherein n, R³, and R⁵ have the same meanings as those mentioned above) as the thiadiazoline derivative, or the salt thereof according to claim 12 wherein R¹ is a hydrogen atom, R² and R³, which are the same, represent lower alkyl, and R^{4A} is amino, which comprises (1) the step of performing optical resolution of the thiadiazoline derivative represented by the general formula (IA), or the salt thereof described in claim 27 by high performance liquid chromatography, and (2) the step of treating the resulting thiadiazoline derivative, or the resulting salt thereof in a solvent selected from the group consisting of methyl acetate, ethyl acetate, propyl acetate, isopropyl acetate, butyl acetate, methanol, ethanol and dioxane in the presence of hydrogen chloride.

30. A method for preparing a thiadiazoline derivative represented by the general formula (IC) or (IIC), or a salt thereof: 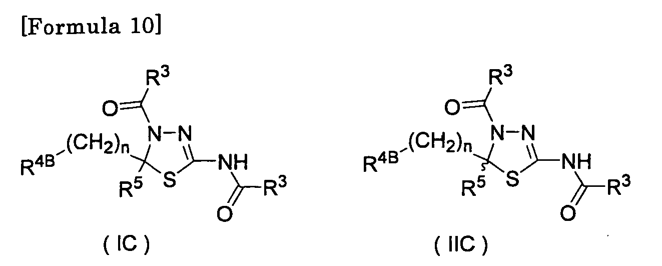 [wherein n, R¹, R³, and R⁵ have the same meanings as those mentioned above, R^{4B} represents NHSO₂R⁶ (R⁶ has the same meaning as that defined above), or NHR⁷ (R⁷ has the same meaning as that defined above)] as the thiadiazoline derivative, or the salt thereof described in any one of claims 1 to 5, 7, 9, 12 to 16, 18, 20 and 21, wherein R¹ is a hydrogen atom, R² and R³, which are the same, represent lower alkyl, and R⁴ or R^{4A} is NHSO₂R⁶ (R⁶ has the same meaning as that defined above), or NHR⁷ (R⁷ has the same meaning as that defined above), which comprises using the thiadiazoline derivative represented by the general formula (IB) or (IIB), or the salt thereof described in claim 28 or 29.

31. A method for therapeutic and/or prophylactic treatment of a solid tumor, which comprises administering an effective amount of the thiadiazoline derivative or the pharmaceutically acceptable salt thereof described in any one of claims 1 to 9.

32. The method according to claim 31, wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.

33. The method according to claim 31, wherein the solid tumor is a tumor selected from the group consisting of lung cancer, breast cancer, thymoma, mesothelioma, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.

34. A method for inhibiting mitotic kinesin Eg5, which comprises administering an effective amount of the thiadiazoline derivative or the pharmaceutically acceptable salt thereof according to any one of claims 12 to 21.

35. A method for therapeutic and/or prophylactic treatment of a solid tumor, which comprises administering an effective amount of the thiadiazoline derivative or the pharmaceutically acceptable salt thereof according to any one of claims 12 to 21.

36. The method according to claim 35, wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.

37. The method according to claim 35, wherein the solid tumor is a tumor selected from the group consisting of lung cancer, breast cancer, thymoma, mesothelioma, colon cancer, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.

38. Use of the thiadiazoline derivative or the pharmaceutically acceptable salt thereof described in any one of claims 1 to 9 for the manufacture of a therapeutic and/or prophylactic agent for a solid tumor.

39. The use according to claim 38, wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.

40. The use according to claim 38, wherein the solid tumor is a tumor selected from the group consisting of lung cancer, breast cancer, thymoma, mesothelioma, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.

41. Use of the thiadiazoline derivative or the pharmaceutically acceptable salt thereof according to any one of claims 12 to 21 for the manufacture of a mitotic kinesin Eg5 inhibitor.

42. Use of the thiadiazoline derivative or the pharmaceutically acceptable salt thereof according to any one of claims 12 to 21 for the manufacture of a therapeutic and/or prophylactic agent for a solid tumor.

43. The use according to claim 42, wherein the solid tumor is a tumor selected from the group consisting of a tumor of chest, gastrointestinal cancer, a tumor of female genitalia, a tumor of male genitalia, a tumor of urinary organ, a tumor of cranial nerve, head and neck cancer, retinoblastoma, mediastinal tumor, skin cancer, bone tumor and soft tissue tumor.

44. The use according to claim 42, wherein the solid tumor is a tumor selected from the group consisting of lung cancer, breast cancer, thymoma, mesothelioma, colon cancer, gastric cancer, esophageal cancer, hepatic cancer, pancreatic cancer, bile duct cancer, gallbladder cancer, ovarian cancer, germ cell tumor, choriocarcinoma, vulvar cancer, uterine cancer, vaginal cancer, uterine sarcoma, prostate cancer, penile cancer, testicular tumor, bladder cancer, renal cancer, renal pelvic-ureteral cancer, brain tumor, hypophyseal tumor, glial tumor, acoustic schwannoma, neuroblastoma, oral cancer, pharyngeal cancer, laryngeal cancer, nasal sinus cancer, thyroid cancer, retinoblastoma, mediastinal tumor, skin cancer, bone tumor and soft tissue tumor.
